# EUROPEAN PATENT SPECIFICATION

(11) **EP 2 853 590 B1**
(45) Date of publication and mention of the grant of the patent: **07.11.2018**
(21) Application number: 13794060.7
(22) Date of filing: 22.05.2013
(51) Int. Cl.: C12N 5/0783, A61K 35/17, A61P 35/00, C12N 15/09

(54) **METHOD FOR PRODUCING ANTIGEN-SPECIFIC T CELLS**
VERFAHREN ZUR HERSTELLUNG ANTIGENSPEZIFISCHER T-ZELLEN
PROCÉDÉ DE PRODUCTION DE LYMPHOCYTES T SPÉCIFIQUES D'UN ANTIGÈNE

(30) Priority: 22.05.2012 JP 2012116639
(43) Date of publication of application: 01.04.2015
(73) Proprietor: The University of Tokyo, Bunkyo-ku, Tokyo 113-8654 (JP)
(72) Inventor: NAKAUCHI, Hiromitsu, Tokyo 113-8654 (JP); KANEKO, Shin, Tokyo 113-8654 (JP); NISHIMURA, Toshinobu, Tokyo 113-8654 (JP)
(74) Representative: Hoffmann Eitle
(86) International application number: PCT/JP2013/064291
(87) International publication number: WO 2013/176197

(56) References cited:
- WO-A1-2011/096482
- WO-A2-2010/141801
- US-A1- 2008 166 325
- US-A1- 2011 318 828
- RIDDELL S R ET AL: "RESTORATION OF VIRAL IMMUNITY IN IMMUNODEFICIENT HUMANS BY THE ADOPTIVE TRANSFER OF T CELL CLONES", SCIENCE, AMERICAN ASSOCIATION FOR THE ADVANCEMENT OF SCIENCE, US, vol. 257, 10 July 1992 (1992-07-10), pages 238-241, XP001179181, ISSN: 0036-8075, DOI: 10.1126/SCIENCE.1352912
- TOSHINOBU NISHIMURA ET AL: "Generation of Rejuvenated Antigen-Specific T Cells by Reprogramming to Pluripotency and Redifferentiation", CELL STEM CELL, vol. 12, no. 1, 1 January 2013 (2013-01-01), pages 114-126, XP055218131, ISSN: 1934-5909, DOI: 10.1016/j.stem.2012.11.002
- TOSHINOBU NISHIMURA ET AL.: 'In Vitro Generation of Mature T Lymphocytes From Human Ips Cells and Genetic Analysis of TCR Gene Rearrangements.' BLOOD vol. 118, no. 21, 2011, page ABSTR.2984, XP055177106
- LAURENCE A. TURKA ET AL.: 'Thymocyte expression of RAG-1 and RAG-2: termination by T cell receptor cross-linking.' vol. 253, 1991, pages 778 - 781, XP055177108
- TOSHINOBU NISHIMURA ET AL.: 'Generation of Monoclonal TCR-Expressing Human T-Lineage Lymphocytes From Induced Pluripotent Stem Cells of Single Peripheral T-Lymphocyte Origin.' BLOOD vol. 116, no. 21, 2010, page ABSTR.490, XP055177505
- SEKI T ET AL.: 'Generation of induced pluripotent stem cells from human terminally differentiated circulating T cell' CELL STEM CELL vol. 7, no. 1, July 2010, pages 11 - 14, XP008162341
- TOSHINOBU NISHIMURA ET AL.: 'Rejuvenation of antigen-specific T cells through reprogramming and redifferentiation.' PROCEEDINGS OF THE JAPANESE SOCIETY FOR IMMUNOLOGY vol. 41, November 2012, page 174, XP008175765
- TOSHINOBU NISHIMURA ET AL.: 'T Saibo Yurai iPS Saibo to T Saibo no Bunka Yudo' REGENERATIVE MEDICINE vol. 11, June 2012, page 212, O-34-4, XP008175756
- ARATA KANEKO: 'Byogensei CD4 Helper T Saibo kara no Reprogramming to Seigyosei CD4T Saibo eno Saibunka Yudo o Mochiita, Atarashii Kogen Tokuiteki Men'eki Yokusei Ryoho no Kaihatsu' THE JAPANESE SOCIETY FOR REGENERATIVE MEDICINE ZASSHI vol. 11, no. 4, November 2012, pages 63 - 66, XP008175578

## Description

### FIELD OF THE INVENTION

The invention relates to a method for producing an antigen specific T cell and, in particular, a method for producing a human CD8 or CD4 single-positive cell having antigen specificity as defined in the appended claims, which comprises the steps of: differentiating an iPS cell that has been induced from a human T cell, into a CD4/CD8 double-negative cell, stimulating a T cell receptor of the CD4/CD8 double-negative cell, and differentiating the CD4/CD8 double-negative cell whose T cell receptor has been stimulated, into a CD8 single-positive cell or a CD4 single-positive cell. The disclosure also relates to a human CD8 or CD4 single-positive cell.

### DESCRIPTION OF THE RELATED ART

T cells play a major role in inducing adaptive immune response and systemic immune response to pathogens. In particular, cytotoxic T cells (CTL) recognize peptide antigens such as a peptide derived from viruses and tumors, which are presented together with a major histocompatibility complex (MHC class I or HLA class I) of an antigen-presenting cell via T cell receptors (TCR) located on the surface of the CTLs, and then CTLs exhibit cytotoxicity specific to the cells presenting the non-self-peptide antigens. Further, a part of the CTLs become long-term survival memory T cells and keep its cytotoxicity to the antigens to achieve a host memory, which facilitate a next response to the same non-self-antigens. Thus, CTLs play a major role in immune system fighting against virus or microbe infections and neoplasms (tumors) (see Non-Patent Documents 1 and 2).

CTLs are differentiated from CD8 single positive (SP) cells. CD8 SP cells are known to be differentiated from immature cells expressing neither of CD4 and CD8 (CD4/CD8 double negative (DN) cells) and no TCR via cells expressing both of CD4 and CD8 (CD4/CD8 double positive (DP) cells) in a thymus.

When T cells such as CTLs specifically recognize peptide antigens under the HLA restriction, they start to exhibit their proliferation function and effector function. The most important advantages of T cell immunity are the specific recognition against the target cells and elimination of the cells (see Non-Patent Documents 3 to 5).

However, T cell immunity can be prevented by incomplete recognition against antigens relating to latent virus infections or tumors/self-antigens. An exposure to long-term latent virus infections or tumors/self-antigens may impair the long-term survival capability, self-replication ability, and effector functions of T cells, and exhaust T cells, which may lead to the loss of antigen responsive T cell pool (see Non-Patent Documents 6 and 7).

Cellular adoptive immunotherapy, which utilizes antigen specific T cells expressing a specific TCR repertoire, has been thought to be a prospective therapy for several types of cancers and chronic virus infections. However, such specific T cells loss their T cell function during *ex vivo* proliferation treatment of samples from patients, and thus cellular adoptive immunotherapy exhibit only an insufficient therapeutic efficacy even though the T cells massively proliferated *ex vivo* are used in the therapy (see Non-Patent Documents 8).

Several approaches to treat such diseases which render antigen specificity to non-specific T cells have been developed (see Non-Patent Documents 9 and 10). However, the therapeutic effect of the approaches would be largely dependent on antigen specificity of the T cells induced and proliferated (see Non-Patent Documents 11 and 12). It has been known that a mispairing TCR is made upon introducing an exogenous TCR in order to induce hematopoietic stem cells or peripheral mature T cells into antigen specific T cells.

A method for inducing antigen specific immature T cells from pluripotent stem cells has been developed (see Non-Patent Document 13). However, it is thought that there are many hurdles before the implementation of the methods because no differentiation method from hematopoietic stem/progenitor cells into sufficiently functional human T cells has been established.

None of the previous studies proved an effective method for producing T cells, especially CTL (CD8 single positive) sells having both of antigen specificity and high proliferation capability (self-proliferation capability), although there is a strong need for such method and many attempts have so far been made.

Considering the above-mentioned situation, the inventors have succeeded in establishing iPS cells from human T cells having antigen specificity and a reconstituted TCR gene (hereinafter, also refer to as "T-iPS cells"), seeking a possibility of induced pluripotent stem cells (iPS cells) which maintain pluripotency and eternal self-replication. Further, the inventor have also succeeded in differentiating the T-iPS cells into T cells and discovered that T cells can be obtained which have the same TCR gene rearrangement pattern as the original T cell by the method (Patent Document 1).

### CITATION LIST

Patent Document 1: WO2011/096482

Non Patent Document 1: Greenberg,P.D., Adv Immunol, 1991, 49: 281-355
Non Patent Document 2: Zhang, N. & Bevan, M.J., Immunity, 2011, 35: 161-168
Non Patent Document 3: Jameson,S.C.&Masopust,D., Immunity, 2009, 31, 859-871
Non Patent Document 4: MacLeod,M.K.et al., Immunology, 2010, 130, 10-15
Non Patent Document 5: Butler,N.S.et al., Cell Microbiol, 2011, 13, 925-933
Non Patent Document 6: Klebanoff, C.A. et al., Immunol Rev, 2006, 211, 214-224
Non Patent Document 7: Wherry, E.J., Nat Immunol, 2011, 12, 492-499
Non Patent Document 8: June,C.H., J Clin Invest, 2007, 117, 1466-1476
Non Patent Document 9: Morgan,R.A.et al., Science, 2006, 314, 126-129
Non Patent Document 10: Porter,D.L., N Engl J Med, 2011, 365, 725-733
Non Patent Document 11: Bendle,G.M.et al., Nat Med, 2010, 16, 565-570
Non Patent Document 12: Topalian,S.L.et al., J Clin Oncol, 2011, 29, 4828-4836
Non Patent Document 13: Timmermans, F. et al., J Immunol, 2009, 182, 6879-6888

### SUMMARY OF THE INVENTION

As mentioned above, the inventors have developed a method for generating iPS cells from human T cells having antigen specificity and a method for differentiating the T-iPS cells into T cells. Further, it has been discovered that T cells can be obtained which have the same TCR gene rearrangement pattern as the original T cell by the method (WO2011/096482).

However, as is shown in Comparative example 1 of the specification, it is now found that the ratio of T cells having the same gene rearrangement pattern as the original ones to the obtained T cells are about 25%.

The invention provides a method for improving a ratio of the T cells having the same gene rearrangement pattern as the original ones to the T cells obtained by re-differentiation via T-iPS cells from human T cells having antigen specificity.

As mentioned above, the inventors studied TCR expression during re-differentiation of T-iPS cells into T cells, because it is known that no TCR is expressed in a normal T cells at the most immature stage (CD4/CD8 DN stage), which express neither of CD4 and CD8 during maturation process of T cells in a thymus.

It was surprisingly discovered that a TCR is expressed at the CD4/CD8 DN stage, which is contrary to the conventional understandings of TCR expression during maturation process of T cells in a thymus (see Example 1).

It was previously known that a TCR signal via an antigen peptide-MHC complex stops the expression of RAG genes and prevent further assembly of TCR genes during a process, so called "positive selection", in a thymus. It was also known that the signal induced by stimulation with anti-CD3 antibody, which is like a TCR signal, results in the similar effect.

Then, in the present invention, the inventors confirmed whether or not it is possible to suppress further rearrangements of TCR genes and improve the ratio of T cells having the same gene rearrangement pattern as the original ones by stimulating a TCR which is expressed on CD4/CD8 cells re-differentiated from T-iPS cells. In addition, the inventors confirmed whether or not the obtained cells can be differentiated into CD8 SP cells by applying molecules contributing to CD8 lineage cells to the CD4/CD8 DN cells which have been stimulated (see Example 1).

As a result, it was discovered that it is possible to dramatically improve the frequency of occurrence of CD8 SP cells having the same gene rearrangement pattern as the original ones, by stimulating a TCR expressing the CD4/CD8 DN cell from T-iPS cells with anti-CD3 antibody and the like, and applying molecules such as IL-7 contributing to CD8 lineage cells to the stimulated CD4/CD8 DN cell. Further, it was also discovered that the produced CD8 SP cell has a teromere longer than the original one and have high proliferation capability (replication capability). Furthermore, CD4 SP cells were obtained by stimulating a TCR expressing the CD4/CD8 DN cell from T-iPS cells with anti-CD3 antibody and the like, and applying molecules such as IL-7 to the CD4/CD8 DN cells which have been stimulated.

The invention was made based on the above-mentioned discovery and provides a method for producing a human CD8 positive cell having antigen specificity, comprising: the steps of: differentiating an iPS cell that has been induced from a human T cell, into a CD4/CD8 double-negative cell, stimulating a T cell receptor of the CD4/CD8 double-negative cell, and differentiating the CD4/CD8 double-negative cell whose T cell receptor has been stimulated, into a single positive T cell, in particular, a CD4 single-positive cell or a CD8 single-positive cell.

The disclosure also provides a single positive T cell, in particular, CD4 single-positive cell or a CD8 single-positive cell, having antigen specificity which is produced by the above methods.

The invention provides the followings:
(1) A method for producing a human single-positive CD4 or CD8 T cell having antigen specificity, which comprises the steps of:
   differentiating an iPS cell that has been induced from a human T cell, into a CD4/CD8 double-negative cell, wherein the human T cell has antigen specificity,
   stimulating a T cell receptor of the CD4/CD8 double-negative cell, and
   differentiating the CD4/CD8 double-negative cell whose T cell receptor has been stimulated, into a single-positive CD4 or CD8 T cell.
(2) The method according to item 1, wherein the single-positive CD4 or CD8 T cell is a CD8 single positive T cell.
(3) An isolated population of human single positive CD4 or CD8 T cells having antigen specificity which are obtainable by the method according to item 1 or 2, wherein the population has an improved ratio of T cells having the same TCR gene rearrangement pattern as the original human T cell compared to a population of human single positive CD4 or CD8 T cells obtained by said method without the step of stimulating a T cell receptor of the CD4/CD8 DN cell.
(4) A pharmaceutical composition, comprising the isolated population of the human single positive CD4 or CD8 T cell according to item 3.
(5) A method of producing a pharmaceutical composition, comprising producing a human single positive CD4 or CD8 T cells according to item 1 or 2 and formulating the human single positive CD4 or CD8 T cells into dosage forms by known pharmaceutical methods.

According to the invention, it is possible to dramatically improve the occurrence of appearance of T cells having the same gene rearrangement pattern as the original ones among the T cells obtained by re-differentiation from a human T cell having antigen specificity via T-iPS cells.

### BRIEF DESCRIPTION OF THE DRAWINGS

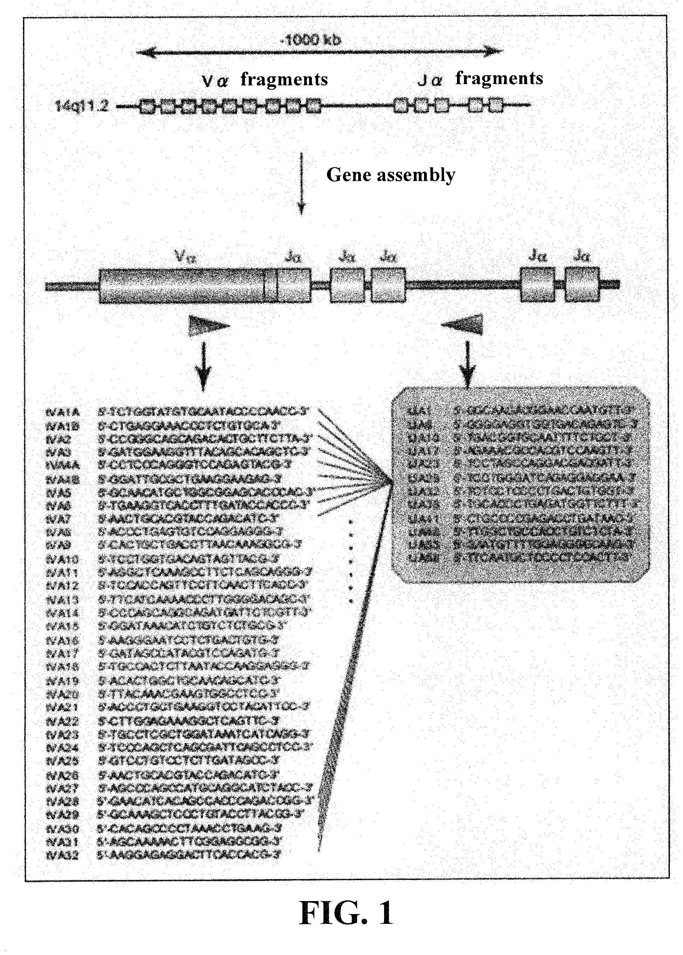

Fig. 1 represents an outline of the method for detecting rearranged TCRA genes. In brief, 34 Vα primers were designed in order to amplify all the arrangements of the Vα segments in the human TCRA gene locus, which lies in a region of more than 1,000 kb on chromosome 14 (14q11.2). In addition, 12 Jα primers were designed, downstream of the sequence of each of the segment Jα1, Ja6, Jα10, Jα17, Ja23, Ja29, Ja32, Ja36, Jα41, Ja48, Ja53, or Ja58, such that 3 to 7 different Jα segments were able to be amplified. All the Jα primers were mixed in one tube, and each of the Vα primers was used with the Jα-primers mix to perform 34 PCR reactions per sample, thereby to detect a rearranged TCRα gene.

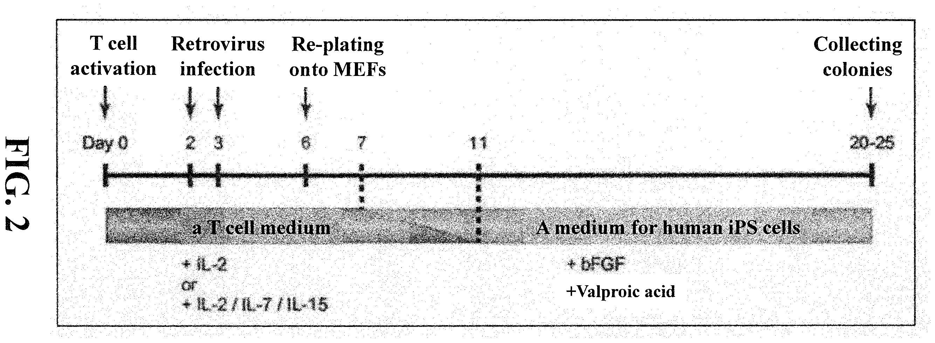

Fig. 2 is a schematic representation showing the process for producing T-cell-derived iPS (T-iPS) cells from peripheral blood T cells using retrovirus vectors, each encoding OCT3/4, SOX2, or KLF4. In the drawing, the tapering portion (a period of 7 to 11 days after the start of activation of T cells) represents a period during which the medium was gradually replaced to human iPS medium.

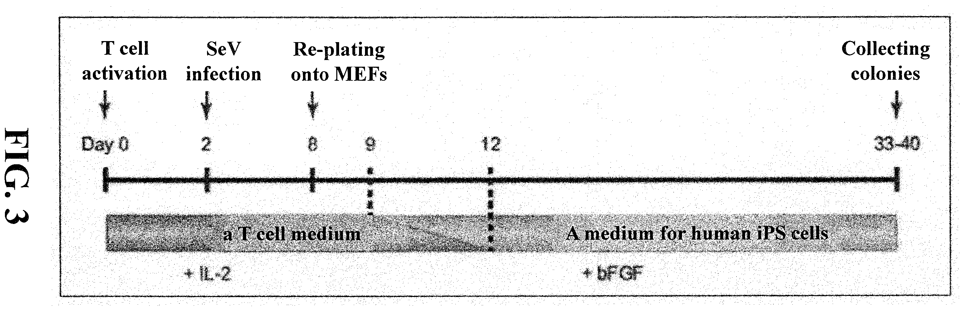

Fig. 3 is a schematic representation showing the process for producing T-iPS cells from cells of a CTL clone (H25-4T cells) using an SeV polycistronically encoding OCT3/4, SOX2, and KLF4, and an SeV encoding SV40LTAg. In the drawing, the tapering portion (a period of 9 to 12 days after the start of T cell activation) represents a period during which the medium was gradually replaced to human iPS medium.

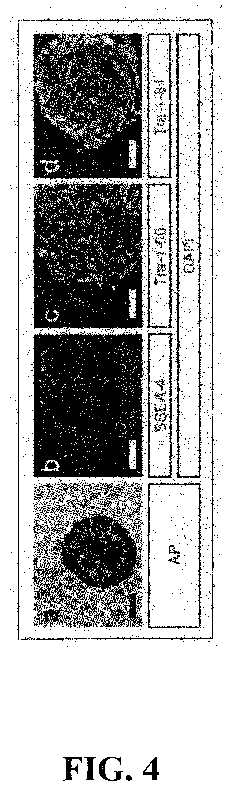

Fig. 4 represents pictures showing the results of observation under a microscope of the expression of alkaline phosphatase (AP) activity and pluripotency markers (SSEA-4, Tra-1-60, and Tra-1-81) in T-iPS cells (H254SeVT-3), cells of a cell line produced from H25-4T cells. In each of the pictures, the scale bar corresponds to 200 µm, and the pictures b to d show the result of counterstaining the nucleus with DAPI (in the pictures, blue-emitting portions).

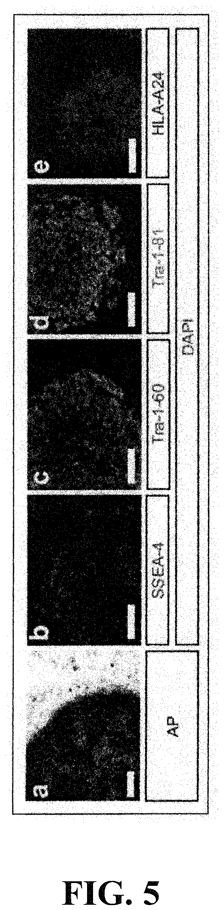

Fig. 5 represents pictures showing the results of observation under a microscope of the expression of AP activity and pluripotency markers (SSEA-4, Tra-1-60, and Tra-1-81) in iPS cells (TkT3V1-7), cells of a cell line produced from peripheral blood T cells. In the picture a, the scale bar corresponds to 500 µm. In each of the pictures b to e, the scale bar corresponds to 200 µm and the results of counterstaining the nucleus with DAPI are shown (in the pictures, blue-emitting portions).

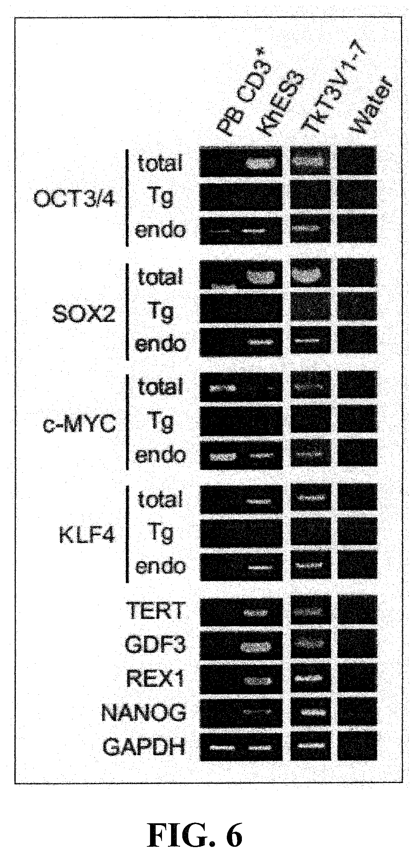

Fig. 6 represents pictures showing the results of an analysis by RT-PCR of the expression of pluripotent genes in TkT3V1-7 cells. In the drawing, "Tg" indicates the expression of the exogenous retrovirus-introduced gene, "endo" indicates the expression of the endogenous expressing gene, and "total" indicates the expression of the exogenous retrovirus-introduced gene and the endogenous expressing gene. "PB CD3+" indicates the results in the originating peripheral blood T cells of TkT3V1-7 cells, "KhES3" indicates the results (as positive control) in ES cells, which are pluripotent stem cells, and "Water" indicates the results (as negative control) of RT-PCR without added template DNA. In the RT-PCR, GAPDH was used as internal standard.

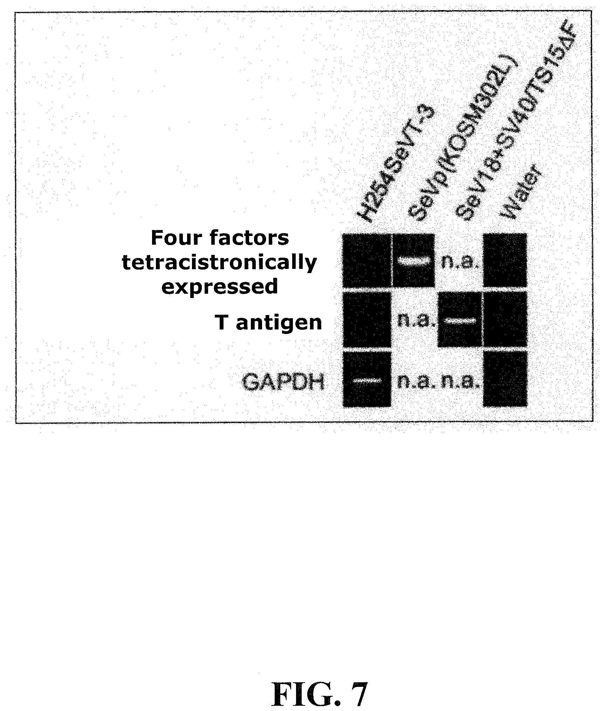

Fig. 7 represents pictures showing the results of detection by RT-PCR of the remaining SeV RNAs (four factors cistronically expressed (tetracistronic factors): OCT4, SOX2, KLF4, and c-MYC, and T antigen). In the drawing, "SeVp(KOSM302L)" and "SeV18+SV40/TS15ΔF" indicate the results in the respective SeVs which were used for establishing the H254SeVT-3, and "Water" indicates the results of RT-PCR without added template DNA (as negative control). In addition, "n.a." indicates "not assessed." In the RT-PCR, GAPDH was used as internal standard.

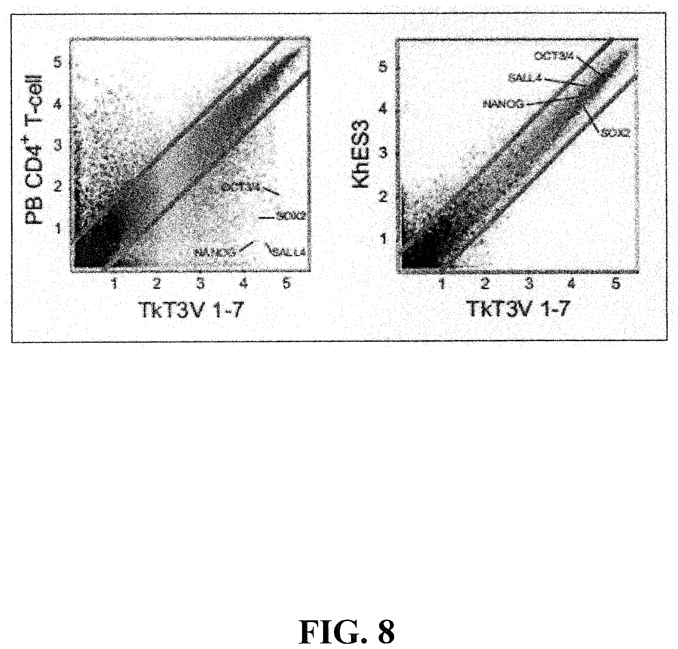

Fig. 8 represents scatter plots showing the results of a comprehensive analysis of gene expression using cDNA microarrays. The left panel shows the results of a comprehensive comparison of gene expression between CD4+ T cells (PB CD4+ T-cells) and TkT3V1-7 cells using cDNA microarrays. The right panel represents a scatter plot showing the results of a comprehensive comparison of gene expression between ES cells (KhES3) and TkT3V1-7 cells using cDNA microarrays. In each of the plots, the two parallel lines indicate that there was a fivefold difference between the corresponding samples.

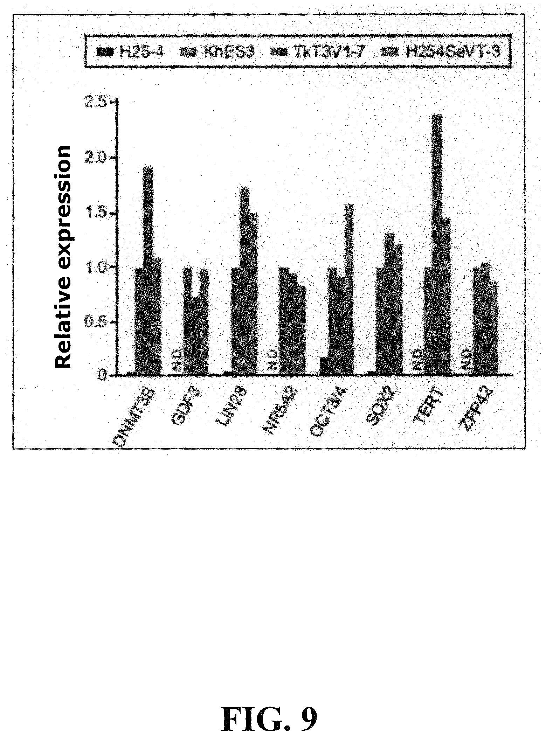

Fig. 9 represents a graph showing the results of an analysis by quantitative PCR of the expression of pluripotent genes in H25-4, KhES3, TkT3V1-7, and H254SeVT-3 cells. In each PCR reaction, the amount of expression of 18S rRNA was used as internal standard. In the graph, the abscissa axis represents, from left to right, the respective results for H25-4, KhES3, TkT3V1-7, and H254SeVT-3 cells. The ordinate axis represents a relative amount of expression when the amount of expression of each of the genes in KhES3 cells was set to be 1.0. "N.D." indicates "below detection limit (not detected)."

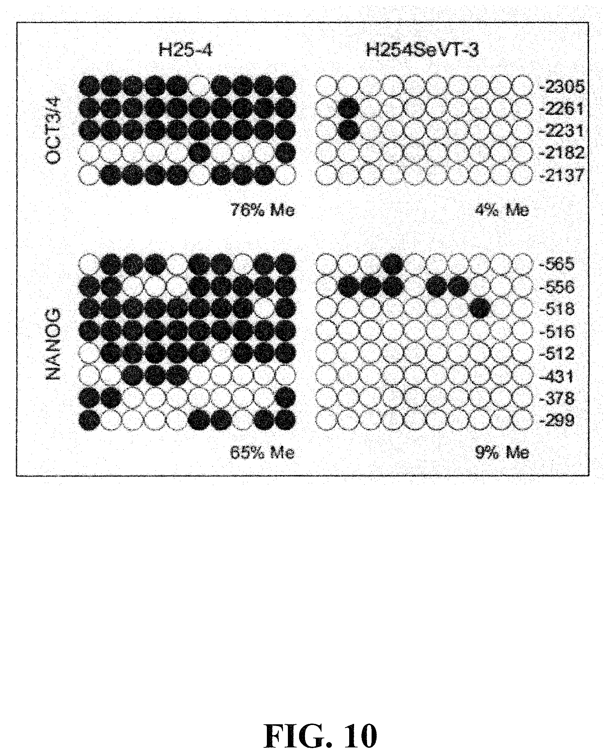

Fig. 10 is a representation showing the results of an analysis by bisulfite sequencing of the promoter region of OCT3/4 and NANOG in H25-4 and H254SeVT-3 cells. In the drawing, the open circles and the filled circles indicate a non-methylated CpG dinucleotide and a methylated CpG dinucleotide, respectively, and "%Me" indicates the percentage of methylation in the respective regions.

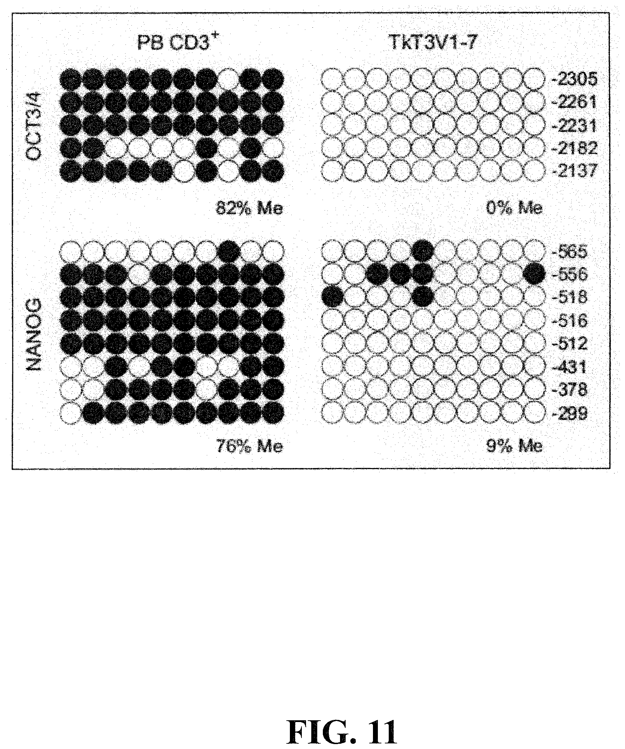

Fig. 11 is a representation showing the results of an analysis by bisulfite sequencing of the OCT3/4 and NANOG promoters in PB CD3+ and TkT3V1-7 cells. In the drawing, the open circles and the filled circles indicate a non-methylated CpG dinucleotide and a methylated CpG dinucleotide, respectively, and "%Me" indicates the percentage of methylation in the respective regions.

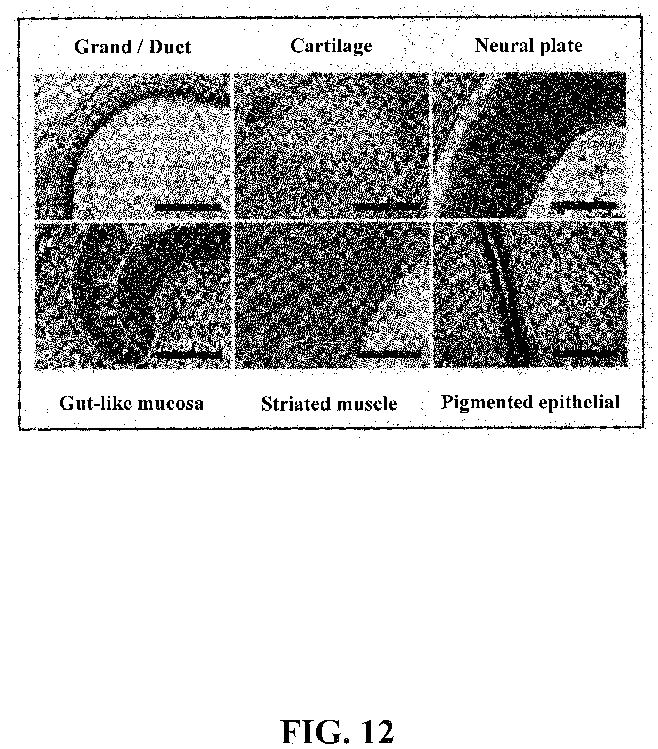

Fig. 12 represents micrographs showing representative results obtained by preparing sections from TkT3V1-7-derived teratomas formed in the testis of NOG mice, followed by HE staining of the sections for observation, which reveal that the teratomas comprise anatomical structures derived from the three germ layers. In these pictures, there are observed glands/ducts and gut-like mucosa as endoderm-derived anatomical structures, cartilage and striated muscle as mesoderm-derived anatomical structures, and neural plate and pigment epithelium as ectoderm-derived anatomical structures. In each of the micrographs, the scale bar corresponds to 100 µm.

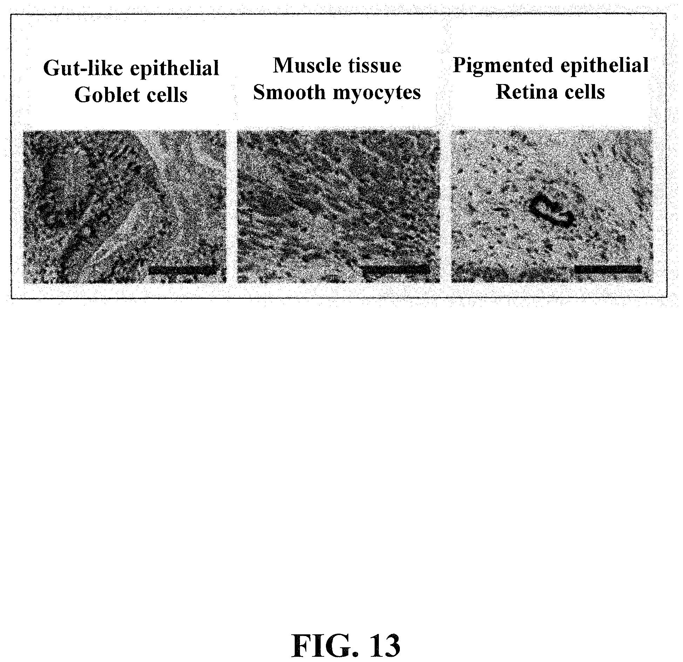

Fig. 13 represents micrographs showing representative results obtained by preparing sections from H254SeVT-3-derived teratomas formed in the testis of NOG mice, followed by HE staining of the sections for observation, which reveal that the H254SeVT-3 cells differentiated into an endoderm-derived cell lineage (goblet cells in gut-like epithelium), a mesoderm-derived cell lineage (smooth myocytes in muscle tissue), and an ectoderm-derived cell lineage (retinal cells in pigment epithelium). In each of the micrographs, the scale bar corresponds to 100 µm.

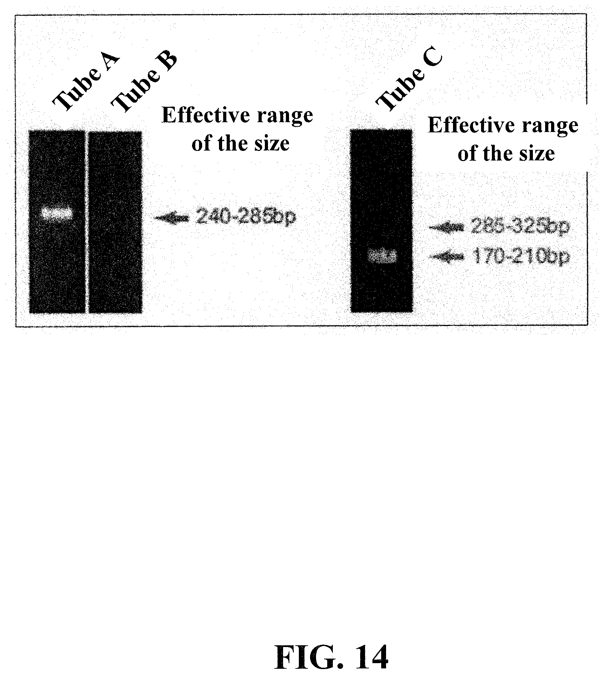

Fig. 14 represents pictures showing a result that a TCRB gene rearrangement in the genome of TkT3V1-7 cells was detected by multiplex PCR analysis. Tubes A and B indicate that the Vβ-(D)Jβ has been rearranged and Tube C indicates that the D-Jβ has been rearranged.

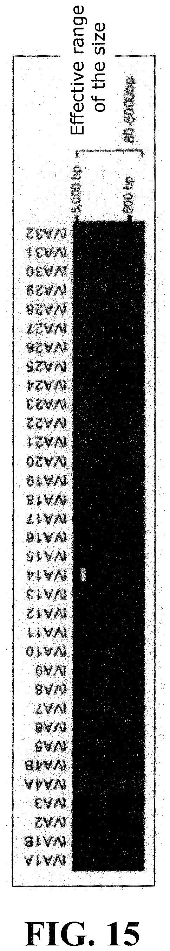

Fig. 15 represents a picture showing a result that a TCRA gene rearrangement (V-Jα rearrangement) in the genome of TkT3V1-7 cells was detected by multiplex PCR analysis.

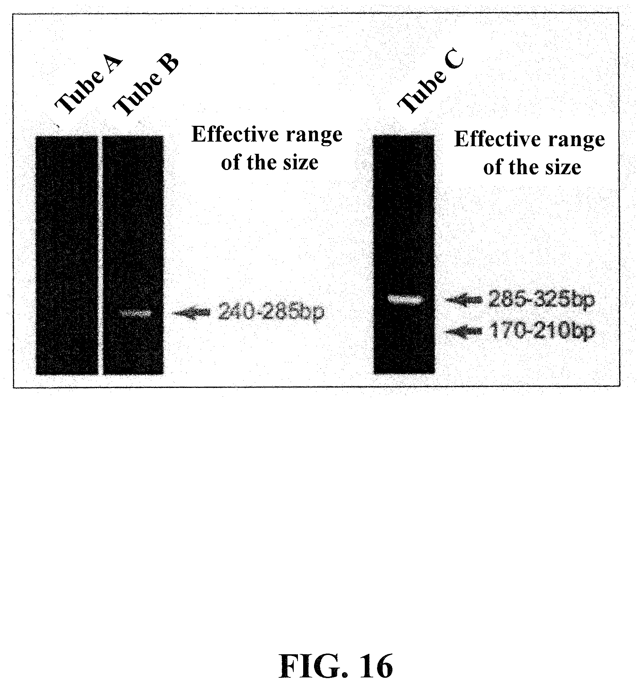

Fig. 16 represents pictures showing a result that a TCRB gene rearrangement in the genome of H254SeVT-3 cells was detected by multiplex PCR analysis. Tubes A and B indicate that the Vβ-(D)Jβ has been rearranged and Tube C indicates that the D-Jβ has been rearranged.

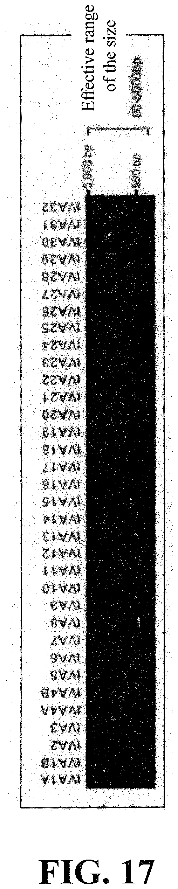

Fig. 17 represents a picture showing a result that a TCRA gene rearrangement (V-Jα rearrangement) in the genome of H254SeVT-3 cells was detected by multiplex PCR analysis.

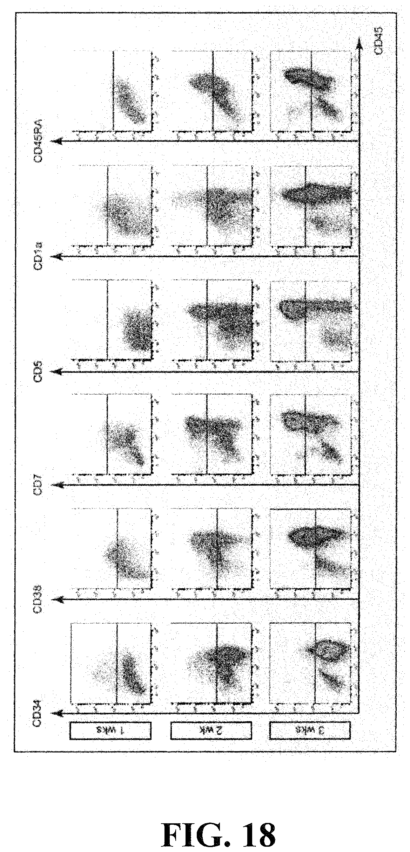

Fig. 18 represents dot plots showing the results of analyzing the course of development from TkT3V1-7-derived CD34+ hematopoietic stem/progenitor cells to human T-lineage cells by flow cytometry as mentioned below. In brief, in order to examine the hematopoietic differentiation potential of T-iPS cells, TkT3V1-7 cells were cultured with VEGF, SCF, and FLT-3L on C3H10T1/2 feeder cells. On day 14 of the culture, the TkT3V1-7-derived CD34+ hematopoietic stem/progenitor cells thus generated (T-iPS-sac cells) were transferred onto OP9 (OP9-DL1) feeder cells expressing delta-like 1. Re-differentiating cells on the OP9-DL1 cells were collected, and then the expression of CD34, CD38, CD7, CD5, CD1a, CD45RA, and CD45 on the cellular surface of TkT3V1-7-derived cells was analyzed by flow cytometry every week for a period of 22 to 36 days after the induction of re-differentiation ("1 wks" to "3 wks" in the drawing).

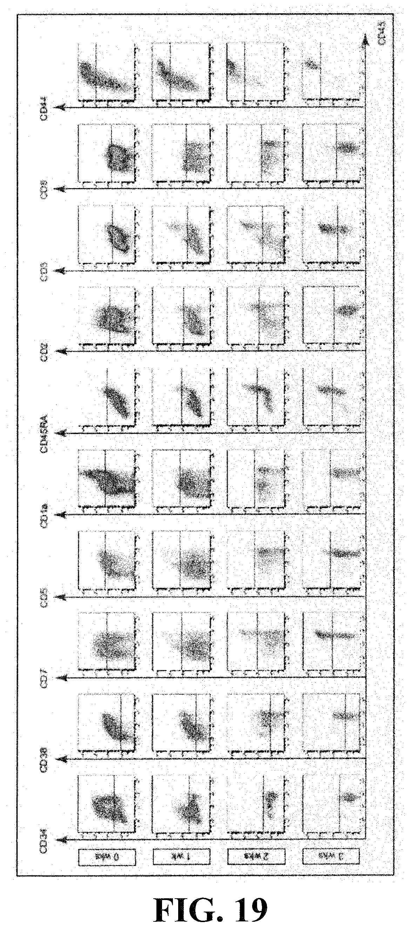

Fig. 19 represents dot plots showing the results of analyzing the course of development from H254SeVT-3-derived T-iPS-sac cells to human T-lineage cells by flow cytometry as mentioned below. In brief, in order to examine the hematopoietic differentiation potential of T-iPS cells, H254SeVT-3 cells were cultured with VEGF, SCF, and FLT-3L on C3H10T1/2 feeder cells. On day 14 of the culture, the TkT3V1-7-derived T-iPS-sac cells thus generated were transferred onto OP9-DL1 feeder cells. Re-differentiating cells on the OP9-DL1 cells were collected, and then the expression of CD34, CD38, CD7, CD5, CD1a, CD45RA, and CD45 on the cellular surface of H254SeVT-3-derived cells was analyzed by flow cytometry every week for a period of 15 to 36 days after the induction of re-differentiation ("0 wks" to "3 wks" in the drawing).

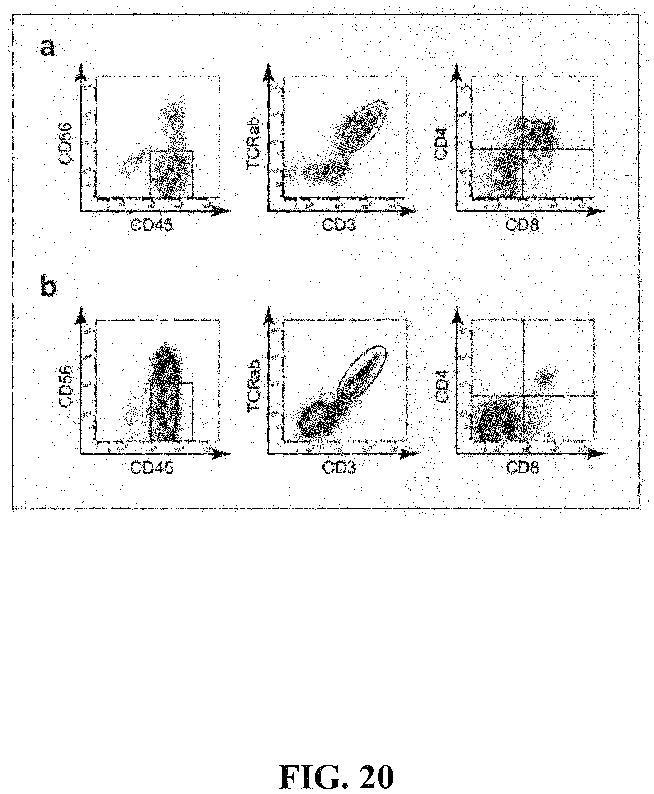

Fig. 20 represents dot plots showing the results of an analysis by flow cytometry of the existence of T-iPS-cell-derived CD4/8 DP cells established *in vitro.* Fig. 20a shows the results of an analysis by flow cytometry of the expression of CD45, CD56, CD3, TCRαβ CD4, and CD8 on the cellular surface of TkT3V1-7-derived cells in a period of 35 to 42 days after the induction of re-differentiation. Fig. 20b shows the results of an analysis by flow cytometry of the expression of CD45, CD56, CD3, TCRαβ, CD4, and CD8 on the cellular surface of H254SeVT-3-derived cells in a period of 35 to 42 days after the induction of re-differentiation. Shown in the drawings are representative results of the results of at least three independent experiments.

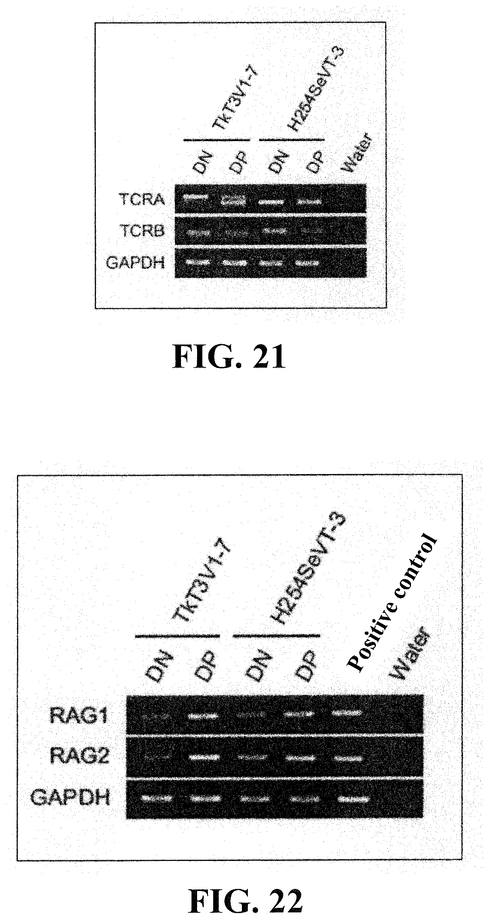

Fig. 21 represents pictures showing the results of RT-PCR amplification of the TCRA and TCRB genes expressed in DN-cell- or DP-cell-derived cDNA libraries synthesized by SMART method. In the drawing, "Water" indicates the results of RT-PCR without added template DNA (as negative control). GAPDH was used as internal standard in each PCR.
Fig. 22 represents pictures showing the results of an analysis by RT-PCR of the expression of RAG1 and RAG2 in DN cells and DP cells. In the drawing, "Water" indicates the results of RT-PCR without added template DNA (as negative control). Samples used as a PCR template were prepared, based on the expression of GAPDH.

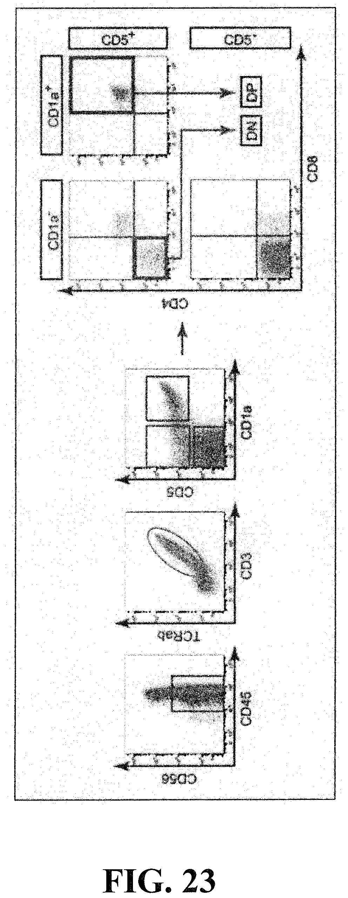

Fig. 23 represents dot plots showing the results of an analysis by flow cytometry of floating cells over OP9-DL1 cells, which were collected on day 42 after the start of the induction of re-differentiation. DN cells and DP cells were sorted from fractionated subsets of CD45+, CD56-, CD3+, TCRαβ+, CD2+, and CD5+ cells, by gating in advance for CD1a- and CD1a+, respectively.

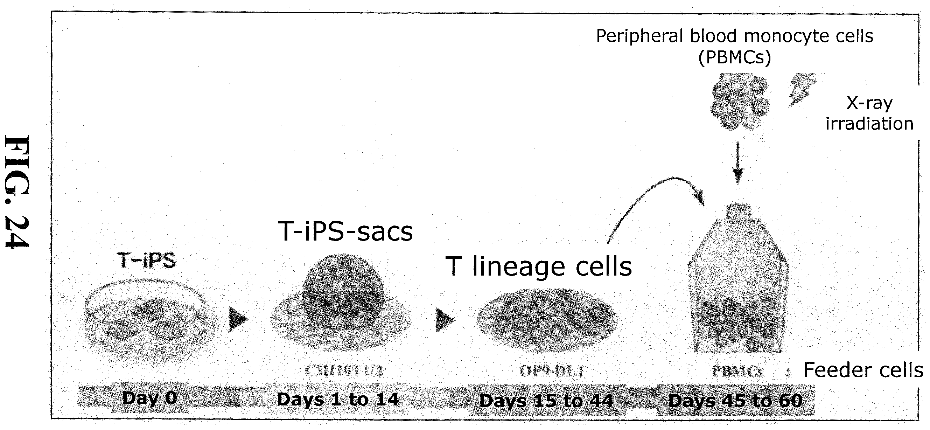

Fig. 24 is a schematic representation showing the method for producing human CD8 SP cells having antigen specificity according to the present invention, in which the process of re-differentiation from T-iPS cells into the human CD8 SP cells is shown.

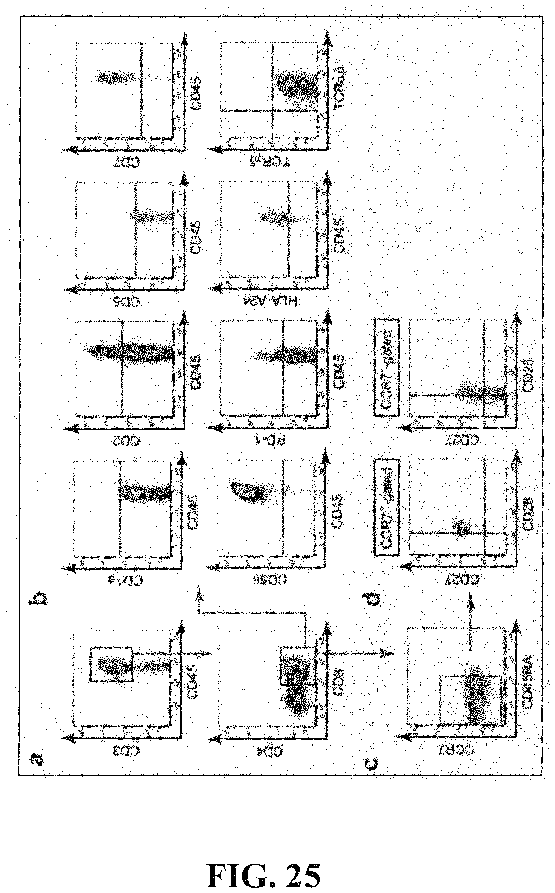

Fig. 25 represents dot plots showing results of an analysis by flow cytometry of the phenotype of cells obtained by re-differentiation of T-iPS cells by the method according to the present invention (cells on days 50 to 60 after the start of the induction of re-differentiation, as shown in Fig. 24). Shown in the drawings are representative results of the results of at least three independent experiments. Figs. 25a and 25b show the results of an analysis of the phenotype as T cell, and Figs. 25c and 25d show the results of an analysis of the phenotype as memory cell. In Fig. 25b, it is also shown that there was no contamination with CD3+/CD8+ cells from PBMCs, because a uniform expression of HLA-A24 was observed.

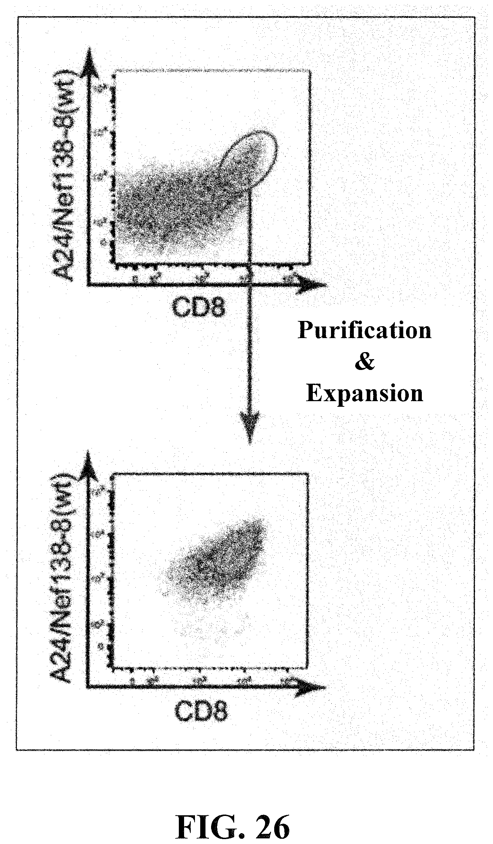

Fig. 26 represents dot plots showing the results of (upper panel) an analysis of cells obtained by re-differentiation of T-iPS cells by the method according to the present invention (cells on days 50 to 60 after the start of the induction of re-differentiation, as shown in Fig. 24), by flow cytometry using A24/Nef-138-8(wt) teratoma, and a re-analysis of T cells expanded by culturing for 14 or more days teratoma-positive cells sorted by FACS or magnetic selection, again by flow cytometry using A24/Nef-138-8(wt) teratoma.

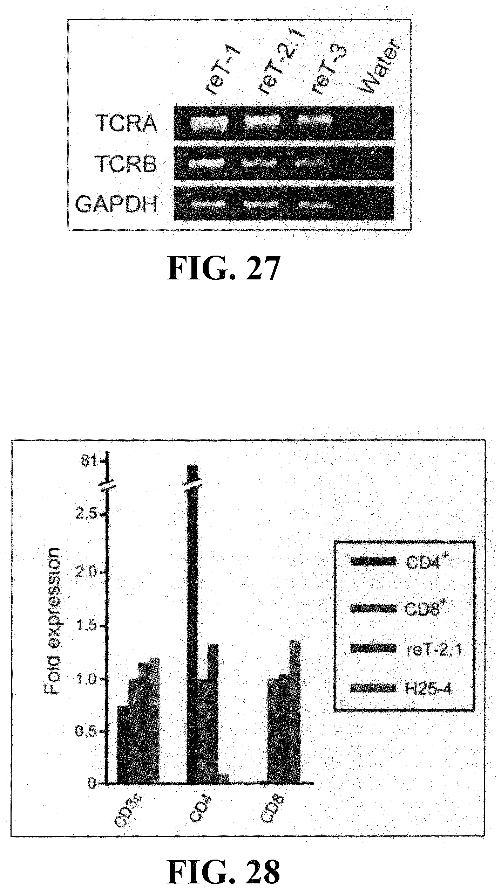

Fig. 27 represents pictures showing that TCR mRNAs were detected by PCR using respective cDNA libraries, synthesized by the SMART method, of various CD8 SP cells (reT-1, reT-2.1, and reT-3) responded to A24/Nef-138-8(wt) teratoma. In the drawing, "Water" indicates the results of RT-PCR without added template DNA (as negative control). GAPDH was used as internal standard in each PCR.
Fig. 28 represents a graph showing the results of comparing the amount of expression of major cell surface molecules among CD4+ cells, CD8+ cells, reT-2.1 cells, and H25-4 cells, using quantitative PCR. In each PCR reaction, the amount of expression of 18S rRNA was used as internal standard. In the drawing, the ordinate axis represents an expression ratio when the amount of expression of each of these genes in the CD8+ cells was set to be 1.0. The abscissa axis represents, from left to right, the results of the respective genes in CD4+ cells, CD8+ cells, reT-2.1 cells, and H25-4 cells.

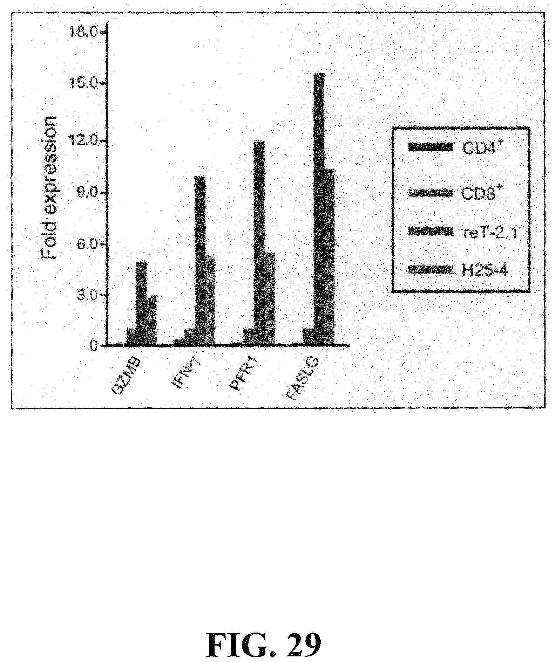

Fig. 29 represents a graph showing the results of comparing the amount of expression of major cytolytic molecules among CD4+ cells, CD8+ cells, reT-2.1 cells, and H25-4 cells, using quantitative PCR. In each PCR reaction, the amount of expression of 18S rRNA was used as internal standard. In the drawing, the ordinate axis represents an expression ratio when the amount of expression of each of these genes in the CD8+ cells was set to be 1.0. The abscissa axis represents, from left to right, the results of the respective genes in CD4+ cells, CD8+ cells, reT-2.1 cells, and H25-4 cells.

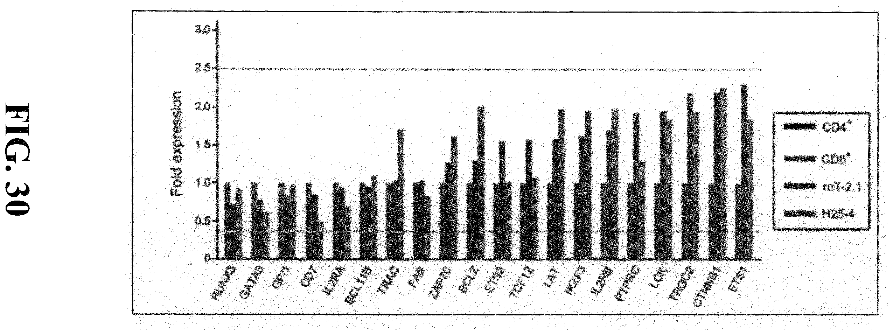

Fig. 30 represents a graph showing the results of comparing the amount of expression of major transcription factors and signaling molecules among CD4+ cells, CD8+ cells, reT-2.1 cells, and H25-4 cells, using quantitative PCR. In each PCR reaction, the amount of expression of 18S rRNA was used as internal standard. In the drawing, the ordinate axis represents an expression ratio when the amount of expression of each of these genes in the CD8+ cells was set to be 1.0. The abscissa axis represents, from left to right, the results of the respective genes in CD4+ cells, CD8+ cells, reT-2.1 cells, and H25-4 cells.

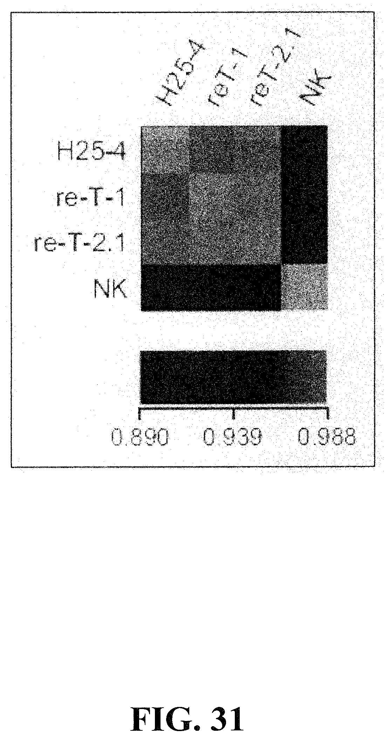

Fig. 31 represents a heat map showing correlation coefficients among samples obtained by a comprehensive analysis of gene expression using cDNA microarrays.

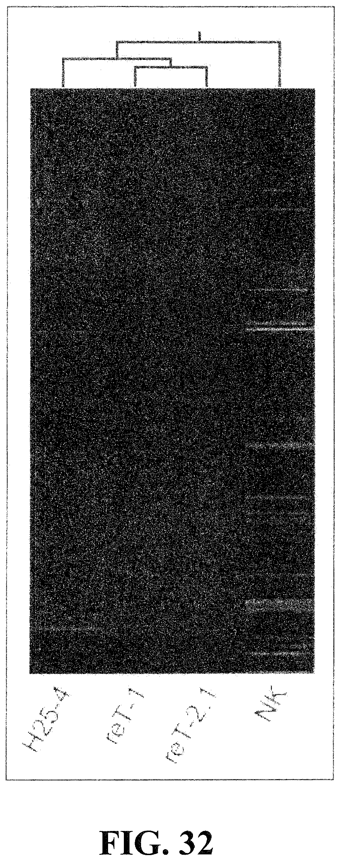

Fig. 32 represents a heat map showing a group of genes exhibiting a more than three-times difference in their expression amount, relative to NK cells, which was obtained by a comprehensive analysis of gene expression using cDNA microarrays. In the drawing, the reds and greens indicate an increase and a decrease in expression, respectively.

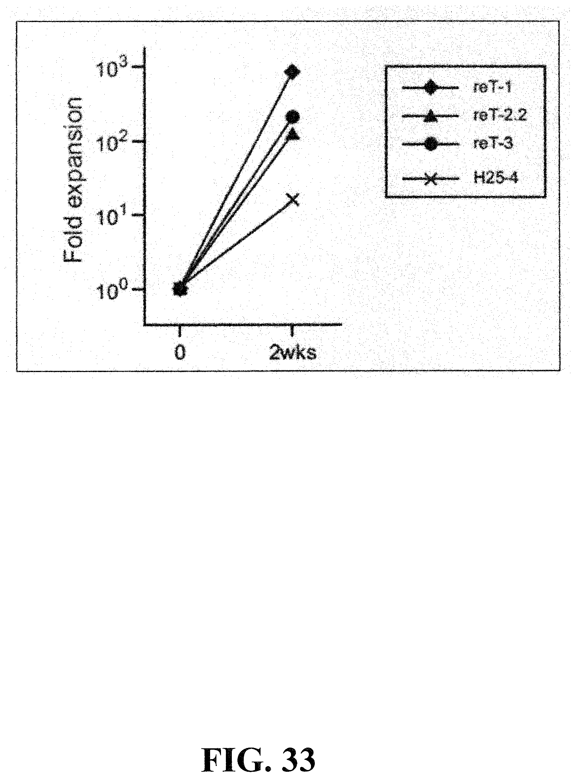

Fig. 33 represents a graph showing the expansion ratio of reT-1, reT-2.2, and reT-3 cells at two weeks after the respective cells were stimulated with PHA, IL-7, and IL-15. The ordinate axis represents an expansion ratio when the number of cells upon the stimulation was set to be 100 for the respective cells.

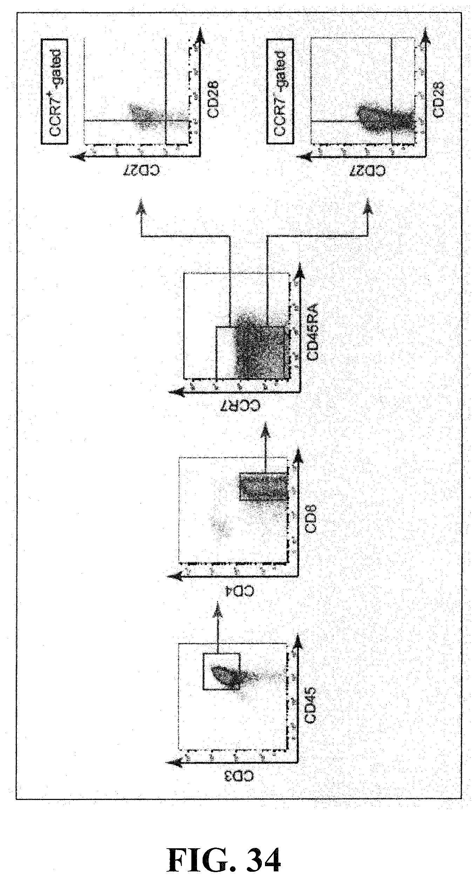

Fig. 34 represents dot plots showing the results of an analysis by flow cytometry of the expression of CD45RA, CCR7, CD27, and CD28 on the cellular surface of the cells purified with A24/Nef-138-8(wt) tetramer and expanded (reT-2.1 and reT-3). In the drawings, the data of reT-2.1 cells are shown as representative results.

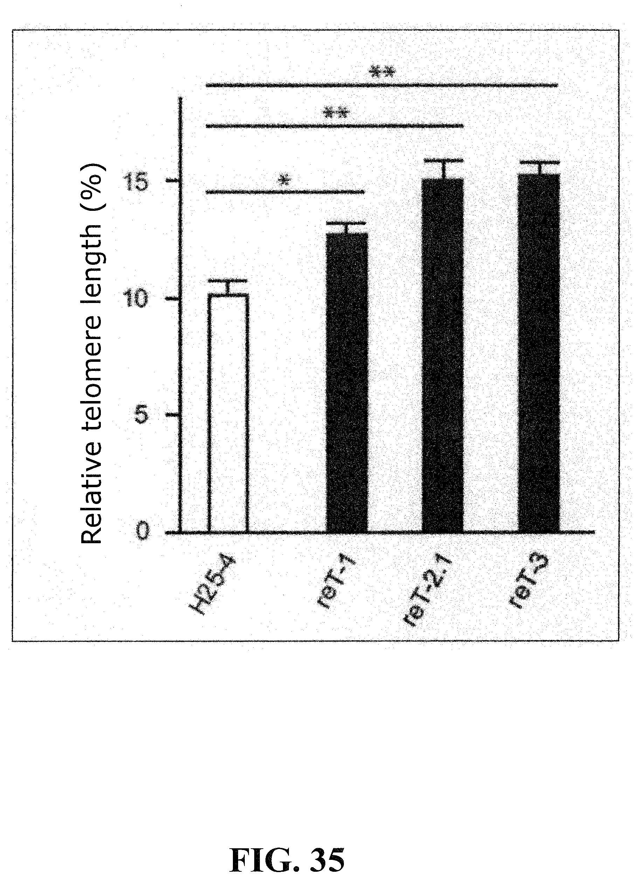

Fig. 35 represents a graph showing the relative telomere length (RTL) as determined by flow-FISH.

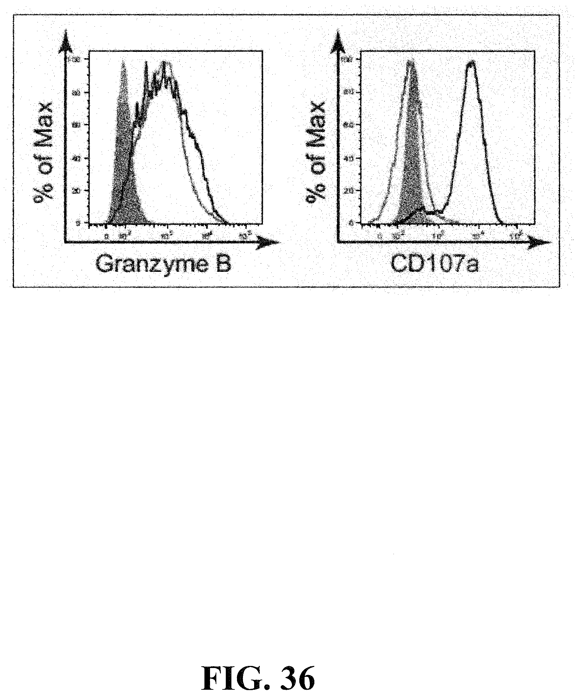

Fig. 36 represents histograms showing the results of an analysis of the secretion of cytolytic molecules in reT-2.1 cells when stimulated with α-CD3/CD28 beads. The left panel is a histogram showing the amount of intracellular production of granzyme B. The open histogram represented by the grey line indicates the reactivity of cells which were not stimulated and an anti-granzyme B antibody, the open histogram represented by the black line indicates the reactivity of cells which were stimulated and an anti-granzyme B antibody, and the closed histogram indicates the reactivity of cells which were stimulated and an antibody used as a negative control (an antibody of the same isotype as that of the anti-granzyme B antibody). The right panel is a histogram showing the results of a CD107a mobilization assay in reT-2.1 cells when stimulated with α-CD3/CD28 beads. The open histogram represented by the grey line indicates the reactivity of cells which were not stimulated and an anti-CD107a antibody, the open histogram represented by the black line indicates the reactivity of cells which were stimulated and an anti-CD 107a antibody, and the closed histogram indicates the reactivity of cells which were stimulated and an antibody used as a negative control (an antibody of the same isotype as that of the anti-CD 107a antibody).

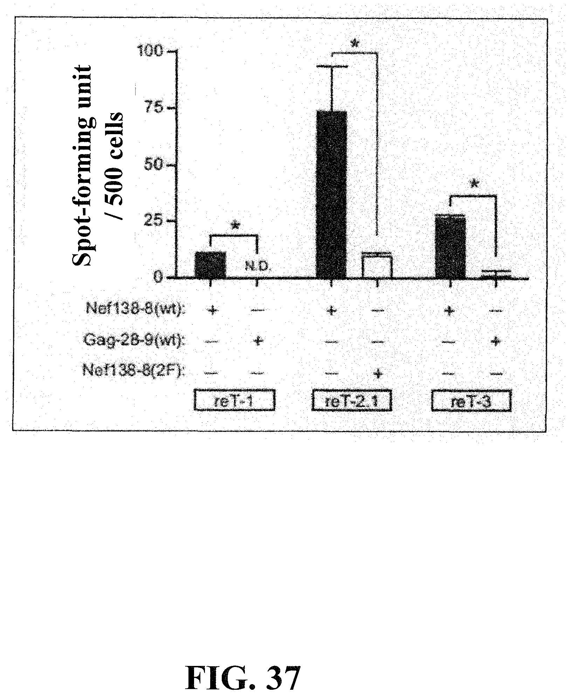

Fig. 37 represents a graph showing the results of an analysis by ELISPOT of IFN-γ produced in response to stimulation with Nef-138-8(wt). Shown in the drawing are representative results of the results of at least three independent experiments. The ordinate axis represents the number of spots formed in 500 cells.

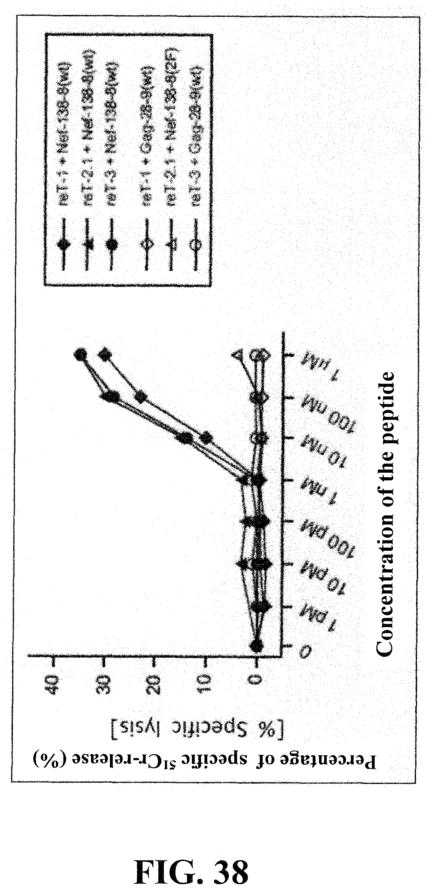

Fig. 38 represents a graph showing the results of a 51Cr release assay using various concentrations of epitope peptides, including Nef-138-8(wt). The 51Cr release assay was performed by reacting effector cells and 51Cr-incorporated target cells at a ratio of 5:1. The ordinate axis represents the percentage of specific 51Cr-release (%).

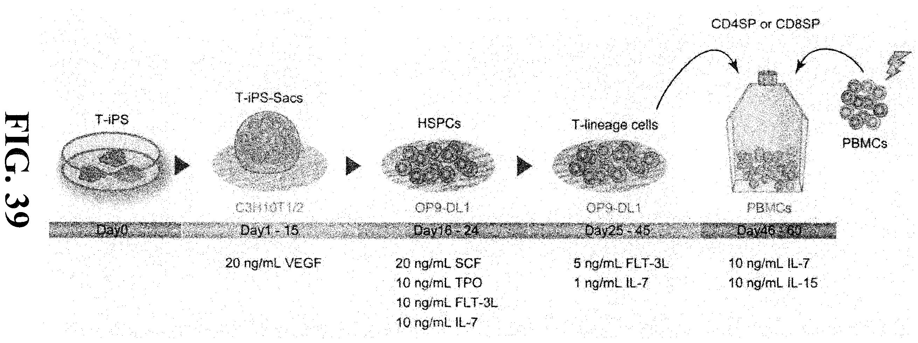

Fig. 39 represents an outline of the method for producing a human CD4 SP cell having antigen specificity from a T-iPS cell.

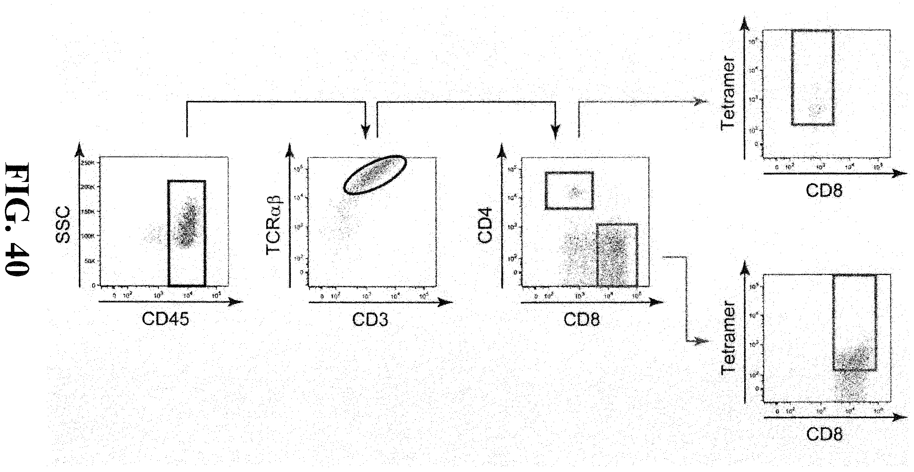

Fig. 40 represents dot plots showing the result of an analysis by flow cytometry of the phenotype of the cells obtained by re-differentiation of T-iPS cells and the cells on days 50 to 60 after the start of the induction of re-differentiation, as shown in Fig. 39), which is the result of an analysis of phenotype of the obtained T cells with several surface expression markers and the result of an analysis by flow cytometry using A24/Nef-138-8(wt) tetramer and was a representative result among the at least triplicated experiments.

### DETAILED DESCRIPTION OF THE INVENTION

### <A method for producing a human CD8 SP cell or CD4 SP cell having antigen specificity>

The invention provides a method for producing an antigen specific T cell which is a method for producing a human CD8 and/or CD4 single-positive cell having antigen specificity, which comprises the steps of: differentiating an iPS cell that has been induced from a human T cell (hereinafter referred to as "T-iPS cells"), into a CD4/CD8 double-negative cell wherein the human T cell has antigen specificity, stimulating a T cell receptor of the CD4/CD8 double-negative cell, and differentiating the CD4/CD8 double-negative cell whose T cell receptor has been stimulated, into a single positive T cell, which is a CD8 single-positive cell and/or a CD4 single-positive cell.

In the present invention, a "human" from which T cells are to be isolated is not limited in particular. Such a human may be a healthy individual, an individual with diminished immune function, or an individual suffering from a malignant tumor, infection, autoimmune disease, or the like. In cases where a CD8 SP cell or a CD4 SP cell obtained according to the present invention are used for a method for cell-based immunotherapy, a human from which T cells are to be isolated preferably has an HLA type identical to that of a human to which T cells obtained according to the present invention are to be administered, and more preferably is the same as a human to which T cells obtained according to the present invention are to be administered, from the viewpoint that rejection is not caused.

In the present invention, a "T cell" refers to a cell on the surface of which an antigen receptor, called a T cell receptor (TCR), is expressed, and its progenitor cells, such as a pro-T cell on which a TCR (TCRαβ) is not expressed, and a pre-T cell on which a TCRβ and a pre-TCRα are associated. A "CD4/CD8 double-negative (DN) cell" refers to a T cell on which neither CD4 nor CD8 is expressed, a "CD4/CD double-positive (DP) cell" refers to a T cell on which both CD4 and CD8 are expressed, and a "CD8 single-positive (SP) cell" refers to a T cell on which only CD8, in terms of CD4 and CD8, is expressed. A "CD4 single-positive (SP) cell" refers to a T cell on which only CD4, in terms of CD4 and CD8, is expressed.

In the present invention, a "T cell which is to be induced into an iPS cell" is of a type of T cell on which CD3 and CD8 are expressed and includes a cytotoxic T cell which is a CD8 positive. A "T cell which is to be induced into an iPS cell" is also of a type of T cell on which CD3 and CD4 are expressed and includes a T cell which is a CD4 positive. Human T cells of such a type include, for example, helper/regulatory T cells ,which are a subset of CD4-positive cells; cytotoxic T cells (CTLs), which are a subset of CD8-positive cells; naive T cells (CD45RA+CD62L+ cells); central memory T cells (CD45RA-CD62L+ cells); effector memory T cells (CD45RA-CD62L- cells); and terminal effector T cells (CD45RA+CD62L- cells). Note that the antigen specificity of T cells is provided by their antigen-specific, rearranged TCR gene. From the viewpoint of production efficacy, an antigen specific CD8 positive T cell, but not limited thereto, can be used as a human T cell to be induced into an iPS cell in order to obtain an antigen specific CD8 SP cells, and an antigen specific CD4 positive T cell, but not limited thereto, can be used as a human T cell to be induced into an iPS cell in order to obtain an antigen specific CD4 SP cells. In cases of carrying out immunotherapy as mentioned below, it is preferable that a human T cell which is differentiated from the iPS cell has the same or substantially the same antigen specificity as that exhibited by a T cell which is to be induced into the iPS cell.

In the present invention, a "T cell which is to be induced into an iPS cell" can be isolated from a human tissue by known procedures. Such a human tissue is not limited in particular, if the tissue contains T cells of the above-mentioned type, and examples thereof include, for example, peripheral blood, a lymph node, bone marrow, thymus, spleen, umbilical cord blood, and a lesion site tissue. Among these, peripheral blood and umbilical cord blood are preferable from the viewpoints that their isolation is less invasive to the human body and that they are prepared with ease. Known procedures for isolating human T cells include, for example, flow cytometry using an antibody directed to a cell surface marker, such as CD4 or CD8, and a cell sorter, as shown in the below-mentioned Example. In addition, a desired type of T cell can be isolated using the secretion of cytokines or the expression of functional molecules as an indicator. In this case, for example, T cells secrete different cytokine, depending on whether they are of the Th1 or Th2 type, and thus T cells of a desired Th type can be isolated by selecting T cells using such a cytokine as an indicator. Similarly, cytotoxic (killer) T cells can be isolated using the secretion or production of granzyme, perforin, or the like as an indicator. An affinity column on which the desired antigen is immobilized, or the like can be used in isolating a "human T cell having antigen specificity" from a human tissue. Methods can be also employed in which "T cells having a desired antigen specificity" are purified from a human tissue using a multimer in which an MHC (major histocompatibility complex) having the desired antigen attached thereto, for example, an "MHC tetramer", or "Pro5TM MHC Class I Pentamer".

An "iPS cell" in the present invention is a cell which is also called an induced pluripotent stem cell, and can be induced by introducing a cell reprogramming factor into T cells of the above-mentioned type. The "cell reprogramming factor" is not limited in particular, if the factor is one which is capable of conferring the pluripotency on somatic cells, alone or in cooperation with other factors for pluripotency, by its introduction into T cells of the above-mentioned type, and preferably is at least one protein selected from the group consisting of Oct3/4, c-Myc, Sox2, Klf4, Klf5, LIN28, Nanog, ECAT1, ESG1, Fbxl5, ERas, ECAT7, ECAT8, Gdf3, Soxl5, ECAT15-1, ECAT15-2, Fthl17, Sal14, Rex1, Utf1, Tcl1, Stella, β-catenin, Stat3, and Grb2. Further, it is more preferable that among these proteins, Oct3/4, c-Myc, Sox2, and Klf4 ("4 factors") are introduced into T cells of the above-mentioned type, from the viewpoint that iPS cells can be efficiently established using a small number of factors. As is shown in WO2011/096482, OCT4, SOX2, KLF4, C-MYC and NANOG are preferably introduced into a human CD8 positive T cell or CD4 positive T cell, and OCT4, SOX2, KLF4, C-MYC, NANOG and LIN28 are more preferably introduced into a human CD8 positive T cell or CD4 positive T cell. In addition, it is more preferable that Oct3/4, Sox2, and Klf4 ("3 factors"), with c-Myc being not included, are introduce into T cells of the above-mentioned type, from the viewpoint of reducing the risk of malignant transformation of the resulting pluripotent stems.

In the present invention, methods for establishing an iPS cells induced from a human T cell (i.e., methods for introducing a cell reprogramming factor into T cells) are not limited in particular and known procedures can be selected and used as appropriate. For example, when a cell reprogramming factor as described above is introduced into T cells of the above-mentioned type in the form of proteins, such methods include ones using protein introducing reagents, fusion proteins with protein transfer domains (PTDs), electroporation, and microinjection. When a cell reprogramming factor as described above is introduced into T cells of the above-mentioned type in the form of nucleic acids encoding the cell reprogramming factor, a nucleic acid(s), such as cDNA(s), encoding the cell reprogramming factor can be inserted in an appropriate expression vector comprising a promoter that functions in T cells, which then can be introduced into T cells of the above-mentioned type by procedures using infection, lipofection, liposomes, electroporation, calcium phosphate co-precipitation, DEAE-dextran, microinjection, and electroporation.

Examples of an "expression vector" in context of the present invention include viral vectors, such as lentiviruses, retroviruses, adenoviruses, adeno-associated viruses, herpes viruses, and sendai virus; and expression plasmids for animal cells. Sendai virus (SeV) is preferably used to introduce a nucleic acid(s) encoding a cell reprogramming factor as described above into T cells of the described-above type, from the viewpoints that insertion mutations are unlikely to be caused, that a high efficiency of gene introduction is achieved, and that an increased number of copies of gene(s) introduced is obtained.

Promoters which are used in such expression vectors include, for example, SRa, SV40, LTR, CMV, RSV, HSV-TK promoters, and others. Such promoters may be ones which can regulate the expression of a gene inserted downstream of the promoter, for example, depending upon the presence or absence of an agent such as tetracycline. Expression vectors further may contain an enhancer, a poly adenylation signal, a selective maker gene (for example, neomycin resistance gene), an SV40 origin of replication, or others, in addition to the promoter.

In the step of inducing an iPS cell from the human T cell induced from a human T cell, it is preferable that before a cell reprogramming factor as described above is introduced, T cells of the above-mentioned type are activated by stimulation with an anti-CD3 antibody and an anti-CD28 antibody in the presence of interleukin 2 (IL-2), as shown in the below-mentioned Example. The T cell can be stimulated and then activated with at least one substance selected from the group consisting of phytohemagglutinin (PHA), interleukin 2 (IL-2), alloantigen expressing cells, an anti-CD3 antibody and an anti-CD28 antibody, as shown in the below-mentioned Example. Such stimulation can be achieved, for example, by adding PHA, IL-2, an anti-CD3 antibody and/or an anti-CD28 antibody to a medium in which T cells of the above-mentioned type are then cultured for a specified period of time, as shown in the below-mentioned Example. In this case, the anti-CD3 antibody and the anti-CD28 antibody may be ones each having been attached to magnetic beads. Further, such stimulation may be achieved by culturing T cells of the above-mentioned type for a specified period of time in a culture dish to the surface of which an anti-CD3 antibody and an anti-CD28 antibody are attached, instead of adding these antibodies to the medium. Further, stimulation of T cells may be also achieved by adding an antigen peptide recognized by the human T cells, together with feeder cells to the medium.

In order to achieve such stimulation as described above, the concentrations of PHA and IL-2 added to the medium are not limited in particular, and preferably are 1 to 100 µg/mL and 1 to 200 ng/mL, respectively. The concentrations of an anti-CD3 antibody and an anti-CD28 antibody added to the medium in such stimulation as described above are not limited in particular, and preferably are one to ten times the amount of culture of the T cells of the above-mentioned type. The concentrations of an anti-CD3 antibody and an anti-CD28 antibody attached to the surface of culture dishes in such stimulation as described above are not limited in particular, and their concentrations in coating processes preferably are 0.1 to 10µg/mL, more preferably 1 to 100 µg/mL for an anti-CD3 antibody and 0.1 to 10 µg/mL for an anti-CD28 antibody.

The culture period for such stimulation is not limited in particular, if the culture period is a period of time which is sufficient to stimulate T cells of the above-mentioned type and which allows the T cells to proliferate to a number of cells which is necessary for introduction of a cell reprogramming factor as described above, and usually is 1 to 14 days, or 1 to 6 days, although the culture period may be one day. From the viewpoint of the efficiency of gene introduction, the culture period is preferably 2 to 7 days and more preferably 2 to 4 days. Further, it is preferable that the culture is carried out in culture dishes having RetroNectin coated thereon, from the viewpoint that the efficiency of gene introduction is increased.

Media in which T cells of the above-mentioned type are cultured and to which PHA, IL-2, an anti-CD3 antibody, and/or an anti-CD28 antibody, and others are added can be, for example, known media suitable for culturing the T cells, more specifically Roswell Park Memorial Institute (RPMI) 1640 medium, minimal essential medium (a-MEM), Dulbecco's modified Eagle medium (DMEM), F12 medium, and others containing other cytokines and human serum. These media may be supplemented with amino acids necessary for culture (for example, L-glutamine), and antibiotics (for example, streptomycin, penicillin), besides PHA, IL-2, an anti-CD3 antibody and/or an anti-CD28 antibody. IL-7 and IL-15 are preferably added to a medium, from the viewpoint that apoptosis is suppressed. The concentrations of IL-7 and IL-15 added are not limited in particular, and preferably are 1 to 100 ng/mL for each of IL-7 and IL-15.

Conditions in or after "introducing a cell reprogramming factor into T cells" in the present invention are not limited in particular. However, the T cells into which a cell reprogramming factor as described above has been introduced are preferably cultured on a layer of feeder cells. These feeder cells are not limited in particular, and include, for example, mouse embryonic fibroblasts (MEFs), STO cells, and SNL cells in which the cell division has been arrested by irradiation or antibiotic treatment.

Further, in the process of inducing from T cells of the above-mentioned type into iPS cells, it is preferable that basic fibroblast growth factor (bFGF) is added to the medium in or after introducing a cell reprogramming factor as described above into the T cells, from the viewpoint that cell differentiation is suppressed.

In addition, in order to achieve a higher efficiency of establishing iPS cells, it is preferable that a histone deacetylase (HDAC) inhibitor (for example, valproic acid (VPA), trichostatin A, sodium butyrate, MC 1293, M344, and other low-molecular inhibitors; an siRNA directed to HDAC), a G9a histone methyltransferase inhibitor (for example, BIX-01294 and other low-molecular inhibitors; an siRNA directed to G9a), or a p53 inhibitor (for example, Pifithrin-α (PFT-α) and other low-molecular inhibitors; an siRNA directed to p53) is added to the medium in or after introducing a cell reprogramming factor as described above into T cells of the above-mentioned type.

Further, in "introducing a cell reprogramming factor as described above into T cells," it is preferable that, when a virus vector is used, protamine sulfate is added to the medium, from the viewpoint of facilitating binding of the vector to T cells of the above-mentioned type.

In addition, it is preferable that in concert with the transition from T cells of the above-mentioned type to iPS cells, culturing is performed while the medium is gradually replaced from a known medium suitable for culturing the T cells to a medium suitable for culturing the iPS cells, as shown in the below-mentioned Example. As a medium suitable for such culturing of the iPS cells, known media can be selected and used as appropriate; such media include, for example, Dulbecco's modified Eagle medium/F12 medium containing KnockOut Serum Replacement, L-glutamine, non-essential amino acids, 2-mercaptoethanol, b-FGF, and the like (human iPS cell medium).

As shown in International Publication No. WO 2011/096482, in cases of inducing human CD8-positive T cells or CD4-positive T cell into iPS cells, it is preferable that after transferring onto the above-mentioned feeder cells, culturing is performed under conditions of low oxygen concentrations (an oxygen concentration of 5%, for example) in a medium to which 1 to 1,000 µM of a ROCK inhibitor is further added, and it is also preferable that 1 to 1,000 µM of an MEK inhibitor (for example, PD0325901) and 1 to 1,000 µM of a GSK3 inhibitor (for example, CHIR99021) are added to the medium until there is formation of colonies as mentioned below, from the viewpoint that a higher efficiency of induction into iPS cells is achieved, as shown in the below-mentioned Example.

Selection of iPS cells thus induced from T cells of the above-mentioned type (hereinafter, also referred to as "T-iPS cells") can be performed by selecting known procedures as appropriate. Such known procedures include, for example, methods in which selection is performed by observation under a microscope of the morphology of ES/iPS-cell-resembling colonies, as shown in the below-mentioned Example, and methods in which selection is performed using recombinant T cells in which a drug-resistance gene or reporter gene (GFP gene or the like) has been targeted to the locus of a gene known to be expressed specifically in iPS cells (for example, a cell reprogramming factor as described above), whereby the drug resistance or reporter activity is used as an indicator.

The fact that cells thus selected are iPS cells (i.e., T-cells) can be ascertained, for example, by methods in which the expression in the selected cells of markers which are specific in undifferentiated cells (such as ALP, SSEA-4, Tra-1-60, and Tra-1-81) is detected by means of immunostaining, RT-PCR, and others, or in which the selected cells are implanted into mice and then observation is made as to whether the formation of teratoma is caused by the implanted cells, as shown in the below-mentioned Example. It can be confirmed by detecting rearrangement of TCR genes by means of genomic PCR that the cells thus selected are derived from T cells, as shown in the below-mentioned Example.

The time at which these cells are selected and harvested can be determined as appropriate with observing the condition of colony growth, and approximately 10 to 40 days, preferably 14 to 28 days, after a cell reprogramming factor as described above were introduced into T cells of the above-mentioned type. Culture conditions preferably are those of 5% CO2 and at 35 to 38°C, more preferably 37°C, unless otherwise specified above.

### <Step of differentiating a T-iPS cell into a CD4/CD8 DN cell>

In order to differentiate the iPS cells (T-iPS cells) which have been induced from human T cells into CD4/CD8 DN cells, it is preferable that the T-iPS cells are first cultured on feeder cells (preferably stroma cells, more preferably human stroma cells) in a medium which may contains cytokines, which is optionally added, serum (for example, fetal bovine serum), insulin, transferin, sodium selenite, L-glutamine, α-monothioglycerol, ascorbic acid, or the like, from the viewpoint of facilitating the induction of differentiation into mesodermal cells. Stroma cells which are to be used are preferably OP9 cells and 10T1/2 cells, such as C3H10T1/2 cells, treated by irradiation or the like, from the viewpoint of facilitating the induction of differentiation into hematopoietic cells. In cases of efficiently inducing the T-iPS cells into CD34-positive hematopoietic progenitor cells, the cytokine to be added into a medium is preferably at least one cytokine selected from the group consisting of VEGF, SCF, TPO, SCF, and FLT3L, and more preferably VEGF, SCF and TPO, or VEGF, SCF and FLT3L. Media include, for example, X-VIVO medium, Iscove's Modified Dulbecco's Medium (IMDM medium), α-MEM, or DMEM; IMDM medium is preferable from the viewpoint of resulting in a high efficiency of formation of sac structures containing hematopoietic progenitor cells and the like (also referred to as "T-iPS-sacs" or "ES-sacs"). The culture period of T-iPS cells preferably is a period of time until T-iPS-sacs are formed, and preferably 8 to 14 days, more preferably 10 to 14 days, after culturing of the T-iPS cells is started. Culture conditions are not limited in particular, and preferably are conditions of 5% CO2 and at 35 to 38°C, more preferably 37°C. In addition, it is more preferable that the T-iPS cells are cultured under conditions of low oxygen concentrations (an oxygen concentration of 5 to 20%, for example), from the viewpoints that a high efficiency of formation of T-iPS-sacs is achieved and that an increased number of blood cells contained in the T-iPS-sacs is obtained.

In order to differentiate a T-iPS cell into CD4/CD8 DN cell, cells which are contained in T-iPS-sacs obtained as described above, such as hematopoietic progenitor cells, CD34-positive cells, and blood cells, preferably are cultured on feeder cells (preferably stroma cells and more preferably human stroma cells) in a medium containing cytokines and serum (such as, FBS). Cells encapsulated within the T-iPS-sacs can be separated by physical means, for example, by passing the cells through sterilized sieve-like apparatus (for example, cell strainers and others). Stroma cells used in the above-mentioned culture preferably are OP9-DL1, OP9-DL4, 10T1/2/DL4, or 10T1/2/DL1 cells treated by irradiation or the like, from the viewpoint that the induction of differentiation into T lymphocytes is achieved through notch signals. The cytokines which are added to the medium include, for example, IL-7, FLT3L, VEGF, SCF, TPO, IL-2, and IL-15. One or more cytokines selected from a group consisting of SCF, TPO, FLT3L and IL-7 and a combination of all of these cytokines can be added to the medium used in the induction of differentiation into CD4 SP cells. Among these cytokines, preference is given to IL-7 and FTL3 from the viewpoint of supporting the T cell differentiation at the early stage. However, it is preferable that SCF is not contained in the medium, from the viewpoint that the differentiation of the T-iPS cells into NKT cells is suppressed. The concentration of IL-7 added to the medium is preferably 0.1 to 9.5 ng/mL, more preferably 1 to 4 ng/mL, from the viewpoints of easily generating CD3-positive CD56-negative T-lineage cells and facilitating the induction of differentiation into CD8 SP T cells or CD4 SP T cells. The concentration of FLT3L added to the medium is preferably 0.1 to 100 ng/mL from the viewpoints of improving the proliferation of blood cells. Media include, for example, α-MEM medium, DMEM medium, and IMDM medium, with α-MEM medium being preferable from the viewpoint of easily maintaining feeder cells. These media may be supplemented with amino acids necessary for culture (for example, L-glutamine), and antibiotics (for example, streptomycin, penicillin), besides IL-7 and FLT3.

The culture period described above for culturing cells which are contained in the T-iPS-sacs is preferably a period of time until T cell receptor (TCR) is expressed on the cellular surface of CD4/CD8 DN cells obtained by differentiation as described above, and is preferably 14 to 28 days after culturing of cells contained in the T-iPS-sacs is started. Culture circumstances are not limited in particular, and preferably are under conditions of 5% CO2 and at 35 to 38°C, more preferably 37°C.

Whether or not T cell receptor (TCR) is expressed on the cellular surface of the resulting CD4/CD8 DN cells can be assessed by flow cytometry using an anti-TCRap antibody, an anti-CD3 antibody, an anti-CD4 antibody, and an anti-CD8 antibody, as shown in the below-mentioned Comparative Example 1 (see Fig. 20).

### <Step of stimulating a T cell receptor of CD4/CD8 DN cells>

As shown in the below-mentioned Comparative Example 1, the present inventors have demonstrated for the first time that, in T cells which have been redifferentiated from the T-iPS cells, the CD4/CD8 DP stage exhibits more intense expression of RAG1 and RAG2 than the CD4/CD8 DN stage. Further, the present inventors have demonstrated for the first time that, in TCRA mRNAs from T cells into which the T-iPS cells have been redifferentiated, the DP stage has a higher frequency of having a TCR-gene rearrangement pattern different from that in their originating human T cells than the DN stage has. These strongly suggest that further rearrangement of the TCRA gene (receptor revision) is caused by RAG1 and RAG2 in the process of redifferentiating the T-iPS cells into T cells, particularly in the process leading from the CD4/CD8 DN stage to the CD4/CD8 DP stage.

As shown in the below-mentioned Comparative Example 1, the present inventors have also demonstrated for the first time that, in T cells obtained by redifferentiation of the T-iPS cells, TCR is expressed even at the DN stage. In a normal living body, T cells do not express TCR at the DN stage. Therefore, it was surprised that TCRs are expressed in DN cells obtained by differentiation of the T-iPS cells.

Until now, it has been known that, in the process of positive selection, TCR signals via peptide-MHC complexes arrest the expression of the RAG genes and suppress further rearrangement of the TCR gene. It has been also shown that TCR-signal-like signals by anti-CD3 antibodies exert similar effects (i.e., suppressing further rearrangement of the TCR gene). The present inventors have succeeded in suppressing further rearrangement of the TCR gene by stimulating the TCR which is expressed on DN cells induced from the T-iPS cells, and based on this, improving the frequency of appearance of T cells having the same rearrangement pattern of the TCR gene as in their originating human T cells.

Therefore, in the method for producing a human CD8 SP cell and/or human CD4 SP cell having antigen specificity according to the present invention, stimulating T-iPS-cell-derived CD4/CD8 DN cells via the TCR expressed on their cellular surface can suppress further rearrangement of the TCR gene, and eventually makes it possible that in CD8 SP cells and/or CD4 SP cells obtained by redifferentiation, there is achieved a highly increased frequency of appearance of T cells having the same rearrangement pattern of the TCR gene as in their originating human T cells.

As a method for stimulating the T cell receptor of T-iPS-cell-derived CD4/CD8 DN cells, preference is given to methods of contacting T-iPS-cell-derived CD4/CD8 DN cells with at least one substances selected from the group consisting of PHA, an anti-CD3 antibody, an anti-CD28 antibody, a cell which expresses a complex of an antigen peptide to which the originating human T cells of the T-iPS cells binds specifically and an HLA restricted to the T cell receptor, an MHC multimer having the antigen peptide attached thereto, PMA, and ionomycin; more preference is given to methods in which cells expressing a specific peptide/HLA complex are contacted with T-iPS-cell-derived CD4/CD8 DN cells, from the viewpoint that physiological stimulation is applied. From the viewpoint of attaching importance to the uniformity of stimulation, more preference is given to methods in which antibodies or reagents are contacted.

Contacting methods are carried out, for example, by culturing the above-mentioned T cells for a specified period of time with PHA and others added to the medium, as shown in the below-mentioned Example. The anti-CD3 antibody and the anti-CD28 antibody may be ones each having been attached to magnetic beads. Further, stimulation may be achieved by culturing the above-mentioned T cells for a specified period of time in a culture dish to the surface of which an anti-CD3 antibody and an anti-CD28 antibody are attached, instead of adding these antibodies to the medium. Further, stimulation may be also achieved by adding the above-mentioned antigen peptide with feeder cells to the medium.

In order to stimulate the TCR of CD4/CD8 DN cells, the concentration of PHA added to the medium is not limited in particular, and preferably is 1 to 100 µg/mL. The concentrations of an anti-CD3 antibody and an anti-CD28 antibody added to the medium are not limited in particular, and preferably are one to ten times the amount of culture of the above-mentioned T cells. The concentrations of an anti-CD3 antibody and an anti-CD28 antibody at which these antibodies are attached to the surface of culture dishes for stimulating the TCR of CD4/CD8 DN cells are not limited in particular, and their concentration in coating processes preferably are 0.1 to 100 µg/mL for an anti-CD3 antibody and 0.1 to 10 µg/mL for an anti-CD28 antibody.

The period for culturing cells contained in the T-iPS-sacs preferably includes a period of time around which T cell receptor (TCR) is expressed on the cellular surface of CD4/CD8 DN cells obtained by differentiation as described above, and preferably is 7 to 29 days after culturing of cells contained in the T-iPS-sacs is started. Culture circumstances are not limited in particular, and preferably are under conditions of 5% CO2 and at 35 to 38°C, more preferably 37°C.

In such a culture period, a glycogen synthase kinase-3p (GSK3β) inhibitor may be added to the medium, from the viewpoint of enhancing the self-renewal ability and the production of CD8+ memory stem cells (stem-cell-like memory T cells (TSCMs)) having a multipotency capable of differentiating into effector memory T cells and effector T cells. Such a GSK3β inhibitor is any inhibitor which can activate the Wnt signaling pathway by suppressing the phosphorylation of β-catenin by GSK3β, and includes, for example, a 4,6-disubstituted pyrrolopyrimidine (TWS119). For TSCMs, see Luca Gattinoni et al., Nature Medicine, 2011, Vol. 17, pp.1290-1298. For the relationship between the inhibition of GSK3β and the enhancement of TSCM production, see Luca Gattinoni et al., Nature Medicine, 2009, Vol. 15, pp.808-813.

### <Step of differentiating the CD4/CD8 DN cell into a CD8 SP cell or a CD4 SP cell>

As shown in the below-mentioned Example section, it has been demonstrated that in the above-mentioned step, the stimulation of T-iPS-cell-derived CD4/CD8 DN cells through the TCR expressed on their cellular surface makes it possible to suppress further rearrangement of the TCRA gene (receptor revision) which may be caused as the T-iPS cells differentiate from the DN stage to the DP stage and further to the SP stage such as the CD8 SP stage or the CD4 SP stage.

Therefore, in the method for producing a human CD8 SP cell or a human CD4 SP cell having antigen specificity according to the present invention, stimulation of T-iPS-cell-derived CD4/CD8 DN cells via the TCR expressed on their cellular surface, followed by differentiation of the stimulated CD4/CD8 DN cells into CD8 SP cells or CD4 SP cells, makes it possible that, in CD8 DP cells or CD4 SP cells obtained by redifferentiation, there is achieved a highly increased frequency of appearance of T cells having the same rearrangement pattern of the TCR gene as in their originating human T cells.

As a result of the above-mentioned "step of stimulating the T cell receptor of the CD4/CD8 DN cell", there are CD4/CD8 DN cells among the T-iPS-cell-derived T-lineage cells, but some of the T-lineage cells have been differentiated into CD4/CD8 DP cells. Therefore, the "step of differentiating the CD4/CD8 DN cell whose T cell receptor has been stimulated, into a single positive cell" includes differentiating the CD4/CD8 DN cells whose T cell receptor has been stimulated into CD8 SP cells or CD4 SP cells, and differentiating CD4/CD8 DP cells whose TCR has been stimulated at the DN stage into CD8 SP cells or CD4 SP cells.

In the present invention, it is preferable that in order to differentiate the CD4/CD8 DN cells whose T cell receptor has been stimulated into CD8 SP cells or CD4 SP cells, the stimulated CD4/CD8 DN cells are cultured in a medium containing cytokines, serum (for example, human serum), and others. The cytokines which are added to the medium are any ones which allow the stimulated CD4/CD8 DN cells to differentiate into CD8 SP cells or CD4 SP cells, and include, for example, IL-7, IL-15, and IL-2. Among these cytokines, IL-7 and IL-15 are preferably added in combination, from the viewpoints of facilitating the production of CD8 lineage cells and further memory-type CD8+ T cells in the differentiation into CD8 SP cells. The concentrations of IL-7 and IL-15 added are not limited in particular, and preferably are 1 to 20 ng/mL. Media include, for example, RPMI-1640 medium, X-VIVO medium, DMEM medium, and α-MEM medium, with RPMI-1640 medium and X-VIVO medium being preferable from the viewpoint that both media are more suitable for growth of blood cells. These media may be supplemented in addition to IL-7 and IL-15, with amino acids necessary for culture (for example, L-glutamine), antibiotics (for example, streptomycin, penicillin), and cytokines other than IL-7 and IL-15.

In such culture, the CD4/CD8 DN cells may be co-cultured with feeder cells. Feeder cells are not limited in particular, and preferably peripheral blood mononuclear cells (PBMCs) from the viewpoint of facilitating the differentiation into CD8 SP cells or CD4 SP cells and their proliferation through cell contact and the like. Such PBMCs are preferably allo (allogeneic) to the CD4/CD8 DN cells, from the viewpoint that the TCR is effectively stimulated. Such PBMCs are preferably auto (autologous) to the CD4/CD8 DN cells, from the viewpoint that survival is conferred while excessive stimulation of the TCR is prevented. Further, it is more preferable to use peripheral blood mononuclear cells presenting an antigen peptide to which the originating human T cells of the CD4/CD8 DN cells bind specifically, from the viewpoint of continuing to stimulate the TCR and to suppress further TCR rearrangement.

The culture period for differentiating the CD4/CD8 DN cells into CD8 SP cells or CD4 SP cells is preferably two to four weeks. Culture circumstances are not limited in particular, and preferably are under conditions of 5% CO2 and at 35 to 38°C, more preferably 37°C.

In connection with the present invention, it has been pointed out by Serwold, T. et al. in Proc Natl Acad Sci USA, 107, 18939-18943, 2010, Vol. 107, pp.18939-18943, that an unusually early TCR signaling in mouse thymus is likely to cause the occurrence of lymphoma. For this reason, the method for producing a human CD8 SP cell or CD4 SP cells according to the present invention may incorporate a system that uses a suicide gene into the cell, from the viewpoint of avoiding the occurrence of tumors resulting from activation of TCR signals at early (CD4/CD8 DN) stages. Examples of such a "system that uses a suicide gene" include, for example, systems using a gene encoding an inducible caspase 9 (iCasp 9) consisting of human caspase 9 and a modified FK-binding protein, as described in Antonio Di Stasi et al., N Engl J Med, 2011, Vol. 365, pp. 1673-1683; thymidine kinase (TK) genes, as described, for example, in Kaneko, S. et. al., BLOOD, 2009, Vol. 113, pp.1006-1015; Fabio Ciceri et al., THE LANCET, 2009, Vol. 10, pp.489-500; and Attilio Bondanza et al., blood, 2011, Vol. 24, pp.6469-6478.

The fact that CD8 SP cells or CD4 SP cells which have been subjected to differentiation induction as described above are derived from the T-iPS cells and from the originating T cells of the T-iPS cells can be ascertained, for example, by detecting the TCR gene rearrangement pattern by means of PCR for genomic DNA, as shown in the below-mentioned Example.

The CD8 SP cells or CD4 SP cells thus produced can be isolated by selecting known procedures as appropriate. Such known procedures include, for example, flow cytometry using an antibody directed to a cell surface marker, such as CD4 or CD8, and a cell sorter, as shown in the below-mentioned Example. In cases where "T cells having a desired antigen specificity" are isolated from a human, methods can be employed in which purification of the T cells is carried out using an affinity column on which the desired antigen (for example, an antigen recognized by the originating T cells of CD8 SP cells, in cases of CD8 SP cells and an antigen recognized by the originating T cells of CD4 SP cells, in cases of CD4 SP cells) is immobilized. Methods can be employed in which "T cells having a desired antigen specificity" are purified using an MHC multimer (for example, an MHC tetramer) to which the desired antigen has been attached.

The resulting CD8 SP cells do not express PD-1, and yet at the same time express CCR7 together with CD27 and CD28 (expression of CD27 and CD28 is one of the representative phenotypes of central memory T cells), have a longer telomere, relative to the original T cells, and possess an improved ability of self-replication. Therefore, the present invention can provide the production of T cells (for example, CD8 SP cells) having the same rearrangement pattern of the TCR gene as in the originating T cells and expressing CD27, CD28, and CCR7, but not PD-1. T cells which have been collected from a human express PD-1, and not CD27, CD28, nor CCR7, and in this respect are different from the resulting T cells, which can also apply to the CD4 SP cells obtained by the present method.

The resulting CD8 SP cells or CD4 SP cells may be stimulated every one to two weeks, for maintaining them. Such stimulation for CD8 SP cells includes contacting the cells with at least one substance selected from the group consisting of an anti-CD3 antibody, an anti-CD28 antibody, IL-2, IL-7, IL-15, an antigen recognized by the CD8 SP cells, an MHC multimer having the antigen attached thereto, feeder cells which are allo to the CD8 SP cells, and feeder cells which are auto to the CD8 SP cells. Such stimulation for CD4 SP cells includes contacting the cells with at least one substance selected from the group consisting of an anti-CD3 antibody, an anti-CD28 antibody, IL-2, IL-7, IL-15, an antigen recognized by the CD4 SP cells, an MHC multimer having the antigen attached thereto, feeder cells which are allo to the CD4 SP cells, and feeder cells which are auto to the CD4 SP cells.

### <Human CD8 SP cells or CD4 SP cells, pharmaceutical compositions, and methods for cell-based immunotherapy>

The human CD8 SP cells or CD4 SP cells produced by the method according to the present invention have antigen-specific immune functions. In addition, the human CD8 SP cells produced by the method according to the present invention do not express PD-1, which is a marker of exhausted T cells (i.e., T cells having significantly decreased long-term survival capability, self-replication ability, and/or effector functions), and yet at the same time express CCR7 together with CD27 and CD28, have a longer telomere, relative to the original T cell, and possess an improved self-replication ability, which can also apply to the human CD4 SP cells obtained by the present method. Thus, the human T cells produced by the method according to the present invention can be used, for example, for treatment or prevention of diseases such as tumors, infections (for example, chronic infections), autoimmune failures, and others.

The present invention provides a pharamceutical composition as defined in the claims. Therefore, the present disclosure provides human T cells produced by the method according to the present invention, a pharmaceutical composition comprising the human T cells, and a method for cell-based immunotherapy using the human T cells (immunocytotherapy or immunotherapy). In particular, the present disclosure provides human CD8 SP cells and/or human CD4 SP cells produced by the method according to the present invention, a pharmaceutical composition comprising the human CD8 SP cells and/or CD4 SP cells, and a method for cell-based immunotherapy using the human CD8 SP cells and/or CD4 SP cells.

The method for cell-based immunotherapy in a subject according to the present disclosure can be carried out, for example, in the following way. First, T cells are collected from a human, preferably a human having an identical HLA type to the subject, more preferably the subject to be treated. Second, T-iPS cells are prepared from the T cells, and subsequently, the T-iPS cells are differentiated into T cells, preferably CD4 SP cells or CD8 SP cells, having the same rearrangement pattern of the TCR gene as in their originating T cells. The resulting T cells express CCR7 together with CD27 and CD28, but not PD-1, and have a longer telomere, relative to the originating T cells, and possess an improved self-replication ability. Accordingly, the resulting cells may be identified by the expression of CD27, CD28, CCR7, and/or PD-1, if desired. The T cells thus obtained can be administered to the subject to be treated.

In the method for cell-based immunotherapy according to the present disclosure, the administration of the resulting T cells to a subject to be treated is not limited in particular, and can be preferably carried out by parenteral administration, such as intravenous, intraperitoneal, subcutaneous, or intramuscular administration, more preferably intravenous administration. Alternatively, topical administration to an affected site can be also done.

The pharmaceutical composition according to the present invention is defined in the claims. Also disclosed are pharmaceutical comositions that can be prepared by formulating the human CD8 SP cells or CD4 SP cells produced by the method according to the present invention into dosage forms by known pharmaceutical methods. For example, the pharmaceutical composition according to the present invention can be mainly administered parenterally, as capsules, liquids, film-coated preparations, suspensions, emulsions, injections (such as venous injections, drip injections, and the like), and others.

In formulation into these dosage forms, the T cells according to the present disclosure can be combined as appropriate, with pharmaceutically acceptable carriers or media, in particular, sterile water and physiological saline, vegetable oils, resolvents, bases, emulsifiers, suspending agents, surfactants, stabilizers, vehicles, antiseptics, binders, diluents, tonicity agents, soothing agents, bulking agents, disintegrants, buffering agents, coating agents, lubricants, coloring agents, solution adjuvants, or other additives. The T cells according to the present disclosure may be also used in combination with known pharmaceutical compositions, immunostimulants, and others which are used for the treatment or prevention of the above-mentioned diseases.

In administrating a pharmaceutical composition of the present invention, the dosage is selected as appropriate, depending upon the age, weight, symptoms, and physical condition of the subject, the type of composition (pharmaceutical, food/drink, etc.), and others.

Products (pharmaceuticals) from the compositions of the present invention or their instructions are ones having affixed thereto an indication that use is made for the treatment or prevention of decrease in immune function. Here, by "having an indication affixed to a product or its instructions," it is meant that the indication has been affixed to the body, container, packaging, or the like of the product, or alternatively its instructions, package insert, advertising materials, or other printed materials disclosing product information.

The method for cell-based immunotherapy according to the present disclosure comprises the steps of isolating T cells having a desired antigen specificity from a human; inducing iPS cells from the T cells having the desired antigen specificity; differentiating the iPS cells into CD4/CD8 double negative cells; stimulating the T cell receptor of the CD4/CD8 double negative cells; differentiating the CD4/CD8 double negative cells whose T cell receptor has been stimulated, into CD8 single positive cells and/or CD4 single positive cells; and administering to a human the resulting CD8 single positive cells and/or CD4 single positive cells.

In cases of carrying out the method for cell-based immunotherapy according to the present disclosure a human from which T cells are to be isolated preferably has an HLA type identical to that of a human to which CD8 single positive cells and/or CD4 single positive cells obtained by the method according to the present invention are to be administered, and more preferably is the same as a human to which CD8 single positive cells and/or CD4 single positive cells obtained by the method according to the present invention are to be administered, from the viewpoint that rejection is not caused. Regarding CD8 single positive cells and/or CD4 single positive cells to be administered, T cells produced by the method according to the present invention may be administered directly or in the form of formulated pharmaceutical compositions as described above.

### EXAMPLES

The present invention will be described more specifically below based on Examples, but is not limited to the below-mentioned Examples. Examples and Comparative Examples were carried out according to the below-mentioned methods.

### <Production of CTL clones>

CTL strain specific for Nefl38-8(wt) were established from PBMCs obtained from an HIV-1-infected patient who had an HLA-A24, as described in Kawana-Tachikawa, A. et al., J. Virol., 2002, Vol. 76, pp.11982-11988.

### <Production of T-iPS cells>

Human iPS cells were established from peripheral blood T cells or CTL clones under conditions optimized for culturing T cells, as described in Takayama, N. et al., J. Exp. Med., 2010, Vol. 207, pp.2817-2830.

In brief, peripheral blood T cells were first stimulated and activated with α-CD3/CD28 antibody-coated beads (manufactured by Miltenyi Biotec). CTL clones, on the other hand, were stimulated and activated with PHA (manufactured by Sigma-Aldrich). CTL clones were also stimulated and activated with irradiated PBMCs (alloantigen expressing cells) which were derived from an individual different from the HIV-1-infected patient. In these cases, there had not been observed significant differences in properties of the resulting T-iPS cells between stimulation with PHA and with alloantigen expressing cells.

Reprogramming factors were introduced into the activated cells with retroviral vectors (pMXs retroviral vectors) or Sendai virus vectors, and then the cells were cultured with 10 ng/mL (200 U) IL-2, 5 to 10 ng/mL IL-7, and 5 to 10 ng/mL IL-15 (manufactured by Peprotech) in RH10 medium. During the culture period, the medium was gradually replaced to human iPS medium (manufactured by Wako) containing bFGF and others.

The introduction of the viral vectors into the cells was carried out by centrifugation in a RetroNectin®-coated plate (manufactured by Takara). The composition of the RH10 medium is as mentioned below:
RPMI-1640 supplemented with 10% human AB serum, 2 mM L-glutamine, 100 U/mL penicillin, and 100 ng/mL streptomycin.
The composition of the human iPS medium is as mentioned below:
DMEM/F12 FAM supplemented with 20% KSR, 2mM L-glutamine, 1% non-essential amino acids, 10 µM 2-mercaptoethanol, and 5 ng/mL b-FGF.

Further, siRNA L527 (see Nishimura, K. et al., J Biol. Chem., 2011, Vol. 286, pp.4760-4771) was introduced into the established iPS clones by Lipofectamine RNAiMAX (manufactured by Invitrogen), in order to remove SeV viruses from the cytoplasm.

### <Alkaline phosphatase staining and immunocytochemistry>

Alkaline phosphatase activity was assessed using an Alkaline Phosphatase Substrate Kit II (manufactured by Vector Laboratories) according to its instructions for use.

Immunocytochemical staining was carried out using the below-mentioned antibodies, as described in Takayama, N. et al., J Exp Med, 2010, Vol. 207, pp.2817-2830. The ratio in the parenthesis indicates a dilution of the indicated antibody.
SSEA-4 (1:50, FAB1435P, manufactured by R&D Systems)
Tra-1-60 (1:100, MAB4360, manufactured by Millipore)
Tra-1-81 (1:100, MAB4381, manufactured by Millipore)
HLA-A24 (1:100, BIH0964, manufactured by Veritas)
Micrographs were taken using an Axio Observer Z1 fluorescence microscope (manufactured by Carl Zeiss).

### <Teratoma formation>

The pluripotency of human iPS cells was assessed through the formation of teratomas in a system using NOD-Scid mice, as described in Masaki, H. et al., Stem Cell Res., 2007, Vol. 1, pp.105-115.

### <Bisulfite sequencing>

Genomic DNA was treated using a MethylEasy Xceed Rapid DNA Bisulphite Modification Kit (manufactured by Human Genetic Signatures) according to its instructions for use.

Promoter regions of the human Oct3/4 and Nanog genes were amplified by PCR using EpiTaq HS (manufactured by Takara). The PCR product obtained was inserted into a pGEM-T-Easy vector (manufactured by Promega), cloned, and subjected to sequencing. The sequences of primers used for the PCR (SEQ ID NOs: 5 to 8) are shown in Table 1.

**Table 1**

| PCR primers for bisulfite sequence | |
|---|---|
| Primer | Sequence |
| bisOCT4-3/F | ATTTGTTTTTTGGGTAGTTAAAGGT |
| bisOCT4-3/R | CCAACTATCTTCATCTTAATAACATCC |
| mehNANOG-F1-S | TGGTTAGGTTGGTTTTAAATTTTTG |
| mehNANOG-F1-AS | AACCCACCCTTATAAATTCTCAATTA |

### <RT-PCR and quantitative PCR>

Respective total RNAs were extracted from ES, iPS, and T cells using an RNeasy Micro Kit (manufactured by Qiagen). Then, each of the total RNAs was used as a template to perform a reverse transcription reaction using a High Capacity cDNA Reverse Transcription Kit (manufactured by Applied Biosystems) and a random 6-base primer. The RT-PCR was performed as described in Takayama, N. et al., J. Exp. Med., 2010, Vol. 207, pp.2817-2830. The names of target genes and the sequences of PCR primers used in the analysis (SEQ ID NOs:9 to 43) are listed in Table 2.

**Table 2**

| PCR primers for RT-PCR | | |
|---|---|---|
| Target gene | Primer | Sequence (5'-3') |
| GAPDH | hGAPDH-PF1 | AACAGCCTCAAGATCATCAGC |
| | hGAPDH-PR2 | TTGGCAGGTTTTTCTAGACGG |
| | | |
| 4-Oct | total-OCT4-F.ips | ATTCAGCCAAACGACCATC |
| | total-OCT4-R.ips | GGAAAGGGACCGAGGAGTA |
| SOX2 | total-SOX2-F.ipS | CAGCGCATGGACAGTTAC |
| | total-SOX2-R.ips | GGAGTGGGAGGAAGAGGT |
| c-MYC | total-c-MYC-F.ips | AGTTTCATCTGCGACCCG |
| | total-c-MYC-R.ips | CCTCATCTTCTTGTTCCTCCT |
| KLF4 | total-KLF4-F.ips | GCGGGAAGGGAGAAGACA |
| | total-KLF4-R.ips | CCGGATCGGATAGGTGAA |
| | | |
| endogenous 4-Oct | endo-hOCT3/4-S1165 | GACAGGGGGAGGGGAGGAGCTAGG |
| | endo-hOCT3/4-AS1283 | CTTCCCTCCAACCAGTTGCCCCAAAC |
| endogenous SOX2 | endo-hSOX2-S1430 | GGGAAATGGGAGGGGTGCAAAAGAGG |
| | endo-hSOX2-AS1555 | TTGCGTGAGTGTGGATGGGATTGGTG |
| endogenous c-MYC | endo-hMYC-S253 | GCGTCCTGGGAAGGGAGATCCGGAGC |
| | endo-hMYC-AS555 | TTGAGGGGCATCGTCGCGGGAGGCTG |
| | | |
| endogenous | endo-hKLF4-S1128 | ACGATCGTGGCCCCGGAAAAGGACC |
| KLF4 | endo-hKLF4-AS1826 | TGATTGTAGTGCTTTCTGGCTGGGCTCC |
| | | |
| Tg-OCT4 | Tg-OCT3/4-S880 | CAACGAGAGGATTTTGAGGCT |
| Tg-SOX2 | Tg-hSOX2-S614 | TGCAGTACAACTCCATGACCA |
| Tg-c-MYC | Tg-hMYC-S1011 | CAACAACCGAAATGCACCAGCCCCAG |
| Tg-KLF4 | Tg-hKLF4-S1180 | TGCGGCAAAACCTACACAAAG |
| Tg-Vector | pMX-Tg-R | TACAGGTGGGGTCTTTCATTC |
| | | |
| TERT | TERT-F.ips | TGTGCACCAACATCTACAAG |
| | TERT-R.ips | GCGTTCTTGGCTTTCAGGAT |
| REX1 | REX1-F.ips | CAGATCCTAAACAGCTCGCAGAAT |
| | REX1-R.ips | GCGTACGCAAATTAAAGTCCAGA |
| GDF3 | GDF-3-F.ips | AAATGTTTGTGTTGCGGTCA |
| | GDF3-R.ips | TCTGGCACAGGTGTCTTCAG |
| NANOG | Nanog-968S | CAGCCCTGATTCTTCCACCAGTCCC |
| | Nanog-1334AS | TGGAAGGTTCCCAGTCGGGTTCACC |
| RAG1 | RAG1-F.rt | GAGCAAGGTACCTCAGCCAG |
| | RAG1-R.rt | AACAATGGCTGAGTTGGGAC |
| RAG2 | RAG2-F.rt | GATTCCTGCTACCTCCCTCC |
| | RAG2-R.rt | AGCGTCCTCCAAAGAGAACA |

Quantitative PCR was performed using TaqMan® Array Human Stem Cell Pluripotency Card and customized card (manufactured by Applied Biosystems).

### <Analysis of rearrangement of the TCR gene in genomic DNA>

Genomic DNA was extracted from about 5 × 10⁶ cells using a QIAamp DNA kit (manufactured by Qiagen) according to its instructions for use.

In order to analyze TCRB rearrangement, multiplex PCR analysis was performed according to slight modifications of the BIOMED-2 protocol (see van Dongen, J. J. et al., Leukemia, 2003, Vol. 17, pp.2257-2317). The primers used in the PCR for the analysis of rearrangement of the TCRβ gene (SEQ ID NOs:44 to 81) are shown in Table 3.

In order to analyze rearrangement of the TCRA gene, PCR was performed using primers shown in Fig. 1 and in Table 4 (SEQ ID NOs:82 to 127) and LATaq HS (manufactured by Takara). In this case, the PCR was performed using a program to carry out 3 cycles of amplification steps at 95°C for 30 seconds, 68°C for 45 seconds, and 72°C for 6 minutes, 15 cycles of amplification steps at 95°C for 30 seconds, 62°C for 45 seconds, and 72°C for 6 minutes, and 12 cycles of amplification steps at 95°C for 15 seconds, 62°C for 30 seconds, and 72°C for 6 minutes. A major band in the range of expected molecular sizes was purified using a QIAquick gel extraction kit (manufactured by Qiagen), and then subjected to sequencing. The use of the V, D, and J segments was identified by using an online tool (IMGT/V-Quest) for comparison to the ImMunoGeneTics (IMGT) databases (http://www.cines.fr/) (see Lefranc, M. P., Leukemia, 2003, Vol. 17, 2003, pp.260-266). The nomenclature of gene fragments (segments) followed the IMGT nomenclature.

**Table 3**

| PCR primers for detecting TCRB rearrangement | |
|---|---|
| Primer | Sequence (5'-3') |
| V*β*2 | AACTATGTTTTGGTATCGTCA |
| V*β*4 | CACGATGTTCTGGTACCGTCAGCA |
| V*β*5/1 | CAGTGTGTCCTGGTACCAACAG |
| V*β*6a/11 | AACCCTTTATTGGTACCGACA |
| V*β*6b/25 | ATCCCTTTTTTGGTACCAACAG |
| V*β*6c | AACCCTTTATTGGTATCAACAG |
| V*β*7 | CGCTATGTATTGGTACAAGCA |
| V*β*8a | CTCCCGTTTTCTGGTACAGACAGAC |
| V*β*9 | CGCTATGTATTGGTATAAACAG |
| V*β*10 | TTATGTTTACTGGTATCGTAAGAAGC |
| V*β*11 | CAAAATGTACTGGTATCAACAA |
| V*β*12a/3/13a/15 | ATACATGTACTGGTATCGACAAGAC |
| V*β*13b | GGCCATGTACTGGTATAGACAAG |
| V*β*13c/12b/14 | GTATATGTCCTGGTATCGACAAGA |
| V*β*16 | TAACCTTTATTGGTATCGACGTGT |
| V*β*17 | GGCCATGTACTGGTACCGACA |
| V*β*18 | TCATGTTTACTGGTATCGGCAG |
| V*β*19 | TTATGTTTATTGGTATCAACAGAATCA |
| V*β*20 | CAACCTATACTGGTACCGACA |
| V*β*21 | TACCCTTTACTGGTACCGGCAG |
| V*β*22 | ATACTTCTATTGGTACAGACAAATCT |
| V*β*23/8b | CACGGTCTACTGGTACCAGCA |
| V*β*24 | CGTCATGTACTGGTACCAGCA |
| | |
| D*β*1 | GCCAAACAGCCTTACAAAGAC |
| D*β*2 | TTTCCAAGCCCCACACAGTC |
| | |
| J*β*1.1 | CTTACCTACAACTGTGAATCTGGTG |
| J*β*1.2 | CTTACCTACAACGGTTAACCTGGTC |
| J*β*1.3 | CTTACCTACAACAGTGAGCCAACTT |
| J*β*1.4 | CATACCCAAGACAGAGAGCTGGGTTC |
| J*β*1.5 | CTTACCTAGGATGGAGAGTCGAGTC |
| J*β*1.6 | CATACCTGTCACAGTGAGCCTG |
| J*β*2.1 | CCTTCTTACCTAGCACGGTGA |
| J*β*2.2 | CTTACCCAGTACGGTCAGCCT |
| J*β*2.3 | CCCGCTTACCGAGCACTGTCA |
| J*β*2.4 | CCAGCTTACCCAGCACTGAGA |
| J*β*2.5 | CGCGCACACCGAGCAC |
| J*β*2.6 | CTCGCCCAGCACGGTCAGCCT |
| J*β*2.7 | CTTACCTGTAACCGTGAGCCTG |

**Table 4**

| PCR primers for detecting TCRA rearrangement | |
|---|---|
| Primer | Sequence (5'-3') |
| tVA1A | TCTGGTATGTGCAATACCCCAACC |
| tVA1B | CTGAGGAAACCCTCTGTGCA |
| tVA2 | CCGGGCAGCAGACACTGCTTCTTA |
| tVA3 | GATGGAAGGTTTACAGCACAGCTC |
| tVA4A | CCTCCCAGGGTCCAGAGTACG |
| tVA4B | GGATTGCGCTGAAGGAAGAG |
| tVA5 | GCAACATGCTGGCGGAGCACGCAC |
| tVA6 | TGAAGGTCACCTTTGATACCACCC |
| tVA7 | AACTGCACGTACCAGACATC |
| tVA8 | ACCCTGAGTGTCCAGGAGGG |
| tVA9 | CACTGCTGACCTTAACAAAGGCG |
| tVA10 | TCCTGGTGACAGTAGTTACG |
| tVA11 | AGGCTCAAAGCCTTCTCAGCAGGG |
| tVA12 | TCCACCAGTTCCTTCAACTTCACC |
| tVA13 | TTCATCAAAACCCTTGGGGACAGC |
| tVA14 | CCCAGCAGGCAGATGATTCTCGTT |
| tVA15 | GGATAAACATCTGTCTCTGCG |
| tVA16 | AAGGGAATCCTCTGACTGTG |
| tVA17 | GATAGCCATACGTCCAGATG |
| tVA18 | TGCCACTCTTAATACCAAGGAGGG |
| tVA19 | ACACTGGCTGCAACAGCATC |
| tVA20 | TTACAAACGAAGTGGCCTCC |
| tVA21 | ACCCTGCTGAAGGTCCTACATTCC |
| tVA22 | CTTGGAGAAAGGCTCAGTTC |
| tVA23 | TGCCTCGCTGGATAAATCATCAGG |
| tVA24 | TCCCAGCTCAGCGATTCAGCCTCC |
| tVA25 | GTCCTGTCCTCTTGATAGCC |
| tVA26 | AACTGCACGTACCAGACATC |
| tVA27 | AGCCCAGCCATGCAGGCATCTACC |
| tVA28 | GAACATCACAGCCACCCAGACCGG |
| tVA29 | GCAAAGCTCCCTGTACCTTACGG |
| tVA30 | CACAGCCCCTAAACCTGAAG |
| tVA31 | AGCAAAAACTTCGGAGGCGG |
| tVA32 | AAGGAGAGGACTTCACCACG |
| | |
| tJA1 | GGCAAGACGGAACCAATGTT |
| tJA6 | GGGGAGGTGGTGACAGAGTC |
| tJA10 | TGACGGTGCAATTTTCTGCT |
| tJA17 | AGAAACGCCACGTCCAAGTT |
| tJA23 | TCCTAGCCAGGACGACGATT |
| tJA29 | TCCTGGGATCAGAGGAGGAA |
| tJA32 | TCTCCTCCCCTGACTGTGGT |
| tJA36 | TGCACCCTGAGATGGTTCTTT |
| tJA41 | CTGCCCCGAGACCTGATAAC |
| tJA48 | TTGGCTGCCACCTGTCTCTA |
| tJA53 | GAATGTTTTGGAGGGOCAAG |
| tJA58 | TTCAATGCTCCCCTCCACTT |

### <Detection of rearrangement of the TCR gene using mRNA>

By a method based on switch mechanism at the 5'-end of the reverse transcript (SMART method; see Du, G. et al., J. Immunol. Methods, 2006, Vol. 308, 19-35 (2006)), double-stranded cDNAs were synthesized using a Super SMART(TM) cDNA synthesis kit (manufactured by Clontech Laboratories) according to its instructions for use. The synthesized double-stranded cDNAs were amplified using an Advantage 2 PCR Kit (manufactured by BD Clontech), and then TCRA- or TCRB-specific amplification from the amplified cDNAs was performed using primers listed in Table 5 (SEQ ID NOs:128 to 130). The PCR product obtained was inserted into a pGEM-T-Easy vector (manufactured by Promega), cloned, and subjected to sequencing.

**Table 5**

| PCR primers for amplifying SMART cDNA | |
|---|---|
| Primer | Sequence |
| 2^{nd}_5' -SMART | CAACGCAGAGTACGCGGG |
| 3' -TRAC | GCTGTTGTTGAAGGCGTTTG |
| 3' -TRBC | TCTCCGAGAGCCCGTAGAAC |

### <Intracellular staining>

For staining intracellular granzyme B, T cells were incubated with α-CD3/28 beads and 10 µg/mL brefeldin A (BFA, manufactured by Invitrogen).

The incubated cells were collected, fixed in a fixation/permeabilization solution (manufactured by BD Pharmingen), and subjected to intracellular staining using an FITC-conjugated anti-granzyme B antibody (manufactured by BD Bioscience) according to its instructions for use.

In order to detect CD107a which was transiently expressed on the cell surface, T cells were incubated with α-CD3/28 beads and cultured with an FITC-conjugated anti-CD 107a antibody (manufactured by BioLegend).

The cells thus prepared were sorted by a FACS Aria II instrument (manufactured by BD Biosciences) and analyzed using a Flowjo software (manufactured by Treestar).

### <Microarray analysis>

Respective total RNAs were extracted from human ES cells, T-iPS cells, redifferentiated T cells, peripheral blood T cells, and peripheral blood NK cells using an RNeasy® Micro Kit (manufactured by Qiagen).

Fluorescently labeled complementary RNAs of each of the total RNAs and Whole Human Genome Microarray 4×44K (G4112F, manufactured by Agilent Technologies) or SurePrint G3 Human Gene Expression 8×60K (G4851A, manufactured by Agilent Technologies) were hybridized in a one-color protocol. The resulting signal data were analyzed using a GeneSpring GX software (manufactured by Agilent Technologies).

### <Determination of the telomere length by Flow-FISH>

A DAKO Telomere PNA Kit/FITC (manufactured by DAKO) was used to determine the telomere length, as described in Neuber, K. et al., Immunology, 2003, Vol. 109, pp.24-31.

### <ELISPOT assay and ⁵¹Cr-release assay>

Antigen-specific response properties of T cells were determined by carrying out an ELISPOT assay for IFN-γ and a standard ⁵¹Cr-release assay, using HLA-A24-expressing autologous B-LCLs as an antigen expressing cell, as described in Kawana-Tachikawa, A. et al., J. Virol., 2002, Vol. 76, pp.11982-11988; and Tsunetsugu-Yokota, Y. et al., J. Virol., 2003, Vol. 77, pp. 10250-10259.

### <Statistical analysis>

All the data in Examples were expressed by mean ± standard deviation (mean ± S.D.). In all the statistical analyses in Examples, Excel (manufactured by Microsoft) or Prism (manufactured by Graphpad Software) was used to carry out unpaired two-tailed Student t-test. Statistical significance was determined at values of P < 0.05.

### (Preparation Example 1)

### <Reprogramming from antigen-specific cytotoxic T cell clones into pluripotent cells>

In order to establish T-cell-derived iPS cells, the present inventors magnetically isolated CD3+ T cells from PBMCs which were obtained from healthy subjects. Then, the isolated CD3+ T cells were stimulated in the presence of 10 ng/mL IL-2, with microbeads coated with an anti-human CD3 antibody and an anti-human CD28 antibody(α-CD3/CD28 beads) in an amount of three times that of the CD3+ T cells. After that, the CD3+ T cells thus activated were subjected to introduction of retroviruses each encoding any of OCT4, SOX2, KLF4, and c-MYC (see Fig. 2).

Similarly, PBMCs derived from a person with chronic HIV infection (having an HLA type of A24) were isolated and used to establish CD8+ CTL clones specific for an antigen peptide derived from the HIV-1 Nef protein (Nef-138-8(wt), RYPLTFGW, SEQ ID NO:1; see Miyazaki, E. et al., AIDS, 2009, Vol. 23, pp.651-660). Among these clones, a clone designated H25-4 was stimulated with 5 µg/mL PHA. The thus-activated cells of the H25-4 clone were subjected to introduction of two SeV vectors, an SeV vector encoding cistronically-expressed four factors (OCT4, SOX2, KLF4, and c-MYC) and the miR-302 target sequence (SeVp[KOSM302L]; see Nishimura, K. et al., J Biol Chem, 2011, Vol. 286, pp.4760-4771) and an SeV vector encoding the SV40 large T antigen (SeV18+SV40/TS15ΔF; see Fusaki, N. et al., Proc. Jpn. Acad. Ser. B Phys. Biol. Sci., 2009, Vol. 85, pp.348-362). Introducing these two SeV vectors into the PHA-activated H25-4 cells, followed by culturing for 40 days, allowed one to observe that a sufficient number of Human ES-cell-resembling colonies appeared (see Figs. 3 and 4).

As results, an ES-cell-resembling colonies derived from a CD3+ T cell and an ES-cell-resembling colony derived from an H25-4 clone cell (which colonies were also referred as hereinafter to "TkT3V1-7" and "H254SeVT-3," respectively) had alkaline phosphatase (AP) activity and were found to express pluripotent cell markers, SSEA-4, Tra-1-60, and Tra-1-81 (see Fig. 4 and 5). It was also found that even after the expression of the exogenous reprogramming factors from the incorporated proviruses (in the TkT3V1-7 clone) or the cytoplasmic SeV RNAs (in the H254SeVT-3 clone) was terminated, human ES-cell-related genes were expressed (see Figs. 6 and 7).

The results of comparing their gene expression profiles showed that the whole gene expression patterns in ES-like cells of these clones were similar to that in human ES cells and significantly different from that in peripheral blood T cells (see Figs. 6, 8, and 9).

In addition, by bisulfite PCR assay, slight methylation was detected in the OCT4 and NANOG promoter regions. In the light of Freberg, C. T. et al., Mol. Biol. Cell, 2007, Vol. 18, pp.1543-1553, therefore, it is demonstrated that reprogramming has been achieved in ES-like cells of these clones (see Figs. 10 and 11).

Further, in the light of Brivanlou, A. H. et al., Science, 2003, Vol. 300, pp.913-916, it is demonstrated that ES-like cells of these clones have pluripotency, because injection of these cells into NOD-Scid mice resulted in the formation of teratomas containing characteristic tissues, each of which was derived from any of the three germ layers (see FIGS. 12 and 13).

### <Rearrangement of the TCR gene in T-iPS cells>

It is known that the TCRαβ gene rearrangement is involved in the process of development of normal αβ T cells in the thymus. Thus, present inventors retrospectively examined, based on TCR gene rearrangement patterns, whether the T-iPS cells established as described above were derived from αβ T cells. In brief, multiplex PCR primers for analysis of rearrangement of the TCRB gene were designed according to the BIOMED-2 consortium (see van Dongen, J. J. et al., Leukemia, 2003, Vol. 17, pp.2257-2317). In addition, primers for detection of rearrangement of the TCRA gene were independently designed (see Fig. 1). These primers were used to perform PCR, thereby detecting rearrangement of the TCRαβ gene in the T-iPS cells described above. The results obtained are shown in Figs. 14 to 17.

As shown in Figs. 14 to 17, the respective rearrangements of the TCRB and TCRA genes were identified as a single band in the allele of cells of each of the TkT3V1-7 and H254SeVT-3 clones.

The structure of the antigen recognition site of a TCR is composed of three complementarity determining regions (CDR1, CDR2, and CDR3). Further, in these three regions, the CDR3 has a most diverse sequence because the CDR3 extends to the V(D)J junction region into which a variety of random nucleotides (N- or P-nucleotides) are inserted (see Alt, F. W. et al., Proc. Natl. Acad. Sci. USA, 1982, Vol. 79, pp.4118-4122; and Lafaille, J. J. et al., Cell, 1989, Vol. 59, pp.859-870).

Thus, sequencing was carried out for the CDR3 region in the TCRA and TCRB genes rearranged in T-iPS cells of the above-mentioned clones (TkT3V1-7 and H254SeVT-3) (SEQ ID NOs: 131 to 133 and 145 to 148). The results obtained are shown in Tables 6 to 9.

**Table 6**

| TCRA gene rearrangement in TkT3V1-7 | | | | | | | | |
|---|---|---|---|---|---|---|---|---|
| TCRA | | | | | | | | |
| Cell | Genome/RNA | Productivity | Rear Rearrangement | | | Sequence of junction region | | |
| | | | Vα | | Jα | 3*Vα | (P)N | 5*Jα |
| TkT3V1-7 | Genome | Productive | TRAV38-2DV8*01 | | TRAJ31*01 | TGTGCTTAT | TGGAGT | AATAACAATGCCAGACTCATGTTT |
| | | Unproductive | | Intact | | | Intact | |

**Table 7**

| TCRB gene rearrangement in TkT3V1-7 | | | | | | | | |
|---|---|---|---|---|---|---|---|---|
| TCRB | | | | | | | | |
| Cell | Genome/RNA | Productivity | | Rearrangement | | Sequence of junction region | | |
| | | | Vβ | Dβ | Jβ | 3*Vβ | N1-Oβ-N2 | 5*Jβ |
| **TkT3V1-7** | Genome | Productive | TRBV29-1*02 | TRBO1*01 | TRBJ2-2*01 | TATATCTCTGCAGCGTTGA | TGGACAGGGA | AACACCGGGGAGCTGTT T |
| | | Unproductive | Germ line | TRBD2*01*02 | TRBJ2-7*01/02 | TATCATGGTGTAACATTGTG | GGGACTAGTTTAC | CCTACGAGCAGTACTTCG |

**Table 8**

| TCRA gene rearrangement in H25-4, H254SeVT-3 or redifferenciated CD8+ T Cells | | | | | | | |
|---|---|---|---|---|---|---|---|
| TCRA | | | | | | | |
| Cell | Genome/RNA | Productivity | Rearrangement | | | Sequence of junction region | |
| | | | Vα | Jα | 3*Vα | ('P)N | 5*Jα |
| H25-4 | Genome | Productive | TRAV8-3'01 | TRAJ10*01 | TGTGCTGTGGGT | T | TCACGGGAGGAGGAAACAAACTCACCTTTT |
| | | Unproductive^{a} | TRAV13-1*01 | TRAJ29*01 | TGTGCAGCAA | TCC | TCAGGAAACACACCTCTTGTCTTT |
| H254SeVT-3 | Genome | Productive | TRAV8-3*01 | TRAJ10*01 | TGTGCTGTGGGT | T | TCACGGGAGGAGGAAACAAACTCACCTTTT |
| | | Unproductive^{a} | TRAV13-1*01 | TRAJ29*01 | TGTGCAGCAA | TCC | TCAGGAAACACACCTCTTGTCTTT |
| reT-1 | mRNA | Productive | TRAV8-3*01 | TRAJ10*01 | TGTGCTGTGGGT | T | TCACGGGAGGAGGAAACAAACTCACCTTT |
| | | Unproductive^{a} | TRAV13-1*01 | TRAJ29*01 | TGTGCAGCAA | TCC | TCAGGAAACACACCTCTTGTCTTT |
| reT-21 | mRNA | Productive | TRAV8-3*01 | TRAJ10*01 | TGTGCTGTGGGT | T | TCACGGGAGGAGGAAACAAACTCACCTTTT |
| | | Unproductive^{a} | TRAV13-1*01 | TRAJ29'01 | TGTGCAGCAA | TCC | TCAGGAAACACACCTCTTGTCTTT |
| reT-3 | mRNA | Productive | TRAV8.3*01 | TRAJ 10'01 | TGTGCTGTGGGT | T | TCACGGGAGGAGGAAACAAACTCACCTTTT |
| | | Unproductive^{a} | TRAV13-1'01 | TRAJ29'01 | TGTGCAGCAA | TCC | TCAGGAAACACACCTCTTGTCTTT |

| | | | | | | | |
|---|---|---|---|---|---|---|---|
| "a" indicates that out-of-frame junction is caused in the CDR3. | | | | | | | |

**Table 9**

| TCRB gene rearrangement in H25-4, H254SeVT-3 or redifferenciated CD8+ T Cells | | | | | | | | |
|---|---|---|---|---|---|---|---|---|
| TCRB | | | | | | | | |
| Cell | Genome/RNA | Productivity | Rearrangement | | | | Sequence of junction region | |
| | | | Vβ | Dβ | Jβ | 3*Vβ | N1-Dβ-N2 | 5*Jβ |
| H25-4 | Genome | Productive | TR8V7-9*01 | TRBO1*01 | TRBJ2-5*01 | TGTGCCAGCAGCTTA | CGGCACAGGGTGCCG | GAGACCCAGTACTTC |
| | | Unproductive^{a} | Germ line | TRBO1*01 | TRBJ2- 7*01 | TACAAAGCTGTAACATTGTG | GGGACAACT | CTACGAGCAGTACTTCGGGCCG |
| **H254SeVT-3** | Genome | Productive | TR8V7-9*01 | TRBO1*01 | TRBJ2-5*01 | TGTGCCAGCAGCTTA | CGGGACAGGGTGCCG | GAGACCCAGTACTTC |
| | | Unproductive^{a} | Germ line | TRBO1*01 | TRBJ2-7*01 | TACAAAGCTGTAACATTGTG | GGGACAACT | CTACGAGCAGTACTTCGGGCCG |
| **reT-1** | **mRNA** | Productive | TR8V7-9*01 | TRBO1*01 | TRBJ2-5*01 | TGTGCCAGCAGCTTA | CGGGACAGGGTGCCG | GAGACCCAGTACTTC |
| **reT-21** | **mRNA** | Productive | TR8V7-9*01 | TRBO1*01 | TRBJ2-5*01 | | CGGGACAGGGTGCCG | GAGACCCAGTACTTC |
| **reT-3** | **mRNA** | Productive | TR8V7-9*01 | TRBO1*01 | TRBJ2-5*01 | TGTGCCAGCAGCTTA | CGGGACAGGGT GCCG | GAGACCCAGTACTTC |

As shown in Tables 6 to 9, a set of respective effective rearrangements of the TCRA and TCRB genes, for example, which had an in-frame junction and in which a stop codon was not generated was identified in the TkT3V1-7 and H254SeVT-3 clones. It was also demonstrated that the respective CDR3 sequences in the H254SeVT-3 and H25-4 clones were completely identical in the TCRA and TCRB gene regions. From these results, it is revealed that an iPS cell is established from a single T cell and that the antigen specificity imprinted in the genomic DNA is kept also in the process of reprogramming.

### (Comparative Example 1)

### <Redifferentiation from T-iPS cells into T cells>

Next, in order to examine the hematopoietic differentiation potential of T-iPS cells of the above-mentioned clones, these T-iPS cells were redifferentiated into mesoderm-derived cells, particularly hematopoietic stem cells/progenitor cells (see International Publication No. WO 2011/096482; Vodyanik, M. A. et al., Blood, 2005, Vol. 105, pp.617-626; and Takayama, N. et al., Blood, 2008, Vol. 111, pp.5298-5306).

In brief, small aggregates (each having not more than 100 cells) of T-iPS cells of the respective above-mentioned clones were transferred onto irradiated C3H10T1/2 cells and co-cultured in EB medium in the presence of 20 ng/mL VEGF, 50 ng/mL SCF, and 50 ng/mL FLT-3L (manufactured by Peprotech). The composition of the EB medium is as mentioned below:
IMDM supplemented with 15% fetal bovine serum (FBS), a cocktail of 10 µg/mL human insulin, 5.5 µg/mL human transferrin, and 5 ng/mL sodium selenite, 2 mM L-glutamine, 0.45 mM α-monothioglycerol, and 50 µg/mL ascorbic acid.

On day 14 after the cells were transferred onto the C3H10T1/2 feeder cells, hematopoietic cells (CD34+ hematopoietic stem cells/progenitor cells) contained in the iPS-sacs were collected and then transferred onto irradiated OP9-DL1 cells. OP9-DL1 cells were provided from the RIKEN BioResource Center through the National BioResource Project, the Ministry of Education, Culture, Sports, Science & Technology in Japan (Japan) (see Watarai, H. et al., Blood, 2010, Vol. 115, pp.230-237). The hematopoietic cells were subjected to differentiation into cells of the T lineage in OP9 medium in the presence of 10 ng/mL FLT-3L and 1 ng/mL IL-7 (see Ikawa, T. et al., Science, 2010, Vol. 329, pp.93-96). The composition of the OP9 medium is as mentioned below:
αMEM supplemented with 15% FBS, 2 mM L-glutamine, 100 U/mL penicillin, and 100 ng/mL streptomycin.

On days 21 to 28 after the start of culturing on OP9-DL1 feeder cells, there was observed the differentiation into CD45+, CD38+, CD7+, CD45RA+, CD3+, and TCRap+ cells of the T lineage (see Figs. 18 and 19).

As shown in Fig. 20, in a particular population of these T-lineage cells, there was found the differentiation into a small number of SP T-cells, which were not able to be characterized in detail by the present inventors, while there was also observed the presence of groups of cells which had been differentiated into a CD4/CD8 double-positive (DP) stage and into a mature CD4 single-positive (SP) or CD8 single-positive (SP) stage.

In the process in which T cells mature in thymus, the CD4/CD8 DN stage or the CD4/CD8 DP stage corresponds to the rearrangement stage of the β chain and the α chain, respectively (see Von Boehmer, H., Advances in Immunology, 2004, Vol. 84, pp.201-238). In addition, the negative feedback control of gene rearrangement and the suppression of further rearrangement in the TCRB gene locus are effected in an extremely strict manner (see Khor, B. et al., Current Opinion in Immunology, 2002, Vol. 14, pp.230-234). On the other hand, a relatively loose negative feedback control system and further gene rearrangement of pre-rearranged genes are also effected, and these phenomena tend to take place in the TCRA gene locus and are known as "receptor revision" (see Huang, C. et al., J. Immunol., 2001, Vol. 166, pp.2597-2601; and Krangel, M. S., Curr. Opin. Immunol., 2009, Vol. 21, pp.133-139).

Further, in experiments using TCRα transgenic mice, genes related to the mechanism of rearrangement (for example, Rag1 and Rag2) are re-activated at the DP stage and the gene rearrangement of an endogenous gene TCRA is also observed (see Petrie, H. T. et al., J Exp. Med., 1993, Vol. 178, pp.615-622; and Padovan, E. et al., Science, 1993, Vol. 262, pp.422-424).

Thus, in order to determine whether such receptor revision takes place also in the process of redifferentiation from T-iPS cells into cells of the T lineage, cells which were at each of the CD1a- DN and CD1a+ DP stages were collected from the above-mentioned CD45+, CD3+, TCRαβ+, and CD5+ cells of the T lineage, and subjected to analysis of the expression and sequences of TCR mRNAs (SEQ ID NOs131 to 149). The results obtained are shown in Fig. 21 and in Tables 10 to 13.

For TkT3V1-7 clone, cells at the CD8+ SP stage, which was a further advanced stage of differentiation than the CD1a- DN and CD1a+ DP stages, were collected and subjected to analysis of TCR mRNA expression. The results obtained are shown in Tables 14 and 15.

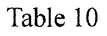

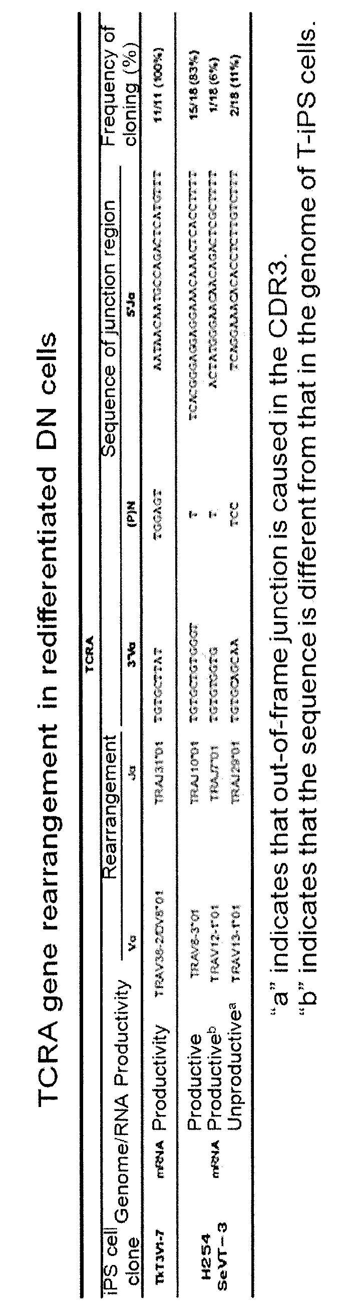

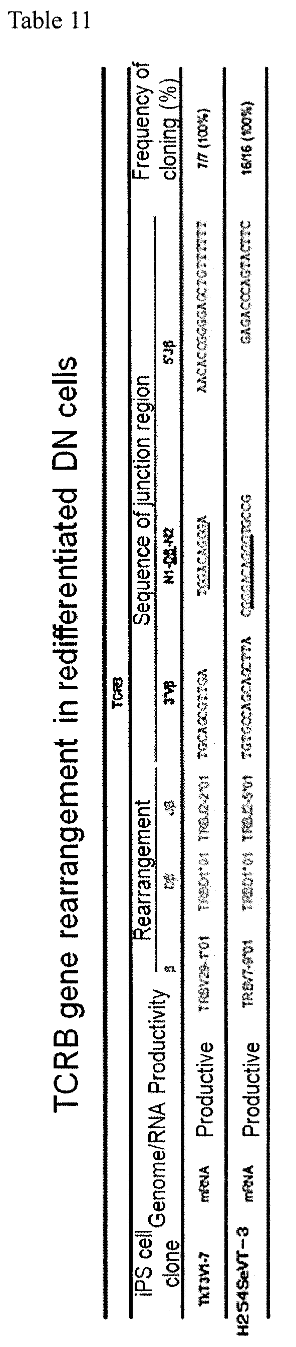

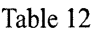

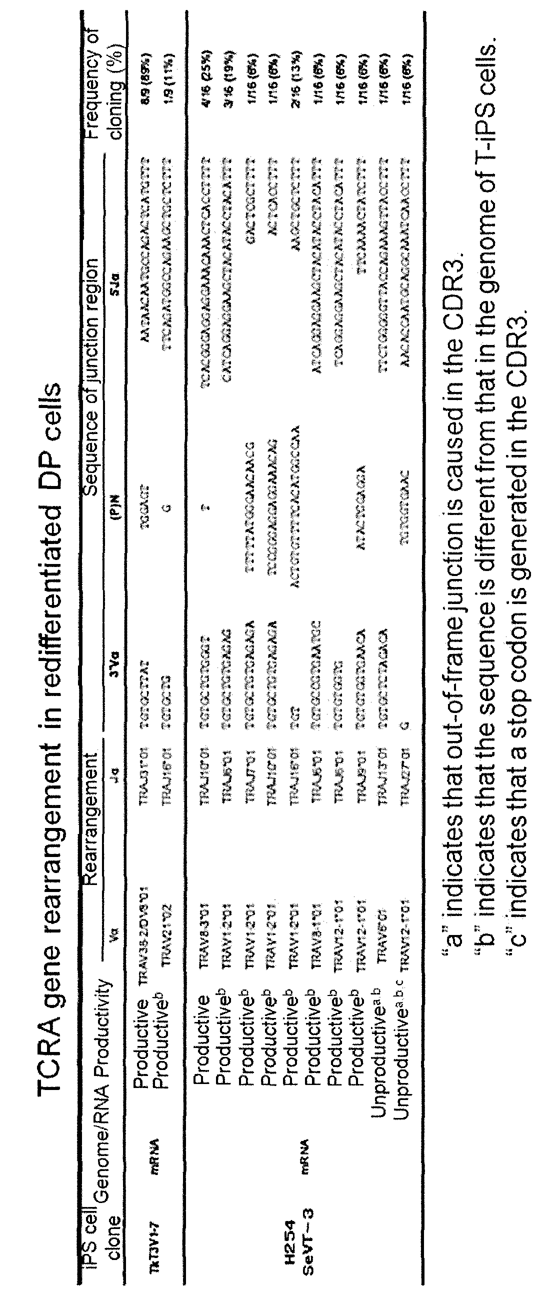

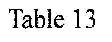

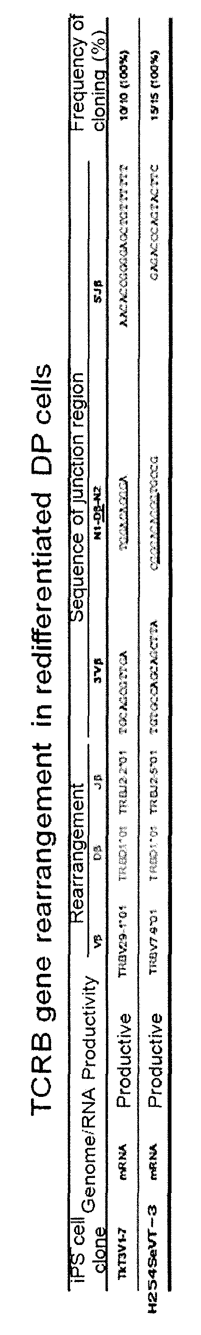

**Table 14**

| Cell | Genome/RNA | Productivity | Rearrangement | | | Frequency of cloning (%) |
|---|---|---|---|---|---|---|
| | | | V*α* | | J*α* | |
| TkT3V1-7 | Genome | Productive | TRAV38-2/DV8*01 | | TRAJ31*01 | - |
| | | Unproductive | | Intact | | - |
| CD8 SP cells derived from TkT3V1-7 cells | mRNA | Productive | TRAV38-2/D\/8*01F | | TRAJ31*01F | 75%(6/8) |
| | | Unproductive | TRAV30*01F | | TRAJ29*01F | 13%(1/8) |
| | | Unproductive | TRAV20*02(F) | | TRAJ28*01F | 13%(1/8) |

**Table 15**

| Cell | Genome/RNA | Productivity | Rearrangement | | | Frequency of cloning (%) |
|---|---|---|---|---|---|---|
| | | | V*β* | D*β* | J*β* | |
| TkT3V1-7 | Genome | Productive | TRBV29-1*01 Germ line | TRBD1*01 | TRBJ2-2*01 | - |
| | | Unproductive | | TRBD2*01 | TREJ2-7*01 | - |
| CD8 SP cells derived from TkT3V1-7 cells | m RNA | Productive | TRBV29-1*01 | TRBD1*01 | TRBJ2-2*01 | 89%(8/9) |
| | | Productive^{b} | TRBV29-1*03 | TRBD1*01 | TRBJ2-2*01 | 11 % (1/9) |

| | | | | | | |
|---|---|---|---|---|---|---|
| "b" indicates that the sequence is different from that in the genome of T-iPS cells. | | | | | | |

As shown in Fig. 21, for both clones, the base sequence of TCRB mRNA expressed in the resulting T-lineage cells was identical to that in their originating T-iPS cells at both the DN and DP stages.

Regarding TCRA mRNA, on the contrary, the identical sequence and a different sequence were included at the DN and DP stages, as shown in Tables 10 and 12. In addition, the DP stage was found to exhibit the different sequence more frequently than the DN stage did. Particularly in redifferentiated DP cells derived from the H254SeVT-3 clone, the frequency of T cells having the same gene rearrangement pattern as in the originating T cells was one forth or so.

Further, as shown in Tables 10, 12, and 14, it was demonstrated that in the TCRA gene of T-lineage cells derived from the TkT3V1-7 clone, the frequency of T cells having the same gene rearrangement pattern as in the originating T cells decreased to be 100%, 89%, and 75%, with differentiation to the DN stage, then to the DP stage, and then to the CD8+ SP stage, respectively.

Furthermore, as shown in Fig. 22, the expression of RAG1 and RAG2 was observed at both the DN and DP stages, and it was found that the DP stage exhibited their stronger expression than the DN stage.

### (Example 1)

### <Redifferentiation from T-iPS cells into CD8 SP cells according to the method of the present invention>

As shown in Comparative Example 1, it was demonstrated that in TCRA mRNA in T cells into which T-iPS cells had been redifferentiated, the DP stage exhibited a TCR gene rearrangement pattern different from that exhibited by the originating human T cells, more frequently than the DN stage. It was also demonstrated that, in T cells into which T-iPS cells had been redifferentiated, the DP stage exhibited a stronger expression of RAG1 and RAG2 than the DN stage.

As shown in Fig. 23, on the other hand, it was also demonstrated for the first time that T cells into which T-iPS cells had been redifferentiated expressed TCR (TCRαβ) on their surface even at the DN stage where it is known that TCR is usually not expressed on their surface at the DN stage in thymus.

As described in Turka, L. A. et al., Science, 1991, Vol. 253, pp.778-781, it has been known in the past that in the process of "positive selection," TCR signals through peptide-MHC complexes terminate the expression of the RAG genes and suppress further rearrangement of the TCR gene. Turka et al. also found that TCR signal-like signals by anti-CD3 antibodies exerted similar effects.

Thus, the present inventors attempted to stimulate the TCR of redifferentiated T-lineage cells before the transition from the DN stage to the DP stage had been completely finished, in order to produce a mature CD8 SP cells from T-lineage cells derived from T-iPS cells without receptor revision as shown in Comparative Example 1, based on a new finding that T cells into which T-iPS cells have been redifferentiated express TCR (TCRαβ) on their surface at the DN stage and on the previous report that activation of TCR signals can lead to the termination of the expression of the RAG genes and eventually the suppression of further rearrangement of the TCR gene.

In brief, as shown in Fig. 24, small aggregates (each having not more than 100 cells) of T-iPS cells obtained in Preparation Example 1 (H254SeVT-3 cells) were transferred onto irradiated C3H10T1/2 cells and co-cultured in EB medium in the presence of 20 ng/mL VEGF, 50 ng/mL SCF, and 50 ng/mL FLT-3L(manufactured by Peprotech).

On day 14 of the culture, hematopoietic cells contained in the iPS-sacs were collected, transferred onto irradiated OP9-DL1 cells, and then subjected to differentiation of the hematopoietic cells into cells of the T lineage in OP9 medium in the presence of 10 ng/mL FLT-3L and 1 ng/mL IL-7.

On day 35 of the culture, stimulation was applied by adding α-CD3/CD28 beads in an amount three times that of the hematopoietic cells or 5 µg/mL PHA to the OP9 medium, followed by continuing the culturing of cells committed to the T lineage on OP9-DL1 (the stimulation with α-CD3/CD28 beads or PHA is referred as hereinafter to the "first stimulation").

Subsequently, on day 45 of the culture, the T-lineage cells were collected and cultured with irradiated HLA-A24-PBMCs in RH10 medium in the presence of 10 ng/mL IL-7 and 10 ng/mL IL-15.

In connection with this Example, there have been reported that the IL-7 signal transduction contributes to selection of the CD8 lineage (see Chong, M. M. et al., Immunity, 2003, Vol. 18, pp.475-487; Singer, A. et al., Nat. Rev. Immunol., 2008, Vol. 8, pp.788-801; and Park, J. H. et al., Nat. Immunol., 2010, Vol. 11, pp.257-264) and additionally that IL-7 and IL-15 are required for the generation of memory-type CD8+ T cells (see Becker, T. C. et al., J. Exp. Med., 2002, Vol. 195, pp.1541-1548; Tan, J. T. et al., J. Exp. Med., 2002, Vol. 195, pp.1523-1532; Prlic, M. et al., J. Exp. Med., 2002, Vol. 195, pp.F49-52; and Kaneko, S. et al., Blood, 2009, Vol. 113, pp.1006-1015).

On day 60 of the culture, CD8 SP cells appeared, and these cells were found to be derived from H254SeV-3 cells based on the expression of HLA-A24, as shown in Fig. 25. In addition, the expression of CD56, which was expressed on in vitro cultured CD8+ T cells, was found on these CD8 SP cells (for CD56, see Lu, P. H. et al., J. Immunol., 1994, Vol. 153, pp.1687-1696). Further, these CD8 SP cells also expressed CD7 and some of these cells were found to express CD2. Furthermore, most of the CD8 SP cells did not express PD-1, which is a marker of exhausted T cells. Meanwhile, some of these CD8 SP cells were found to express CCR7 together with CD27 and CD28, which are representative of the phenotype of central memory T cells. Fig. 25, Figs. 26 to 39 described below, and Tables 8 and 9 show the results in cases where the above-mentioned T-lineage cells were stimulated with PHA as the first stimulation. Although not shown in any drawings, it was ascertained that the stimulation of the above-mentioned T-lineage cells with α-CD3/CD28 beads as the first stimulation resulted in similar results to those obtained by the stimulation with PHA.

### (Example 2)

### <Antigen specificity of CD8 SP cells obtained by the method according to the present invention >

An examination was made as to whether redifferentiated CD8 SP cells obtained in Example 1 were capable of recognizing antigen peptides for which the specificity was exhibited by the originating H25-4 cells, by having the same rearrangement pattern as in the originating TCR gene of the H25-4 cells. In brief, all redifferentiated T cells obtained in Example 1 and A24/Nef-138-8(wt) tetramer were mixed, and then subjected to flow cytometry analysis as described in Kawana-Tachikawa, A. et al., J. Virol., 2002, Vol. 76, pp.11982-11988. "A24/Nef-138-8(wt) tetramer" is a tetramer in which Nef-138-8(wt), an antigen which is recognized by H25-4 cells, and HLA(A24) are tetramerized. The results obtained are shown in Fig. 26.

As will be apparent from the results shown in Fig. 26, most of the T-iPS-cell-derived CD8 SP cells were stained with the A24/Nef-138-8(wt) tetramer. The percentage of CD8 SP positive cells (antigen-specific CD8 cells) in the T-iPS-cell-derived CD8 SP cells (CD8 SP tetramer-positive and tetramer-negative cells) was 77%. However, although not shown in any drawings, the T-iPS-cell-derived CD8 SP cells, used as control, were not stained with an HLA-24 tetramer, which presents an HIV-1-envelop-derived peptide (RYLRDQQLL, SEQ ID NO:2). Therefore, it turned out that the frequency of T cells having the same gene rearrangement pattern as in the originating T cells, which was observed in Comparative Example 1 (one forth), was greatly improved by stimulating the TCR of redifferentiated T-lineage cells before the transition from the DN stage to the DP stage had been completely finished.

Next, as shown in Fig. 26, CD8+ cells responded to the A24/Nef-138-8(wt) tetramer were collected, expanded, and stimulated again with PHA (this stimulation with PHA is referred as hereinafter to the "second stimulation"). This redifferentiation experiment was repeated independently several times, thereby eventually generating CD8 SP cell lines that responded to the A24/Nef-138-8(wt) tetramer (reT-1, reT-2.1, reT-2.2, and reT-3).

From the results of an analysis of the sequences of TCRA and TCRB mRNAs in these redifferentiated CD8 SP cell lines, it turned out that their respective rearrangement patterns of the TCR genes were identical to those exhibited by their originating CD8 T-cell clone H25-4, as shown in Fig. 27 and in Tables 8 and 9. Even in cases of antibodies recognizing the above-mentioned tetramer, their amino acid sequences (particularly of the CDR3) are often different; however, the respective rearrangement patterns of these TCR genes were completely identical between the CD8 SP cell lines responding to the A24/Nef-138-8(wt) tetramer (reT-1, reT-2.1, reT-2.2, and reT-3) and their originating CD8 T-cell clone H25-4, as shown in Tables 8 and 9. Also from such date, therefore, it was confirmed that the frequency of T cells having the same gene rearrangement pattern as in the originating T cells, which was observed in Comparative Example 1 (one forth), was greatly improved by stimulating the TCR of redifferentiated T-lineage cells before the transition from the DN stage to the DP stage had been completely finished.

Further, in order to examine that these redifferentiated CD8 SP cell lines were of the T cell lineage, the gene expression profile in cells of the reT-2.1 cell line was compared to those in peripheral blood (PB) CD4+ T cells, PB CD8+ T cells, and cells of the H25-4 clone by quantitative PCR. The results obtained are shown in Figs. 28 to 30.

As will be apparent from the results shown in Fig. 28, the respective expression patterns of CD3, CD4, and CD8 were the same in the PB CD8+ T cells, the redifferentiated CD8 SP cells, and the cells of the originating T-cell clone H25-4 (hereinafter referred as to the "H25-4 original T-cell clone"), except for the PB CD4+ T cells.

As shown in Fig. 29, it was also demonstrated that genes characterized by their cytotoxicity, for example, granzyme B (GZMB), perforin (PFR1), IFN-γ (IFNG), and FAS ligand (FASLG) were expressed on the PB CD8+ T cells and at a relatively high level on the CD8 SP cells, which was a population of T cells that had been already received antigen stimulation, and on the cells of the H25-4 original T-cell clone.

Further, as will be apparent from the results shown in Fig. 30, the expression patterns of several transcription or signaling factors and cell surface molecules were the same in the PB CD8+ T cells, the redifferentiated CD8 SP cells, and the cells of the H25-4 original T-cell clone.

The overall gene expression profile was also analyzed with cDNA microarrays for redifferentiated CD8 cells of the above-mentioned cell line, cells of the H25-4 original T-cell clone, and peripheral blood NK cells, in order to exclude the possibility that the redifferentiated CD8 SP cells might be those which acquired NK-like properties during the period of co-culturing with OP9-DL1 or PBMC cells. The results obtained are shown in Figs. 31 and 32.

As will be apparent from the results shown in Figs. 31 and 32, it turned out that the gene expression profile of the redifferentiated CD8 SP cells was similar to that of the cells of the H25-4 original T-cell clone. However, the correlation coefficients and cluster analysis did not find any relationship between the redifferentiated CD8 SP cells and the NK cells. Therefore, it was demonstrated that T-iPS cells were capable of redifferentiation into CD8+ T cells exhibiting the same antigen specificity as that exhibited by their originating T cells.

### (Example 3)

### <Increased proliferative properties of CD8 SP cells obtained by the method according to the present invention >

In Example 1, the number of T-lineage cells obtained by co-culturing T-iPS cells with OP9-DL1 cells in a 6-cm dish was less than 10⁵ cells. However, although not shown in any drawings, the first stimulation allowed the number of cells to be increased to more than 10⁸ cells. Based on this finding, cells of each of the cell lines reT-1, reT-2.2, and reT-3 were stimulated with 5 µg/mL PHA, 10 ng/mL IL-7, and 10 ng/mL IL-15, and then subjected to determination of the respective increase (expansion) ratios of these cell lines at two weeks after the stimulation, in order to examine the proliferative properties of CD8 SP cells of these cell lines obtained by the method according to the present invention. The results obtained are shown in Fig. 33.

As will be apparent from Fig. 33, the number of cells of the H25-4 original T-cell clone was expanded about 20 times, whereas the number of CD8+ cells of each of these cell lines was expanded 100 to 1,000 times, at two weeks after the stimulation.

In addition, as shown in Fig. 34, a particular population of cells was found to express central memory T-cell markers, for example, CCR7, CD27, and CD28, even after 100 to 1,000 times expansion was achieved (for central memory T-cell markers, see Romero, P. et al., J. Immunol., 2007, Vol. 178, pp.4112-4119).

Regarding enhanced proliferative (replicative) ability of the CD8 SP cells of the present invention as shown above, it is supposed that by going through a state of iPS cells having an extremely high telomerase activity, the telomere of which the length have become short in cells of the H25-4 original T-cell clone is re-elongated, so that enhanced proliferative ability can be conferred on redifferentiated T cells (see Takahashi, K. et al., Cell, 2007, Vol. 131, pp.861-872; Marion, R. M. et al., Cell Stem Cell, 2009, Vol. 4, pp.141-154; Monteiro, J. et al., J. Immunol., 1996, Vol. 156, pp.3587-3590; and Weng, N. P. et al., Immunity, 1998, Vol. 9, pp.151-157). Thus, the present inventors determined the telomere length in CD8 SP cells of each of the three cell lines obtained by the method according to the present invention. The results obtained are shown in Fig. 35.

As will be apparent from the results shown in Fig. 35, the redifferentiated T cells of each of the three cell lines actually had a longer telomere length than their originating T cells.

Therefore, it is demonstrated that by go through a state of T-iPS cells, the CD8 SP cells obtained by the method according to the present invention (cloned cytotoxic T cells) can be rejuvenated to central memory-like T cells exhibiting superior proliferative ability and viability.

Although not shown in any drawings, there was observed, throughout this Example, neither autonomous cellular proliferation nor survival of abnormal cells in the absence of cytokines.

### (Example 4)

### <Antigen-specific functions of CD8 SP cells obtained by the method according to the present invention>

A major mechanism of cytotoxicity of CTLs is secreting cytolytic molecules which are produced together with TCR signals. Thus, the present inventors examined by intracellular staining as to whether CD8 SP cells of a cell line obtained by the method according to the present invention (reT-2.2) also secreted cytolytic molecules by stimulating TCR with α-CD3/CD28 beads in an amount three times that of the CD8 SP cells. The results obtained are shown in Fig. 36.

As shown in Fig. 36, it was demonstrated that after the stimulation with α-CD3/28 beads, granzyme B, a cytolytic molecule, was produced and accumulated in granules of the redifferentiated CD8 T cells (see Fig. 36, left panel).

CD107a, known as lysosome-associated membrane protein 1 (LAMP1), is a granulocyte membrane protein which is transiently expressed when CTLs are stimulated, in conjunction with degranulation in which cytolytic molecules are secreted, and is known to return to the cytoplasm after its secretion (Rubio, V. et al., Nat. Med., 2003, Vol. 9, pp.1377-1382). Thus, redifferentiated CD8 T cells of an above-mentioned cell line were cultured in the presence or absence of stimulation with α-CD3/28 beads, and then CD107a molecules on the cellular surface of these cells were detected with a fluorescent dye-conjugated antibody. The results obtained are shown in Fig. 36.

As shown in Fig. 36, the incorporation of the antibody was detected only when the redifferentiated T cells of an above-mentioned cell line were stimulated with α-CD3/28 beads (see Fig. 36, right panel).

Next, function assays, an ELISPOT (Enzyme-Linked ImmunoSpot) assay and a ⁵¹Cr-release assay, were carried out using HLA-A24-positive B-LCL cells (cells derived from the patient from which the H25-4 T-cell clone was derived) acting as an antigen-presenting cell, in order to assess whether or not redifferentiated CD8 T cells of the three cell lines of the present invention were capable of exerting cytotoxicity by recognizing a particular peptide in MHC complexes. The results obtained are shown in Figs. 37 and 38.

Regarding the peptides which were used in these assays, Gag-28-9(wt) (KYKLKHIVW, SEQ ID NO:3) is an antigen peptide of the HIV-1 Gag protein (amino acid residues 28 to 36) and Nef-138-8(2F) (RFPLTFGW, SEQ ID NO:4) is a one-residue mutant of Nef-138-8(wt) in which a tyrosine residue is substituted with phenylalanine, which both are present on HLA-A24.

As shown in Fig. 37, from the results of evaluating the cytokine production capability per cell by ELISPOT, it turned out that the redifferentiated CD8 T cells of each of the cell lines of the present invention remarkably produced IFN-γ, responding to the stimulation with a specific antigen, Nef-138-8(wt).

As shown in Fig. 38, from the results of examining the cytolytic ability by ⁵¹Cr-release assay, it turned out that only in the presence of Nef-138-8(wt), the redifferentiated CD8 T cells of each of the cell lines of the present invention lyzed B-LCLs into which ⁵¹Cr was incorporated.

Therefore, it is demonstrated that the CD8 SP cells obtained by the method according to the present invention release cytotoxic molecules and specifically kill target cells expressing a specific antigen, and thus are functional, antigen-specific T cells.

### (Example 5)

### <Re-differentiation from a T-iPS cell into a CD4 single positive cell according to the present method>

In this Example, the inventors attempted producing a mature CD4 SP cell from a T lineage cell derived from a T-iPS cell, by using a method wherein stimulating a TCR of the re-differentiated T lineage cell before the completion of the transition from the DN stage to the DP stage.

As shown in Fig. 39, small aggregates (less than 100 cells) of T-iPS cells (H254SeVt-3) obtained in Preparation Example 1 were transferred on irradiated C3H10T1/2 cells, and co-cultured in EB medium containing 20 ng/mL of VEGF. On day 14, hematopoietic cells contained in the iPS-sacs were collected and transferred onto irradiated OP9-DL1 cells, and then co-cultured in EB medium containing 20ng/mL of SCF, 10 ng/mL of TPO, 10 ng/mL FLT-3L and 10ng/mL of IL-7. These cells were transferred onto irradiated OP9-DL1 cells and differentiated the hematopoietic cells into T lineage cells in OP9 medium in the presence of 5ng/mL FLT-3L and 1 ng/mL IL-7. On day 35, the cells directed to T lineage cells were stimulated by adding to the medium α-CD3/28 beads of three time amount of the cells or 5 µg/mL of PHA and further cultured on the OP9-DL 1 cells (the stimulation withα-CD3/28 beads or PHA is defined as the first stimulation). Then, the T lineage cells were harvested on day 45, and co-cultured with irradiated HLA-A24-PBMCs in RH10 medium in the presence of 10 ng/mL of IL-7 and 10 ng/mL of IL-15.

As a result, as shown in Fig. 40, when the cells were stimulated with PHA as the first stimulation, CD4 SP cells appeared on day 60 as well as CD8 SP cells. The similar results were obtained when the cells were stimulated withα-CD3 as the first stimulation (data not shown).

As mentioned above, it is possible to improve the occurrence of the appearance of T cells having the same rearrangement pattern of TCR genes as the original ones among the CD8 SP cells obtained by re-differentiation from a human T cells having antigen specificity via iPS cells. Further, a human CD8 SP cell obtained by the method has antigen specific immune functions. The similar result can be also obtained in a human CD4 SP cells. Furthermore, the human CD8 SP cell does not express PD-1, which is a marker of exhausted T cells, but expresses CCR7 together with CD27 and CD28, which represent a phenotype of central memory T cells, and has a teromere longer than the original ones and high self-reproduction capability.

Therefore, a method according to the invention and CD8 SP cells or CD4 SP cells obtained by the method are useful in the treatment or prevention of diseases, such as malignant tumors, infections (chronic infections) and autoimmune diseases.

## Claims

1. A method for producing a human single-positive CD4 or CD8 T cell having antigen specificity, which comprises the steps of:
differentiating an iPS cell that has been induced from a human T cell, into a CD4/CD8 double-negative cell, wherein the human T cell has antigen specificity,
stimulating a T cell receptor of the CD4/CD8 double-negative cell, and
differentiating the CD4/CD8 double-negative cell whose T cell receptor has been stimulated, into a single-positive CD4 or CD8 T cell.

2. The method according to claim 1, wherein the single-positive CD4 or CD8 T cell is a CD8 single positive T cell.

3. An isolated population of human single positive CD4 or CD8 T cells having antigen specificity which are obtainable by the method according to claim 1 or 2, wherein the population has an improved ratio of T cells having the same TCR gene rearrangement pattern as the original human T cell compared to a population of human single positive CD4 or CD8 T cells obtained by said method without the step of stimulating a T cell receptor of the CD4/CD8 DN cell.

4. A pharmaceutical composition, comprising the isolated population of the human single positive CD4 or CD8 T cell according to claim 3.

5. A method of producing a pharmaceutical composition, comprising producing a human single positive CD4 or CD8 T cells according to claim 1 or 2 and formulating the human single positive CD4 or CD8 T cells into dosage forms by known pharmaceutical methods.

## Patentansprüche

1. Verfahren zur Produktion einer humanen einzel-positiven CD4- oder CD8-T-Zelle, welche Antigen-Spezifität aufweist, welches die Schritte umfasst von:
Differenzieren einer iPS-Zelle, welche aus einer humanen T-Zelle induziert wurde, in eine CD4/CD8 doppel-negative Zelle, wobei die humane T-Zelle Antigen-Spezifität aufweist,
Stimulieren eines T-Zell-Rezeptors der CD4/CD8 doppel-negativen Zelle, und
Differenzieren der CD4/CD8 doppel-negativen Zelle, deren T-Zell-Rezeptor stimuliert wurde, in eine einzel-positive CD4- oder CD8-T-Zelle.

2. Verfahren gemäß Anspruch 1, wobei die einzel-positive CD4- oder CD8-T-Zelle eine einzel-positive CD8-T-Zelle ist.

3. Isolierte Population von humanen einzel-positiven CD4- oder CD8-T-Zellen, welche Antigen-Spezifität aufweisen, welche durch das Verfahren gemäß Anspruch 1 oder 2 erhältlich sind, wobei die Population ein verbessertes Verhältnis von T-Zellen hat, welche dasselbe TCR-Gen-Neuanordnungsmuster (TCR gene rearrangement) wie die ursprüngliche humane T-Zelle aufweisen, verglichen mit einer Population von humanen einzel-positiven CD4- oder CD8-T-Zellen, erhalten durch das Verfahren ohne den Schritt des Stimulierens eines T-Zell-Rezeptors der CD4/CD8 DN-Zellen.

4. Pharmazeutische Zusammensetzung, umfassend die isolierte Population der humanen einzel-positiven CD4- oder CD8-T-Zellen gemäß Anspruch 3.

5. Verfahren zur Produktion einer pharmazeutischen Zusammensetzung, umfassend Produzieren einer humanen einzel-positiven CD4- oder CD8-T-Zelle(n) gemäß Anspruch 1 oder 2 und Formulieren der humanen einzel-positiven CD4- oder CD8-T-Zelle(n) als Darreichungsformen mittels bekannter pharmazeutischer Verfahren.

## Revendications

1. Procédé de production d'une cellule T CD4 ou CD8 simple positive humaine présentant une spécificité d'antigène, qui comprend les étapes suivantes :
la différenciation d'une cellule iPS qui a été induite à partir d'une cellule T humaine, en cellule double négative CD4/CD8, dans lequel la cellule T humaine présente une spécificité d'antigène,
la stimulation d'un récepteur de cellule T de la cellule double négative CD4/CD8, et
la différenciation de la cellule double négative CD4/CD8 dont le récepteur de cellule T a été stimulé en une cellule T CD4 ou CD8 simple positive.

2. Procédé selon la revendication 1, dans lequel la cellule T CD4 ou CD8 simple positive est une cellule T simple positive CD8.

3. Population isolée de cellules T CD4 ou CD8 simples positives humaines présentant une spécificité d'antigène qui peuvent être obtenues par le procédé selon la revendication 1 ou 2, dans laquelle la population présente un rapport amélioré de cellules T ayant le même motif de réarrangement de gènes TCR que la cellule T humaine d'origine par rapport à une population de cellules T CD4 ou CD8 simples positives humaines obtenues par ledit procédé sans l'étape de stimulation d'un récepteur de cellule T de la cellule DN CD4/CD8.

4. Composition pharmaceutique, comprenant la population isolée de la cellule T CD4 ou CD8 simple positive humaine selon la revendication 3.

5. Procédé de production d'une composition pharmaceutique, comprenant la production de cellules T CD4 ou CD8 simples positives humaines selon la revendication 1 ou 2 et la formulation des cellules T CD4 ou CD8 simples positives humaines en formes galéniques par des procédés pharmaceutiques connus.


### SEQUENCE LISTING

<110> The University of Tokyo
   <120> METHOD FOR PRODUCING ANTIGEN-SPECIFIC T CELLS
   <130> 199922PX
   <160> 149
   <170> PatentIn version 3.1
<210> 1
   <211> 8
   <212> PRT
   <213> Human immunodeficiency virus
   <223> Nef-138-8(WT)
   <400> 1
   Arg Tyr Pro Leu Thr Phe Gly Trp
   1               5
<210> 2
   <211> 9
   <212> PRT
   <213> Human immunodeficiency virus
   <223> HIV-1 envelope-derived peptides
   <400> 2
   Arg Tyr Leu Arg Asp Gln Gln Leu Leu
   1               5
<210> 3
   <211> 9
   <212> PRT
   <213> Human immunodeficiency virus
   <223> Gag-28-9(wt)
   <400> 3
   Lys Tyr Lys Leu Lys His Ile Val Trp
   1               5
<210> 4
   <211> 8
   <212> PRT
   <213> artificial
   <220>
   <223> Artificially synthesized polypeptide sequence (Nef-138-8(2F))
   <400> 4
   Arg Phe Pro Leu Thr Phe Gly Trp
   1 5
<210> 5
   <211> 25
   <212> DNA
   <213> Artificial
   <220>
   <223> Artificially synthesized primer sequence
   <400> 5
   atttgttttt tgggtagtta aaggt 25
<210> 6
   <211> 27
   <212> DNA
   <213> Artificial
   <220>
   <223> Artificially synthesized primer sequence
   <400> 6
   ccaactatct tcatcttaat aacatcc 27
<210> 7
   <211> 25
   <212> DNA
   <213> Artificial
   <220>
   <223> Artificially synthesized primer sequence
   <400> 7
   tggttaggtt ggttttaaat ttttg 25
<210> 8
   <211> 26
   <212> DNA
   <213> Artificial
   <220>
   <223> Artificially synthesized primer sequence
   <400> 8
   aacccaccct tataaattct caatta 26
<210> 9
   <211> 21
   <212> DNA
   <213> Artificial
   <220>
   <223> Artificially synthesized primer sequence
   <400> 9
   aacagcctca agatcatcag c 21
<210> 10
   <211> 21
   <212> DNA
   <213> Artificial
   <220>
   <223> Artificially synthesized primer sequence
   <400> 10
   ttggcaggtt tttctagacg g 21
<210> 11
   <211> 19
   <212> DNA
   <213> Artificial
   <220>
   <223> Artificially synthesized primer sequence
   <400> 11
   attcagccaa acgaccatc 19
<210> 12
   <211> 19
   <212> DNA
   <213> Artificial
   <220>
   <223> Artificially synthesized primer sequence
   <400> 12
   ggaaagggac cgaggagta 19
<210> 13
   <211> 18
   <212> DNA
   <213> Artificial
   <220>
   <223> Artificially synthesized primer sequence
   <400> 13
   cagcgcatgg acagttac 18
<210> 14
   <211> 18
   <212> DNA
   <213> Artificial
   <220>
   <223> Artificially synthesized primer sequence
   <400> 14
   ggagtgggag gaagaggt 18
<210> 15
   <211> 18
   <212> DNA
   <213> Artificial
   <220>
   <223> Artificially synthesized primer sequence
   <400> 15
   agtttcatct gcgacccg 18
<210> 16
   <211> 21
   <212> DNA
   <213> Artificial
   <220>
   <223> Artificially synthesized primer sequence
   <400> 16
   cctcatcttc ttgttcctcc t 21
<210> 17
   <211> 18
   <212> DNA
   <213> Artificial
   <220>
   <223> Artificially synthesized primer sequence
   <400> 17
   gcgggaaggg agaagaca 18
<210> 18
   <211> 18
   <212> DNA
   <213> Artificial
   <220>
   <223> Artificially synthesized primer sequence
   <400> 18
   ccggatcgga taggtgaa 18
<210> 19
   <211> 24
   <212> DNA
   <213> Artificial
   <220>
   <223> Artificially synthesized primer sequence
   <400> 19
   gacaggggga ggggaggagc tagg 24
<210> 20
   <211> 26
   <212> DNA
   <213> Artificial
   <220>
   <223> Artificially synthesized primer sequence
   <400> 20
   cttccctcca accagttgcc ccaaac 26
<210> 21
   <211> 26
   <212> DNA
   <213> Artificial
   <220>
   <223> Artificially synthesized primer sequence
   <400> 21
   gggaaatggg aggggtgcaa aagagg 26
<210> 22
   <211> 26
   <212> DNA
   <213> Artificial
   <220>
   <223> Artificially synthesized primer sequence
   <400> 22
   ttgcgtgagt gtggatggga ttggtg 26
<210> 23
   <211> 26
   <212> DNA
   <213> Artificial
   <220>
   <223> Artificially synthesized primer sequence
   <400> 23
   gcgtcctggg aagggagatc cggagc 26
<210> 24
   <211> 26
   <212> DNA
   <213> Artificial
   <220>
   <223> Artificially synthesized primer sequence
   <400> 24
   ttgaggggca tcgtcgcggg aggctg 26
<210> 25
   <211> 25
   <212> DNA
   <213> Artificial
   <220>
   <223> Artificially synthesized primer sequence
   <400> 25
   acgatcgtgg ccccggaaaa ggacc 25
<210> 26
   <211> 28
   <212> DNA
   <213> Artificial
   <220>
   <223> Artificially synthesized primer sequence
   <400> 26
   tgattgtagt gctttctggc tgggctcc 28
<210> 27
   <211> 21
   <212> DNA
   <213> Artificial
   <220>
   <223> Artificially synthesized primer sequence
   <400> 27
   caacgagagg attttgaggc t 21
<210> 28
   <211> 21
   <212> DNA
   <213> Artificial
   <220>
   <223> Artificially synthesized primer sequence
   <400> 28
   tgcagtacaa ctccatgacc a 21
<210> 29
   <211> 26
   <212> DNA
   <213> Artificial
   <220>
   <223> Artificially synthesized primer sequence
   <400> 29
   caacaaccga aatgcaccag ccccag 26
<210> 30
   <211> 21
   <212> DNA
   <213> Artificial
   <220>
   <223> Artificially synthesized primer sequence
   <400> 30
   tgcggcaaaa cctacacaaa g 21
<210> 31
   <211> 21
   <212> DNA
   <213> Artificial
   <220>
   <223> Artificially synthesized primer sequence
   <400> 31
   tacaggtggg gtctttcatt c 21
<210> 32
   <211> 20
   <212> DNA
   <213> Artificial
   <220>
   <223> Artificially synthesized primer sequence
   <400> 32
   tgtgcaccaa catctacaag 20
<210> 33
   <211> 20
   <212> DNA
   <213> Artificial
   <220>
   <223> Artificially synthesized primer sequence
   <400> 33
   gcgttcttgg ctttcaggat 20
<210> 34
   <211> 24
   <212> DNA
   <213> Artificial
   <220>
   <223> Artificially synthesized primer sequence
   <400> 34
   cagatcctaa acagctcgca gaat 24
<210> 35
   <211> 23
   <212> DNA
   <213> Artificial
   <220>
   <223> Artificially synthesized primer sequence
   <400> 35
   gcgtacgcaa attaaagtcc aga 23
<210> 36
   <211> 20
   <212> DNA
   <213> Artificial
   <220>
   <223> Artificially synthesized primer sequence
   <400> 36
   aaatgtttgt gttgcggtca 20
<210> 37
   <211> 20
   <212> DNA
   <213> Artificial
   <220>
   <223> Artificially synthesized primer sequence
   <400> 37
   tctggcacag gtgtcttcag 20
<210> 38
   <211> 25
   <212> DNA
   <213> Artificial
   <220>
   <223> Artificially synthesized primer sequence
   <400> 38
   cagccctgat tcttccacca gtccc 25
<210> 39
   <211> 25
   <212> DNA
   <213> Artificial
   <220>
   <223> Artificially synthesized primer sequence
   <400> 39
   tggaaggttc ccagtcgggt tcacc 25
<210> 40
   <211> 20
   <212> DNA
   <213> Artificial
   <220>
   <223> Artificially synthesized primer sequence
   <400> 40
   gagcaaggta cctcagccag 20
<210> 41
   <211> 20
   <212> DNA
   <213> Artificial
   <220>
   <223> Artificially synthesized primer sequence
   <400> 41
   aacaatggct gagttgggac 20
<210> 42
   <211> 20
   <212> DNA
   <213> Artificial
   <220>
   <223> Artificially synthesized primer sequence
   <400> 42
   gattcctgct acctccctcc 20
<210> 43
   <211> 20
   <212> DNA
   <213> Artificial
   <220>
   <223> Artificially synthesized primer sequence
   <400> 43
   agcgtcctcc aaagagaaca 20
<210> 44
   <211> 21
   <212> DNA
   <213> Artificial
   <220>
   <223> Artificially synthesized primer sequence
   <400> 44
   aactatgttt tggtatcgtc a 21
<210> 45
   <211> 24
   <212> DNA
   <213> Artificial
   <220>
   <223> Artificially synthesized primer sequence
   <400> 45
   cacgatgttc tggtaccgtc agca 24
<210> 46
   <211> 22
   <212> DNA
   <213> Artificial
   <220>
   <223> Artificially synthesized primer sequence
   <400> 46
   cagtgtgtcc tggtaccaac ag 22
<210> 47
   <211> 21
   <212> DNA
   <213> Artificial
   <220>
   <223> Artificially synthesized primer sequence
   <400> 47
   aaccctttat tggtaccgac a 21
<210> 48
   <211> 22
   <212> DNA
   <213> Artificial
   <220>
   <223> Artificially synthesized primer sequence
   <400> 48
   atcccttttt tggtaccaac ag 22
<210> 49
   <211> 22
   <212> DNA
   <213> Artificial
   <220>
   <223> Artificially synthesized primer sequence
   <400> 49
   aaccctttat tggtatcaac ag 22
<210> 50
   <211> 21
   <212> DNA
   <213> Artificial
   <220>
   <223> Artificially synthesized primer sequence
   <400> 50
   cgctatgtat tggtacaagc a 21
<210> 51
   <211> 25
   <212> DNA
   <213> Artificial
   <220>
   <223> Artificially synthesized primer sequence
   <400> 51
   ctcccgtttt ctggtacaga cagac 25
<210> 52
   <211> 22
   <212> DNA
   <213> Artificial
   <220>
   <223> Artificially synthesized primer sequence
   <400> 52
   cgctatgtat tggtataaac ag 22
<210> 53
   <211> 26
   <212> DNA
   <213> Artificial
   <220>
   <223> Artificially synthesized primer sequence
   <400> 53
   ttatgtttac tggtatcgta agaagc 26
<210> 54
   <211> 22
   <212> DNA
   <213> Artificial
   <220>
   <223> Artificially synthesized primer sequence
   <400> 54
   caaaatgtac tggtatcaac aa 22
<210> 55
   <211> 25
   <212> DNA
   <213> Artificial
   <220>
   <223> Artificially synthesized primer sequence
   <400> 55
   atacatgtac tggtatcgac aagac 25
<210> 56
   <211> 23
   <212> DNA
   <213> Artificial
   <220>
   <223> Artificially synthesized primer sequence
   <400> 56
   ggccatgtac tggtatagac aag 23
<210> 57
   <211> 24
   <212> DNA
   <213> Artificial
   <220>
   <223> Artificially synthesized primer sequence
   <400> 57
   gtatatgtcc tggtatcgac aaga 24
<210> 58
   <211> 24
   <212> DNA
   <213> Artificial
   <220>
   <223> Artificially synthesized primer sequence
   <400> 58
   taacctttat tggtatcgac gtgt 24
<210> 59
   <211> 21
   <212> DNA
   <213> Artificial
   <220>
   <223> Artificially synthesized primer sequence
   <400> 59
   ggccatgtac tggtaccgac a 21
<210> 60
   <211> 22
   <212> DNA
   <213> Artificial
   <220>
   <223> Artificially synthesized primer sequence
   <400> 60
   tcatgtttac tggtatcggc ag 22
<210> 61
   <211> 27
   <212> DNA
   <213> Artificial
   <220>
   <223> Artificially synthesized primer sequence
   <400> 61
   ttatgtttat tggtatcaac agaatca 27
<210> 62
   <211> 21
   <212> DNA
   <213> Artificial
   <220>
   <223> Artificially synthesized primer sequence
   <400> 62
   caacctatac tggtaccgac a 21
<210> 63
   <211> 22
   <212> DNA
   <213> Artificial
   <220>
   <223> Artificially synthesized primer sequence
   <400> 63
   taccctttac tggtaccggc ag 22
<210> 64
   <211> 26
   <212> DNA
   <213> Artificial
   <220>
   <223> Artificially synthesized primer sequence
   <400> 64
   atacttctat tggtacagac aaatct 26
<210> 65
   <211> 21
   <212> DNA
   <213> Artificial
   <220>
   <223> Artificially synthesized primer sequence
   <400> 65
   cacggtctac tggtaccagc a 21
<210> 66
   <211> 21
   <212> DNA
   <213> Artificial
   <220>
   <223> Artificially synthesized primer sequence
   <400> 66
   cgtcatgtac tggtaccagc a 21
<210> 67
   <211> 21
   <212> DNA
   <213> Artificial
   <220>
   <223> Artificially synthesized primer sequence
   <400> 67
   gccaaacagc cttacaaaga c 21
<210> 68
   <211> 20
   <212> DNA
   <213> Artificial
   <220>
   <223> Artificially synthesized primer sequence
   <400> 68
   tttccaagcc ccacacagtc 20
<210> 69
   <211> 25
   <212> DNA
   <213> Artificial
   <220>
   <223> Artificially synthesized primer sequence
   <400> 69
   cttacctaca actgtgaatc tggtg 25
<210> 70
   <211> 25
   <212> DNA
   <213> Artificial
   <220>
   <223> Artificially synthesized primer sequence
   <400> 70
   cttacctaca acggttaacc tggtc 25
<210> 71
   <211> 25
   <212> DNA
   <213> Artificial
   <220>
   <223> Artificially synthesized primer sequence
   <400> 71
   cttacctaca acagtgagcc aactt 25
<210> 72
   <211> 26
   <212> DNA
   <213> Artificial
   <220>
   <223> Artificially synthesized primer sequence
   <400> 72
   catacccaag acagagagct gggttc 26
<210> 73
   <211> 25
   <212> DNA
   <213> Artificial
   <220>
   <223> Artificially synthesized primer sequence
   <400> 73
   cttacctagg atggagagtc gagtc 25
<210> 74
   <211> 22
   <212> DNA
   <213> Artificial
   <220>
   <223> Artificially synthesized primer sequence
   <400> 74
   catacctgtc acagtgagcc tg 22
<210> 75
   <211> 21
   <212> DNA
   <213> Artificial
   <220>
   <223> Artificially synthesized primer sequence
   <400> 75
   ccttcttacc tagcacggtg a 21
<210> 76
   <211> 21
   <212> DNA
   <213> Artificial
   <220>
   <223> Artificially synthesized primer sequence
   <400> 76
   cttacccagt acggtcagcc t 21
<210> 77
   <211> 21
   <212> DNA
   <213> Artificial
   <220>
   <223> Artificially synthesized primer sequence
   <400> 77
   cccgcttacc gagcactgtc a 21
<210> 78
   <211> 21
   <212> DNA
   <213> Artificial
   <220>
   <223> Artificially synthesized primer sequence
   <400> 78
   ccagcttacc cagcactgag a 21
<210> 79
   <211> 16
   <212> DNA
   <213> Artificial
   <220>
   <223> Artificially synthesized primer sequence
   <400> 79
   cgcgcacacc gagcac 16
<210> 80
   <211> 21
   <212> DNA
   <213> Artificial
   <220>
   <223> Artificially synthesized primer sequence
   <400> 80
   ctcgcccagc acggtcagcc t 21
<210> 81
   <211> 22
   <212> DNA
   <213> Artificial
   <220>
   <223> Artificially synthesized primer sequence
   <400> 81
   cttacctgta accgtgagcc tg 22
<210> 82
   <211> 24
   <212> DNA
   <213> Artificial
   <220>
   <223> Artificially synthesized primer sequence
   <400> 82
   tctggtatgt gcaatacccc aacc 24
<210> 83
   <211> 20
   <212> DNA
   <213> Artificial
   <220>
   <223> Artificially synthesized primer sequence
   <400> 83
   ctgaggaaac cctctgtgca 20
<210> 84
   <211> 24
   <212> DNA
   <213> Artificial
   <220>
   <223> Artificially synthesized primer sequence
   <400> 84
   ccgggcagca gacactgctt ctta 24
<210> 85
   <211> 24
   <212> DNA
   <213> Artificial
   <220>
   <223> Artificially synthesized primer sequence
   <400> 85
   gatggaaggt ttacagcaca gctc 24
<210> 86
   <211> 21
   <212> DNA
   <213> Artificial
   <220>
   <223> Artificially synthesized primer sequence
   <400> 86
   cctcccaggg tccagagtac g 21
<210> 87
   <211> 20
   <212> DNA
   <213> Artificial
   <220>
   <223> Artificially synthesized primer sequence
   <400> 87
   ggattgcgct gaaggaagag 20
<210> 88
   <211> 24
   <212> DNA
   <213> Artificial
   <220>
   <223> Artificially synthesized primer sequence
   <400> 88
   gcaacatgct ggcggagcac ccac 24
<210> 89
   <211> 24
   <212> DNA
   <213> Artificial
   <220>
   <223> Artificially synthesized primer sequence
   <400> 89
   tgaaggtcac ctttgatacc accc 24
<210> 90
   <211> 20
   <212> DNA
   <213> Artificial
   <220>
   <223> Artificially synthesized primer sequence
   <400> 90
   aactgcacgt accagacatc 20
<210> 91
   <211> 20
   <212> DNA
   <213> Artificial
   <220>
   <223> Artificially synthesized primer sequence
   <400> 91
   accctgagtg tccaggaggg 20
<210> 92
   <211> 23
   <212> DNA
   <213> Artificial
   <220>
   <223> Artificially synthesized primer sequence
   <400> 92
   cactgctgac cttaacaaag gcg 23
<210> 93
   <211> 20
   <212> DNA
   <213> Artificial
   <220>
   <223> Artificially synthesized primer sequence
   <400> 93
   tcctggtgac agtagttacg 20
<210> 94
   <211> 24
   <212> DNA
   <213> Artificial
   <220>
   <223> Artificially synthesized primer sequence
   <400> 94
   aggctcaaag ccttctcagc aggg 24
<210> 95
   <211> 24
   <212> DNA
   <213> Artificial
   <220>
   <223> Artificially synthesized primer sequence
   <400> 95
   tccaccagtt ccttcaactt cacc 24
<210> 96
   <211> 24
   <212> DNA
   <213> Artificial
   <220>
   <223> Artificially synthesized primer sequence
   <400> 96
   ttcatcaaaa cccttgggga cagc 24
<210> 97
   <211> 24
   <212> DNA
   <213> Artificial
   <220>
   <223> Artificially synthesized primer sequence
   <400> 97
   cccagcaggc agatgattct cgtt 24
<210> 98
   <211> 21
   <212> DNA
   <213> Artificial
   <220>
   <223> Artificially synthesized primer sequence
   <400> 98
   ggataaacat ctgtctctgc g 21
<210> 99
   <211> 20
   <212> DNA
   <213> Artificial
   <220>
   <223> Artificially synthesized primer sequence
   <400> 99
   aagggaatcc tctgactgtg 20
<210> 100
   <211> 20
   <212> DNA
   <213> Artificial
   <220>
   <223> Artificially synthesized primer sequence
   <400> 100
   gatagccata cgtccagatg 20
<210> 101
   <211> 24
   <212> DNA
   <213> Artificial
   <220>
   <223> Artificially synthesized primer sequence
   <400> 101
   tgccactctt aataccaagg aggg 24
<210> 102
   <211> 20
   <212> DNA
   <213> Artificial
   <220>
   <223> Artificially synthesized primer sequence
   <400> 102
   acactggctg caacagcatc 20
<210> 103
   <211> 20
   <212> DNA
   <213> Artificial
   <220>
   <223> Artificially synthesized primer sequence
   <400> 103
   ttacaaacga agtggcctcc 20
<210> 104
   <211> 24
   <212> DNA
   <213> Artificial
   <220>
   <223> Artificially synthesized primer sequence
   <400> 104
   accctgctga aggtcctaca ttcc 24
<210> 105
   <211> 20
   <212> DNA
   <213> Artificial
   <220>
   <223> Artificially synthesized primer sequence
   <400> 105
   cttggagaaa ggctcagttc 20
<210> 106
   <211> 24
   <212> DNA
   <213> Artificial
   <220>
   <223> Artificially synthesized primer sequence
   <400> 106
   tgcctcgctg gataaatcat cagg 24
<210> 107
   <211> 24
   <212> DNA
   <213> Artificial
   <220>
   <223> Artificially synthesized primer sequence
   <400> 107
   tcccagctca gcgattcagc ctcc 24
<210> 108
   <211> 20
   <212> DNA
   <213> Artificial
   <220>
   <223> Artificially synthesized primer sequence
   <400> 108
   gtcctgtcct cttgatagcc 20
<210> 109
   <211> 20
   <212> DNA
   <213> Artificial
   <220>
   <223> Artificially synthesized primer sequence
   <400> 109
   aactgcacgt accagacatc 20
<210> 110
   <211> 24
   <212> DNA
   <213> Artificial
   <220>
   <223> Artificially synthesized primer sequence
   <400> 110
   agcccagcca tgcaggcatc tacc 24
<210> 111
   <211> 24
   <212> DNA
   <213> Artificial
   <220>
   <223> Artificially synthesized primer sequence
   <400> 111
   gaacatcaca gccacccaga ccgg 24
<210> 112
   <211> 23
   <212> DNA
   <213> Artificial
   <220>
   <223> Artificially synthesized primer sequence
   <400> 112
   gcaaagctcc ctgtacctta cgg 23
<210> 113
   <211> 20
   <212> DNA
   <213> Artificial
   <220>
   <223> Artificially synthesized primer sequence
   <400> 113
   cacagcccct aaacctgaag 20
<210> 114
   <211> 20
   <212> DNA
   <213> Artificial
   <220>
   <223> Artificially synthesized primer sequence
   <400> 114
   agcaaaaact tcggaggcgg 20
<210> 115
   <211> 20
   <212> DNA
   <213> Artificial
   <220>
   <223> Artificially synthesized primer sequence
   <400> 115
   aaggagagga cttcaccacg 20
<210> 116
   <211> 20
   <212> DNA
   <213> Artificial
   <220>
   <223> Artificially synthesized primer sequence
   <400> 116
   ggcaagacgg aaccaatgtt 20
<210> 117
   <211> 20
   <212> DNA
   <213> Artificial
   <220>
   <223> Artificially synthesized primer sequence
   <400> 117
   ggggaggtgg tgacagagtc 20
<210> 118
   <211> 20
   <212> DNA
   <213> Artificial
   <220>
   <223> Artificially synthesized primer sequence
   <400> 118
   tgacggtgca attttctgct 20
<210> 119
   <211> 20
   <212> DNA
   <213> Artificial
   <220>
   <223> Artificially synthesized primer sequence
   <400> 119
   agaaacgcca cgtccaagtt 20
<210> 120
   <211> 20
   <212> DNA
   <213> Artificial
   <220>
   <223> Artificially synthesized primer sequence
   <400> 120
   tcctagccag gacgacgatt 20
<210> 121
   <211> 20
   <212> DNA
   <213> Artificial
   <220>
   <223> Artificially synthesized primer sequence
   <400> 121
   tcctgggatc agaggaggaa 20
<210> 122
   <211> 20
   <212> DNA
   <213> Artificial
   <220>
   <223> Artificially synthesized primer sequence
   <400> 122
   tctcctcccc tgactgtggt 20
<210> 123
   <211> 21
   <212> DNA
   <213> Artificial
   <220>
   <223> Artificially synthesized primer sequence
   <400> 123
   tgcaccctga gatggttctt t 21
<210> 124
   <211> 20
   <212> DNA
   <213> Artificial
   <220>
   <223> Artificially synthesized primer sequence
   <400> 124
   ctgccccgag acctgataac 20
<210> 125
   <211> 20
   <212> DNA
   <213> Artificial
   <220>
   <223> Artificially synthesized primer sequence
   <400> 125
   ttggctgcca cctgtctcta 20
<210> 126
   <211> 20
   <212> DNA
   <213> Artificial
   <220>
   <223> Artificially synthesized primer sequence
   <400> 126
   gaatgttttg gaggggcaag 20
<210> 127
   <211> 20
   <212> DNA
   <213> Artificial
   <220>
   <223> Artificially synthesized primer sequence
   <400> 127
   ttcaatgctc ccctccactt 20
<210> 128
   <211> 18
   <212> DNA
   <213> Artificial
   <220>
   <223> Artificially synthesized primer sequence
   <400> 128
   caacgcagag tacgcggg 18
<210> 129
   <211> 20
   <212> DNA
   <213> Artificial
   <220>
   <223> Artificially synthesized primer sequence
   <400> 129
   gctgttgttg aaggcgtttg 20
<210> 130
   <211> 20
   <212> DNA
   <213> Artificial
   <220>
   <223> Artificially synthesized primer sequence
   <400> 130
   tctccgagag cccgtagaac 20
<210> 131
   <211> 43
   <212> DNA
   <213> Homo sapiens
   <223> TRAV8-3*01 TRAJ10*01
   <400> 131
   tgtgctgtgg gtttcacggg aggaggaaac aaactcacct ttt 43
<210> 132
   <211> 37
   <212> DNA
   <213> Homo sapiens
   <223> TRAV13-1*01 TRAJ29*01
   <400> 132
   tgtgcagcaa tcctcaggaa acacacctct tgtcttt 37
<210> 133
   <211> 39
   <212> DNA
   <213> Homo sapiens
   <223> TRAV38-2/DV8*01 TRAJ31*01
   <400> 133
   tgtgcttatt ggagtaataa caatgccaga ctcatgttt 39
<210> 134
   <211> 36
   <212> DNA
   <213> Homo sapiens
   <223> TRAV12-1*01 TRAJ7*01
   <400> 134
   tgtgtggtgt actatgggaa caacagactc gctttt 36
<210> 135
   <211> 33
   <212> DNA
   <213> Homo sapiens
   <223> TRAV21*02 TRAJ16*01
   <400> 135
   tgtgctggtt cagatggcca gaagctgctc ttt 33
<210> 136
   <211> 42
   <212> DNA
   <213> Homo sapiens
   <223> TRAV1-2*01 TRAJ6*01
   <400> 136
   tgtgctgtga gagcatcagg aggaagctac atacctacat tt 42
<210> 137
   <211> 42
   <212> DNA
   <213> Homo sapiens
   <223> TRAV1-2*01 TRAJ7*01
   <400> 137
   tgtgctgtga gagattttta tgggaacaac ggactcgctt tt 42
<210> 138
   <211> 42
   <212> DNA
   <213> Homo sapiens
   <223> TRAV1-2*01 TRAJ10*01
   <400> 138
   tgtgctgtga gagatccggg aggaggaaac agactcacct tt 42
<210> 139
   <211> 36
   <212> DNA
   <213> Homo sapiens
   <223> TRAV1-2*01 TRAJ16*01
   <400> 139
   tgtactgtgt tttcacatgg ccaaaagctg ctcttt 36
<210> 140
   <211> 42
   <212> DNA
   <213> Homo sapiens
   <223> TRAV8-1*01 TRAJ6*01
<400> 140
   tgtgccgtga atgcatcagg aggaagctac atacctacat tt 42
<210> 141
   <211> 36
   <212> DNA
   <213> Homo sapiens
   <223> TRAV12-1*01 TRAJ6*01
   <400> 141
   tgtgtggtgt caggaggaag ctacatacct acattt 36
<210> 142
   <211> 39
   <212> DNA
   <213> Homo sapiens
   <223> TRAV12-1*01 TRAJ9*01
   <400> 142
   tgtgtggtga acaatactgg aggattcaaa actatcttt 39
<210> 143
   <211> 41
   <212> DNA
   <213> Homo sapiens
   <223> TRAV6*01 TRAJ13*01
   <400> 143
   tgtgctctag acattctggg ggttaccaga aagttacctt t 41
<210> 144
   <211> 38
   <212> DNA
   <213> Homo sapiens
   <223> TRAV12-1*01 TRAJ27*01
   <400> 144
   gtgtggtgaa caacaccaat gcaggcaaat caaccttt 38
<210> 145
   <211> 45
   <212> DNA
   <213> Homo sapiens
   <223> TRBV7-9*01 TRBD1*01 TRBJ2-5*01
   <400> 145
   tgtgccagca gcttacggga cagggtgccg gagacccagt acttc 45
<210> 146
   <211> 51
   <212> DNA
   <213> Homo sapiens
   <223> Germline TRBD1*01 TRBJ2-7*01
   <400> 146
   tacaaagctg taacattgtg gggacaactc tacgagcagt acttcgggcc g 51
<210> 147
   <211> 47
   <212> DNA
   <213> Homo sapiens
   <223> TRBV29-1*02 TRBD1*01 TRBJ2-2*01
   <400> 147
   tatatctctg cagcgttgat ggacagggaa acaccgggga gctgttt 47
<210> 148
   <211> 51
   <212> DNA
   <213> Homo sapiens
   <223> Germline TRBD2*01/*02 TRBJ2-7*01/*02
   <400> 148
   tatcatggtg taacattgtg gggactagtt taccctacga gcagtacttc g 51
<210> 149
   <211> 42
   <212> DNA
   <213> Homo sapiens
   <223> TRBV29-1*01 TRBD1*01 TRBJ2-2*01
   <400> 149
   tgcagcgttg atggacaggg aaacaccggg gagctgtttt tt 42